# EUROPEAN PATENT SPECIFICATION

(11) **EP 3 167 071 B1**
(45) Date of publication and mention of the grant of the patent: **07.10.2020**
(21) Application number: 15818477.0
(22) Date of filing: 08.07.2015
(51) Int. Cl.: C12N 15/10, C12N 15/62, C12N 15/66, C12N 9/22, C12P 19/34

(54) **COMPOSITIONS AND METHODS FOR SITE-DIRECTED DNA NICKING AND CLEAVING**
ZUSAMMENSETZUNGEN UND VERFAHREN FÜR ORTSSPEZIFISCHES DNA-NICKING UND SPALTUNG
COMPOSITIONS ET PROCÉDÉS POUR LE CLIVAGE ET LA COUPURE D'ADN DIRIGÉS

(30) Priority: 09.07.2014 US 201462022617 P; 17.10.2014 US 201462065238 P
(43) Date of publication of application: 17.05.2017
(73) Proprietor: Gen9, Inc., Boston MA 02210 (US)
(72) Inventor: JACOBSON, Joseph, Newton, Massachusetts 02459 (US); SAAEM, Ishtiaq E., Chelsea, Massachusetts 02150 (US); HUDSON, Michael E., Framingham, Massachusetts 01701 (US); LEAKE, Devin, Lexington, Massachusetts 02420 (US)
(74) Representative: Mewburn Ellis LLP
(86) International application number: PCT/US2015/039517
(87) International publication number: WO 2016/007604

(56) References cited:
- WO-A1-2014/093694
- WO-A1-2014/144288
- WO-A1-2014/191518
- WO-A1-2015/200334
- WO-A2-2015/035162
- DE-A1- 4 343 591
- US-A1- 2003 022 317
- US-A1- 2010 047 805
- US-A1- 2015 031 089
- US-A1- 2015 031 089
- US-B2- 8 058 004
- JOHN P GUILINGER ET AL: "Fusion of catalytically inactive Cas9 to FokI nuclease improves the specificity of genome modification", NATURE BIOTECHNOLOGY (ADVANCE ONLINE PUBLICATION), vol. 32, no. 6, 25 April 2014 (2014-04-25) , pages 577-582, XP055157221, ISSN: 1087-0156, DOI: 10.1038/nbt.2909
- YANNICK DOYON ET AL: "Enhancing zinc-finger-nuclease activity with improved obligate heterodimeric architectures", NATURE METHODS, vol. 8, no. 1, 1 January 2011 (2011-01-01) , pages 74-79, XP055075068, ISSN: 1548-7091, DOI: 10.1038/nmeth.1539
- J. WANG ET AL: "Targeted gene addition to a predetermined site in the human genome using a ZFN-based nicking enzyme", GENOME RESEARCH, vol. 22, no. 7, 20 March 2012 (2012-03-20) , pages 1316-1326, XP055075075, ISSN: 1088-9051, DOI: 10.1101/gr.122879.111
- E. KIM ET AL: "Precision genome engineering with programmable DNA-nicking enzymes", GENOME RESEARCH, vol. 22, no. 7, 20 April 2012 (2012-04-20) , pages 1327-1333, XP055106309, ISSN: 1088-9051, DOI: 10.1101/gr.138792.112
- C. L. RAMIREZ ET AL: "Engineered zinc finger nickases induce homology-directed repair with reduced mutagenic effects", NUCLEIC ACIDS RESEARCH, vol. 40, no. 12, 1 July 2012 (2012-07-01), pages 5560-5568, XP055065311, ISSN: 0305-1048, DOI: 10.1093/nar/gks179
- GABSALILOW LILIA ET AL: "Site- and strand-specific nicking of DNA by fusion proteins derived from MutH and I-SceI or TALE repeats.", NUCLEIC ACIDS RESEARCH, vol. 41, no. 7, E83, April 2013 (2013-04), pages 1-11, XP002776137, ISSN: 1362-4962
- TSAI ET AL.: 'Dimeric CRISPR RNA-guided Fokl nucleases for highly specific genome editing.' NAT BIOTECHNOL vol. 32, no. 6, ISSN 1087-0156 pages 569 - 576, XP055178523
- RUI-YAN WANG ET AL: "DNA Fragments Assembly Based on Nicking Enzyme System", PLOS ONE, vol. 8, no. 3, 6 March 2013 (2013-03-06), page e57943, XP55226294, DOI: 10.1371/journal.pone.0057943
- JIA-WANG WANG ET AL: "CRISPR/Cas9 nuclease cleavage combined with Gibson assembly for seamless cloning", BIOTECHNIQUES RAPID DISPATCHES, vol. 58, no. 4, 1 April 2015 (2015-04-01), XP55385711, US ISSN: 0736-6205, DOI: 10.2144/000114261

## Description

### FIELD

The present disclosure relates to compositions and methods for site-directed DNA nicking and cleaving, useful in, for example, in vitro nucleic acid assembly.

### BACKGROUND

Recombinant and synthetic nucleic acids have many applications in research, industry, agriculture, and medicine. Recombinant and synthetic nucleic acids can be used to express and obtain large amounts of polypeptides, including enzymes, antibodies, growth factors, receptors, and other polypeptides that may be used for a variety of medical, industrial, or agricultural purposes. Recombinant and synthetic nucleic acids can also be used to produce genetically modified organisms including modified bacteria, yeast, mammals, plants, and other organisms. Genetically modified organisms may be used in research (e.g., as animal models of disease, as tools for understanding biological processes, etc.), in industry (e.g., as host organisms for protein expression, as bioreactors for generating industrial products, as tools for environmental remediation, for isolating or modifying natural compounds with industrial applications, etc.), in agriculture (e.g., modified crops with increased yield or increased resistance to disease or environmental stress, etc.), and for other applications. Recombinant and synthetic nucleic acids may also be used as therapeutic compositions (e.g., for modifying gene expression, for gene therapy, etc.) or as diagnostic tools (e.g., as probes for disease conditions, etc.).

Numerous techniques have been developed for modifying existing nucleic acids (e.g., naturally occurring nucleic acids) to generate recombinant nucleic acids. For example, combinations of nucleic acid amplification, mutagenesis, nuclease digestion, ligation, cloning and other techniques may be used to produce many different recombinant nucleic acids. Chemically synthesized polynucleotides are often used as primers or adaptors for nucleic acid amplification, mutagenesis, and cloning.

Techniques also are being developed for de novo nucleic acid assembly whereby nucleic acids are made (e.g., chemically synthesized) and assembled to produce longer target nucleic acids of interest. For example, different multiplex assembly techniques are being developed for assembling oligonucleotides into larger synthetic nucleic acids that can be used in research, industry, agriculture, and/or medicine. However, one limitation of current assembly techniques is the unsatisfactory tools available to efficiently produce precisely designed synthetic oligonucleotides that are the building blocks to be assembled into desired nucleic acids. Rather, common techniques such as cleaving with restriction enzymes require introduction of specific recognition sites and upon re-ligation of the cleavage products, often leave behind extraneous nucleotide bases that are undesirable. Even where type IIS restriction enzymes are used which cut outside of the recognition site, it is still necessary to engineer the corresponding recognition sites into the construction oligonucleotides.

Thus, a need exists for an efficient DNA editing tool that can produce precisely designed synthetic oligonucleotides, to assist high-throughput DNA synthesis and assembly.

Guilinger at al., Nature Biotechnology 32, 6:557-583 (2014), reports fusion of catalytically inactive Cas9 to FokI improves the specificity of genome modification. Tsai et al., Nature Biotechnology 32, 6:569-577 (2014), reports dimeric CRISPR RNA-guided FokI nucleases can recognize extended sequences and edit endogenous genes with high efficiencies. US2010/0047805 reports methods and compositions for generating a single-stranded break in a target sequence. Doyon et al., Nature Methods 8, 1:74-79 (2011), reports a yeast-based selection system used to engineer zinc-finger nucleases with superior cleavage activity while suppressing homodimerization. Wang et al., Genome Research 22:1316-1326 (2012) reports zinc-finger nucleases can be engineered to induce a site-specific DNA single-stranded break. Kim at al., Genome Research 22:1327-1333 (2012) reports programmable DNA-nicking enzymes (nickases) that produce single-strand breaks, which are repaired by error-free homologous recombination. Ramirez et al., Nucleic Acids Research 40, 12:5560-5568 (2012) reports a method for converting zinc-finger nucleases to zinc finger nickases by inactivating the catalytic activity of one monomer. Gabsalilow et al., Nucleic Acids Research 41, 7:e83 (2013) reports site- and strand-specific nicking of DNA by fusion proteins containing MutH and I-SceI or TALE repeats. US 2003/0022317 reports the use of site-specific DNA nicking endonucleases in the production of single stranded regions in DNA to facilitate assembly of multiple nucleic acid fragments in an ordered array. Wang et al., Plos One 8, 3:e57943 (2013) reports a method of generating single-stranded DNA overlaps based on Nicking Endonucleases, for use in ligation-independent cloning. DE4343591 reports a method for producing oligomeric or polymeric functional elements by linking at least two design elements.

### SUMMARY

Aspects of the invention are set out in the claims. Compositions and methods for site-directed DNA nicking and cleaving are disclosed herein. In addition, methods for DNA assembly are described.

Disclosed herein are fusion proteins comprising a catalytically inactive Cas9 fused directly or indirectly to the catalytic domain of a nuclease. The catalytic domain may be, for example, the cleavage or cleaving domain of an endonuclease. In some embodiments of the disclosure, the endonuclease may be a restriction endonuclease, including, for example a type IIS restriction endonuclease. Embodiments of the disclosure include endonucleases that are wild type and/or catalytically active in a dimeric or multimeric form, including, without limitation, FokI, BsaI, AlwI, and BfilI. According to aspects of the disclosure, the nuclease catalytic domain of the fusion proteins of the disclosure may include a mutation that modifies the cleavage activity. For example, a catalytic domain of the endonuclease may include a modification that renders the nuclease catalytic domain a nickase that cleaves only one strand of a double-stranded oligonucleotide. In embodiments of the disclosure in which the catalytic domain functions in a dimeric or multimeric form, the catalytic domain may include a mutation on fewer than all of the monomers that make up the dimer or multimers, and/or two or more monomers may include different mutations. For example, FokI cleavage requires dimerization of the catalytic domain and thus, a wild-type monomer and a mutated, catalytically inactive monomer can dimerize to form a nickase. In certain embodiments of the disclosure, the nickase or nuclease activity may be provided by a hybrid between two or more different endonucleases and/or their catalytic domains. In one non-limiting example of the disclosure, the hybrid is FokI/BsaI.

Aspects of the disclosure relate to compositions and systems for site-directed nicking and cleaving of synthetic oligonucleotides, comprising: (a) a fusion protein comprising a Cas9 bound or fused, directly or indirectly, to one or more monomers of a dimeric or multimeric catalytic domains of a nuclease ("fCas9"); and (b) a second or more such monomers that are not bound to the same Cas9 as the bound monomers of (a). Such second monomer may be bound to another protein (including, for example, a second Cas9) or unbound. According to an embodiment of the disclosure, such compositions and systems may further comprise one or more gRNAs having a designed sequence (e.g., non-naturally occurring) bound to the Cas9; and may further comprise one or more designed oligonucleotides having a recognition region (e.g., non-naturally occurring) that is complementary to the gRNA sequence. As a result, the gRNA:fCas9 complex can specifically bind to the oligonucleotides at the recognition region, directing the catalytic domain of the nuclease to cleave or nick at a predetermined distance from the binding site. In some embodiments of the disclosure, a plurality (e.g., 3, 4, 5, 10, 20, 50, or more or less) of oligonucleotides each having a recognition region (e.g., non-naturally occurring) that is complementary to or the same as the gRNA, wherein the plurality of oligonucleotides (e.g., excluding the recognition region) together comprise a target polynucleotide. According to one embodiment of the disclosure, each of the plurality of oligonucleotides may comprise a flanking region on the 3' terminus, 5' terminus, or both termini; such flanking region comprising a primer site and/or a recognition region (e.g., non-naturally occurring) complementary to a gRNA sequence. In one aspect of the disclosure, the primer site may be or include, in whole or in part, the recognition region that is complementary to a gRNA sequence. The plurality of oligonucleotides may together comprise a target polynucleotide with or without the flanking regions.

According to one embodiment of the disclosure, the compositions and systems of the disclosure comprise a first Cas9-nuclease fusion protein that is bound to a first gRNA and a second Cas9-nuclease fusion protein bound to a second gRNAs that is different from the first gRNA. Additional different gRNAs (a third, fourth, fifth, etc. gRNA) may be employed in the compositions and systems of the disclosure. In one aspect of the disclosure, the first and second gRNA sequences comprise non-naturally occurring sequences that are complementary to each other and which may be employed in separate steps of methods of the disclosure. According to another aspect of the disclosure, composition and systems of the disclosure comprise first and second gRNA sequences that are not complementary.

Also disclosed are methods of using the compositions and systems described herein in applications of synthetic biology. For example, methods for nucleic acid synthesis and assembly using the compositions and systems of the disclosure are disclosed herein. According to some methods of the disclosure, a plurality of oligonucleotides that together comprise a target polynucleotide are disclosed. Each of the plurality of oligonucleotides comprises a flanking region on one or both termini. The flanking regions comprise a primer site within which is a recognition region comprising a non-naturally occurring sequence. The oligonucleotides may be amplified by a template-driven enzymatic reaction such as PCR. Following amplification, the plurality of oligonucleotides (each comprising a P strand and a complementary N strand) are contacted with a Cas9-nuclease fusion protein such as a catalytically inactive Cas9 fused to a monomer of a FokI catalytic domain. Bound to the Cas9 is a designed synthetic gRNA that is non-naturally occurring and complementary to the recognition region in the flanking region of the P and/or the N strand of each of the plurality of oligonucleotides. The plurality of oligonucleotides are brought into contact with the Cas9-FokI fusion protein in the presence of a second monomer of the FokI (or another endonuclease) catalytic domain (which can be stand-alone or in another Cas9-nuclease fusion) under conditions suitable for binding of the gRNA to the recognition region of the flanking region of the P strand and dimerization between the FokI monomer of the fusion protein and the second FokI monomer (or the monomer of another endonuclease). In some embodiments of the disclosure, one or both of the FokI monomers are mutated such that only the P or N strand is cut, making the catalytic activity of the FokI dimer that of a nickase. For example, in one embodiment of the disclosure, the FokI monomer of the fusion protein is modified (e.g., mutated, FokI*) such that it does not cut the P strand, but the second FokI monomer cuts the N strand (Cas9-FokI*:FokI or Cas9-FokI*:Cas9-FokI). In another embodiment of the disclosure, the second FokI monomer is mutated such that it does not cut the N strand, but the FokI monomer of the fusion protein cuts the P strand (Cas9-FokI:FokI* or Cas9-FokI:Cas9-FokI*). The different complexes (Cas9-FokI*:FokI, Cas9-FokI*:Cas9-FokI, Cas9-FokI:FokI* and Cas9-FokI:Cas9-FokI*) can be used together in any combination (in one reaction mixture or in a step-wise process) to nick one or both strands of a double-stranded DNA. In another embodiment of the disclosure, both the FokI monomer of the fusion protein and the second FokI monomer are catalytically active such that both the P and N strands are cut and the flanking region is cleaved from the remainder of the oligonucleotide. The resulting oligonucleotides may have blunt ends or sticky ends and may then be ligated in a predefined order to assemble a target polynucleotide, or subjected to further processing such as the production and/or modification of cohesive single-stranded overhanging ends, or polymerase assembly where a polymerase is used to extend the oligonucleotides by one or more nucleotide. According to one embodiment of the disclosure, the one or both flanking regions of the plurality of oligonucleotides are cleaved from the remainder of the oligonucleotides in first and second nicking steps using different Cas9-nuclease fusion proteins comprising different length linkers, and/or comprising different gRNAs designed to position the catalytic domains of the fusion protein at different locations on the P and N strands so as to produce single-stranded overhanging ends on the oligonucleotides that are designed to permit cohesive end assembly of the oligonucleotides to form a target polynucleotide.

It should be noted that while FokI is used as an exemplary endonuclease to illustrate the present disclosure, one of ordinary skill in the art would understand that other endonucleases can also be used in place of or together with FokI.

In one specific aspect of the disclosure, a method for cleaving a polynucleotide is provided, comprising: (a) nicking a first strand of a double-stranded polynucleotide with a first nickase to produce a first nick, wherein the first nickase is configured to recognize and bind a first site on the double-stranded polynucleotide; and (b) nicking a second strand of the double-stranded polynucleotide with a second nickase to produce a second nick, wherein the second nickase is configured to recognize and bind a second site on the double-stranded polynucleotide, thereby producing a cleaved polynucleotide fragment having an overhang defined by the first nick and the second nick, wherein the overhang is predesigned by selecting the first and second site. The overhang can have a predetermined length and/or sequence such that it can specifically and at least partially anneal with another overhang to facilitate ligation with another oligonucleotide. In some embodiments of the disclosure, the overhang can be 1, 2, 3, 4, 5, 6, 7, 8, 9, 10, 11, 12, 13, 14, 15, 16, 17, 18, 19 or 20 nucleotides in length, or longer.

Another aspect of the disclosure is directed to a composition for site-directed DNA cleavage, comprising: (a) a first nickase bound to a first non-naturally occurring guide sequence such as gRNA, wherein the first nickase is configured to recognize and bind a first site on a double-stranded polynucleotide, and to produce a first nick at a first distance therefrom; and (b) a second nickase bound to a second non-naturally occurring guide sequence such as gRNA, wherein the second nickase is configured to recognize and bind a second site on the double-stranded polynucleotide, and to produce a second nick at a second distance therefrom, wherein the first and second nickase together produce a cleaved polynucleotide fragment having an overhang defined by the first nick and the second nick, wherein the overhang is predesigned by selecting the first and second site.

In some embodiments of the method and composition of the present disclosure, the first nickase or the second nickase each comprises one or more of: Cas9 fused to a nuclease via a linker at the N terminus ("fCas9"), Cas9 fused to a nuclease via a linker at the C terminus ("Cas9f'), RISC complexed with or fused to a nuclease, transcription activator-like effector (TALE) complexed with or fused to a nuclease, zinc-finger complexed with or fused to a nuclease, meganuclease, and any combination thereof. The Cas9 may be catalytically inactive. The nuclease may be incapable of binding to DNA. The nuclease can be any suitable type IIS restriction endonuclease, such as FokI, BsaI, AlwI, and BfilI. In one example, the nuclease is FokI. The FokI may be a catalytically inactive monomer of FokI cleavage domain which may dimerize with a catalytically active monomer of FokI cleavage domain. The FokI can also be a catalytically active monomer of FokI cleavage domain and can dimerize with a catalytically active or inactive monomer of FokI cleavage domain. This way, the first nickase or the second nickase can be a dimer. In some embodiments of the disclosure, the first nickase or the second nickase is a heterodimer. In certain embodiments of the disclosure, in the first nickase, the Cas9 or RISC is directed by a first guide sequence such as gRNA to the first site, wherein the first guide sequence such as gRNA comprises a first sequence that is complementary to the first site. In some embodiments of the disclosure, in the second nickase, the Cas9 or RISC is directed by a second guide sequence such as gRNA to the second site, wherein the second guide sequence such as gRNA comprises a second sequence that is complementary to the second site. The first and second guide sequences, in various embodiments of the disclosure, are non-naturally occurring. In one embodiment of the disclosure, the first nickase and the second nickase nick at a predetermined position upstream or downstream to the first site and the second site, respectively, to produce the first nick and the second nick, respectively. The first and second sites may be selected such that the first nick and the second nick are offset by a predefined number of nucleotides.

A further composition provided by the present disclosure comprises: (a) a first nickase bound to a non-naturally occurring guide sequence such as gRNA, wherein the first nickase is configured to recognize and bind a first site on a double-stranded polynucleotide, and to produce a first nick at a first distance therefrom; and (b) a second nickase configured to recognize and bind a second site on the double-stranded polynucleotide, and to produce a second nick at a second distance therefrom, wherein the first and second nickase together produce a cleaved polynucleotide fragment having an overhang defined by the first nick and the second nick, wherein the overhang is predesigned by selecting the first and second site. In some embodiments of the disclosure, the first nickase comprises one or more of: Cas9 fused to a nuclease via a linker at the N terminus ("fCas9"), Cas9 fused to a nuclease via a linker at the C terminus ("Cas9f'), RISC complexed with or fused to a nuclease, and any combination thereof. The second nickase may comprise one or more of: transcription activator-like effector (TALE) complexed with or fused to a nuclease, zinc-finger complexed with or fused to a nuclease, meganuclease, and any combination thereof.

A method for nucleic acid assembly is also disclosed, comprising: producing the cleaved polynucleotide fragment according to the above method and/or using the above compositions, and assembling the cleaved polynucleotide fragment with another polynucleotide. In some embodiments of the disclosure, the assembling step comprises ligating the cleaved polynucleotide fragment with another polynucleotide having a complementary overhang to the overhang of the cleaved polynucleotide fragment. The assembling can also comprise polymerase assembly. In various embodiments of the disclosure, the polynucleotide is provided on a solid support, which may be, for example, an array or a bead. The method for nucleic acid assembly may further comprise releasing the ligated product from the solid support.

### BRIEF DESCRIPTION OF THE FIGURES

FIG. 1A illustrates an exemplary fusion protein according to a non-limiting embodiment of the disclosure.
FIG. 1B illustrates an exemplary fusion protein according to a non-limiting embodiment of the disclosure.
FIG. 1C illustrates an exemplary fusion protein according to a non-limiting embodiment of the disclosure.
FIG. 1D illustrates an exemplary fusion protein according to a non-limiting embodiment of the disclosure.
FIG. 2A and FIG. 2B illustrate an exemplary method for the synthesis of a cleaved DNA sequence according to a non-limiting embodiment of the disclosure.
FIG. 3A and FIG. 3B illustrate an exemplary method for the synthesis of a nicked DNA sequence using a mutant FokI-bottom with the fusion protein illustrated in FIG. 1A according to a non-limiting embodiment of the disclosure.
FIG. 3C and FIG. 3D illustrate an exemplary method for the synthesis of a cleaved DNA sequence using a mutant FokI-bottom with the fusion protein illustrated in FIG. 1B according to a non-limiting embodiment of the disclosure.
FIG. 4A and FIG. 4B illustrate an exemplary method for the synthesis of a nicked DNA sequence using a mutant FokI-bottom with the fusion protein illustrated in FIG. 1A according to a non-limiting embodiment of the disclosure.
FIG. 4C and FIG. 4D illustrate an exemplary method for the synthesis of a cleaved DNA sequence using a mutant Fokl-top with the fusion protein illustrated in FIG. 1C according to a non-limiting embodiment of the disclosure.
FIG. 5A and FIG. 5B illustrate an exemplary method for the synthesis of a cleaved DNA sequence using the two fusion proteins illustrated in FIG. 1D according to a non-limiting embodiment of the disclosure.
FIG. 6A illustrates an exemplary method for the synthesis of DNA sequences according to a non-limiting embodiment of the disclosure.
FIG. 6B illustrates an exemplary method for the synthesis of extended DNA sequences according to a non-limiting embodiment of the disclosure.
FIG. 7 illustrates an exemplary method of the disclosure for preparing oligonucleotides for assembly into a target polynucleotide comprising cleaving an amplification site from the oligonucleotides in a single cleavage step to produce a blunt double strand terminus.
FIGS. 8A and 8B illustrate an exemplary method of the disclosure for preparing oligonucleotides for assembly into a target polynucleotide comprising cleaving an amplification site from the oligonucleotides in two nicking steps to produce a single-stranded overhanging terminus.
FIGS. 9A and 9B illustrate an exemplary method of the disclosure for preparing oligonucleotides for assembly into a target polynucleotide comprising cleaving an amplification site from the oligonucleotides in two nicking steps to produce a single-stranded overhanging terminus.

### DETAILED DESCRIPTION OF THE DISCLOSURE

Aspects of the disclosure relate to compositions and methods for the production of site-directed nicked or cleaved DNA. Aspects of the disclosure further relate to compositions and methods for assembling a polynucleotide from oligonucleotides that have been subject to site-directed nicking or cleaving.

### Definitions

As used herein, "clustered regularly interspaced short palindromic repeats" or "CRISPRs" are DNA loci containing short repetitions of base sequences. CRISPRs play a functional role in phage defense in prokaryotes. Briefly, CRISPRs work as follows. When exposed to a phage infection or invasive genetic element, some members of the bacterial population incorporate short sequences from the foreign DNA ("spacers") between repeated sequences within the CRISPR locus. The combined unit of repeats and spacers in tandem is referred to as the "CRISPR array." The CRISPR array is transcribed and then processed into short crRNAs (CRISPR RNAs) each containing a single spacer and flanking repeated sequences. Spacers are derived from foreign DNA (which contains corresponding protospacers that can base pair with the spacers) and are generally stably inherited by daughter cells such that when later exposed to a phage or invasive DNA element with the same sequence, the strain is resistant to infection. CRISPRs are known to operate in conjunction with cognate Cas (CRISPR associated) protein(s) that show specificity to the repeat sequences separating the spacers. The Cas protein(s) operate in conjunction with the crRNA to mediate the cleavage of incoming foreign DNA where the crRNA forms an effector complex with the Cas proteins and guides the complex to the foreign DNA, which is then cleaved by the Cas proteins. There are several pathways of CRISPR activation, one of which requires a tracrRNA (trans-activating crRNA, also transcribed from the CRISPR array) which plays a role in the maturation of crRNA. Then a crRNA/tracrRNA hybrid forms and acts as a guide for the Cas9 to the foreign DNA.

As used herein, "Cas9" (CRISPR associated protein 9) is an RNA-guided DNA nuclease enzyme that can induce site-directed double strand breaks in DNA. In some embodiments of the disclosure, Cas9 can include at least one mutation (e.g., D10A) that renders Cas9 a nickase that nicks a single strand on DNA. In some embodiments of the disclosure, Cas9 can include at least two mutations (e.g., D10A and H840A) that render Cas9 catalytically inactive, and is referred to as "dCas9."

As used herein, "cleavage" or "cleave" refers to cutting a double stranded DNA, resulting in two DNA molecules having blunt or sticky ends.

The term "complementary" means that two nucleic acid sequences are capable of at least partially base-pairing according to the standard Watson-Crick complementarity rules. For example, two sticky ends can be partially complementary, wherein a region of one overhang complements and anneals with a region or all of the other overhang. The gap(s) can be filled in by chain extension in the presence of a polymerase and single nucleotides, followed by or simultaneously with a ligation reaction.

As used herein, a "dimer" is a macromolecular complex formed by two, non-covalently bound, macromolecules. As used herein, a "homodimer" is formed by two identical molecules. As used herein, a "heterodimer" is formed by two non-identical molecules. In some embodiments of the disclosure, a heterodimer of the present disclosure can be FokI:FokI*, where FokI* contains at least one mutation (e.g., D450A for full-length FokI or D69A for the FokI fragment). The mutation may render FokI catalytically inactive. In some embodiments of the disclosure, one or both of FokI and FokI* can be in the form of a fusion protein where it is fused to, for example, dCas9. In one example, a dimer such as a heterodimer of the present disclosure can be a fusion protein, e.g., dCas9-FokI or dCas9-FokI*, complexed with FokI or FokI*. The dimer of the present disclosure may be an obligate dimer or a non-obligate dimer. As used herein, an "obligate dimer" can be a homodimer or a heterodimer, and can only exist associated to each other and is not found in the monomeric state. A "non-obligate dimer" can be a homodimer or a heterodimer, and can exist in the monomeric state.

As used herein, "FokI" refers to an enzyme naturally found in *Flavobacterium okeanokoites.* See, for example, Kita et al., "The FokI Restriction-Modification System," The Journal of Biological Chemistry, Vol. 264, No. 10, pp. 5751-56 (part I) (1989), and Sugisaki, et al., "The FokI Restriction-Modification System," The Journal of Biological Chemistry, Vol. 264, No. 10, pp. 5757-5761 (part II) (1989). FokI is a type IIS restriction endonuclease including an N-terminal DNA-binding domain and a non-specific DNA cleavage domain at the C-terminal. Once the protein is bound to duplex DNA via its DNA-binding domain at the recognition site, the DNA cleavage domain is activated and cleaves, without further sequence specificity, the first strand 9 nucleotides downstream and the second strand 13 nucleotides upstream of the nearest nucleotide of the recognition site. In some embodiments of the disclosure, FokI is a full-length protein and is composed of 587 amino acids. In some embodiments of the disclosure, FokI is a partial protein and is composed of less than 587 amino acids. In an embodiment of the disclosure, FokI is a partial protein as in SEQ ID NO.:4. In some embodiments of the disclosure, FokI is wild type. In some embodiments of the disclosure, FokI contains at least one mutation ("FokI*"). In some embodiments of the disclosure, the mutation is D450A. In some embodiments of the disclosure, the mutation is D69A as in the partial FokI sequence of SEQ ID NO.:5.

As used herein, a "fusion protein" or "chimeric protein" is a protein generated through the joining of two or more genes or parts of genes (e.g., fragments) that originally code for two or more separate proteins. In some embodiments of the disclosure, the fusion protein further contains a linker such as XTEN. In some embodiments of the disclosure, a fusion protein includes Cas9 and FokI fused together, optionally via a linker. The Cas9 may be catalytically inactive (e.g., dCas9). The FokI may be catalytically active (e.g., wild type) or catalytically inactive (e.g., containing a mutation (e.g., D450A or D69A)). In some embodiments, the fusion protein binds to a guide RNA. In some embodiments of the disclosure, the fusion protein binds to a specific DNA sequence through, e.g., the guide RNA. In some embodiments of the disclosure, the fusion protein includes a nuclear localization sequence ("NLS"). In some embodiments of the disclosure, the fusion protein binds to FokI and forms a dimer. In some embodiments of the disclosure, the FokI bound to the fusion protein is wild type. In some embodiments of the disclosure, the bound FokI is catalytically inactive. In some embodiments of the disclosure, FokI is full-length. In some embodiments of the disclosure, FokI is a protein fragment.

A "guide sequence" can be any synthetic, non-naturally occuring DNA (double or single stranded), RNA, or other artificial nucleic acid sequence such as peptide nucleic acid (PNA), morpholino and locked nucleic acid (LNA), glycol nucleic acid (GNA) and threose nucleic acid (TNA) that is capable of guiding a protein of interest to a specific sequence by way of complementarity. In certain embodiments of the disclosure, the guide sequence is gRNA.

As used herein, "guide RNA" or "gRNA" represents a synthetic, non-naturally occurring RNA molecule capable of guiding a protein of interest to a specific sequence by way of complementarity. In certain embodiments of the disclosure, the gRNA may be a single hybrid hairpin guide RNA which is a designed to mimic the crRNA:tracrRNA complex to load Cas9 for sequence-specific DNA cleavage or nicking. In some embodiments of the disclosure, gRNA can guide and/or localize a Cas9 protein or a Cas9-nuclease fusion protein to a DNA sequence that is complementary to the gRNA or a partial sequence thereof. In additional embodiments of the disclosure, the gRNA can be a small interfering RNA (siRNA) or microRNA (miRNA), which can bind and direct RISC (RNA-induced silencing complex) to specific sequence of interest.

As used herein, a "linker" is a synthetic (e.g., peptide) sequence or non-peptide moiety that occurs between and physically links two peptide sequences (e.g., protein domains). The peptide sequence can be a full-length protein or a protein fragment, or a peptide. The linker may be positioned between NLS and FokI, and/or between FokI and dCas9. In an embodiment of the disclosure, a linker is used to generate a fusion protein of the present disclosure. In an embodiment of the disclosure, FokI-L8 is used to generate a fusion protein of the present disclosure (e.g., fCas9).

As used herein, "nicking" refers to cutting a single strand (P or N) of a double stranded DNA sequence.

As used herein, a "nickase" is a protein configured to cut a single strand of a double stranded DNA sequence. In some embodiments of the disclosure, a nickase is a fusion protein bound to FokI or FokI* in the form of a heterodimer. In some embodiments of the disclosure, a nickase is a fusion protein bound to an identical nickase, forming a homodimer. In some embodiments of the disclosure, the fusion protein is fCas9 where Cas9 is fused to FokI at the N terminus, optionally via a linker. In some embodiments of the disclosure, the fusion protein is Cas9f where Cas9 is fused to FokI at the C terminus, optionally via a linker. In some embodiments of the disclosure, the Cas9 domain of the fusion protein is catalytically inactive. In some embodiments of the disclosure, the fusion protein contains catalytically active FokI. In some embodiments of the disclosure, the fusion protein contains catalytically inactive FokI. In certain embodiments of the disclosure, the nickase is fCas9 or Cas9f bound to FokI. In some embodiments of the disclosure, the bound FokI is the full-length endonuclease. In some embodiments of the disclosure, the bound FokI is a fragment of the endonuclease. In some embodiments of the disclosure, the bound FokI contains a mutation (e.g., D450A or D69A) that renders the bound FokI catalytically inactive. In some embodiments of the disclosure, the bound FokI is catalytically active. The nickase can also be a TALEN (transcription activator-like effector nuclease), ZFN (zinc-finger nuclease) and/or meganuclease, or a monomer thereof. Exemplary TALENs and ZFNs are reviewed in Joung, et al., "TALENs: a widely applicable technology for targeted genome editing," Nat. Rev. Mol. Cell Biol. 14, 49-55 (2012) and Urnov, et al., "Genome editing with engineered zinc finger nucleases," Nat. Rev. Genet. 11, 636-646 (2010). Exemplary meganucleases are reviewed in Silva et al., "Meganucleases and Other Tools for Targeted Genome Engineering: Perspectives and Challenges for Gene Therapy," Curr. Gene Ther. Feb 2011; 11(1): 11-27.

As used herein, an "oligonucleotide" may be a nucleic acid molecule comprising at least two covalently bonded nucleotide residues. The terms "oligonucleotide', "polynucleotide" and "nucleic acid" are used interchangeably. In some embodiments of the disclosure, an oligonucleotide may be between 10 and 50,000 nucleotides long. In some embodiments of the disclosure, an oligonucleotide may be between 50 and 10,000 nucleotides long. In some embodiments of the disclosure, an oligonucleotide may be between 100 and 1,000 nucleotides long. For example, an oligonucleotide may be between 10 and 500 nucleotides long, or between 500 and 1,000 nucleotides long. In some embodiments of the disclosure, an oligonucleotide may be between about 20 and about 300 nucleotides long (e.g., from about 30 to 250, 40 to 220, 50 to 200, 60 to 180, or about 65 or about 150 nucleotides long), between about 100 and about 200, between about 200 and about 300 nucleotides, between about 300 and about 400, or between about 400 and about 500 nucleotides long. However, shorter or longer oligonucleotides may be used. An oligonucleotide may be a single-stranded nucleic acid. However, in some embodiments of the disclosure a double-stranded oligonucleotide may be used as described herein. In certain embodiments of the disclosure, an oligonucleotide may be chemically synthesized as described in more detail below. In some embodiments of the disclosure, nucleic acids (e.g., synthetic oligonucleotides) may be amplified before use. The resulting product may be double-stranded. Oligonucleotides can be DNA, RNA and/or other naturally or non-naturally occurring nucleic acids. One or more modified bases (e.g., a nucleotide analog) can be incorporated. Examples of modifications include, but are not limited to, one or more of the following: methylated bases such as cytosine and guanine; universal bases such as nitro indoles, dP and dK, inosine, uracil; halogenated bases such as BrdU; fluorescent labeled bases; non-radioactive labels such as biotin (as a derivative of dT) and digoxigenin (DIG); 2,4-Dinitrophenyl (DNP); radioactive nucleotides; post-coupling modification such as dR-NH2 (deoxyribose-NEb); Acridine (6-chloro-2-methoxiacridine); and spacer phosphoramides which are used during synthesis to add a spacer "arm" into the sequence, such as C3, C8 (octanediol), C9, C12, HEG (hexaethlene glycol) and C18.

As used herein, the term "vector" refers to any genetic element, such as a plasmid, phage, transposon, cosmid, chromosome, artificial chromosome, episome, virus, virion, etc., capable of replication when associated with the proper control elements and which can transfer gene sequences into or between cells. The vector may contain a selection module suitable for use in the identification of transformed or transfected cells. For example, selection modules may provide antibiotic resistant, fluorescent, enzymatic, as well as other traits. As a second example, selection modules may complement auxotrophic deficiencies or supply critical nutrients not in the culture media.

"A plurality" means more than 1, e.g., 2, 3, 4, 5, 6, 7, 8, 9, 10, 11, 12, 13, 14, 15, 16, 17, 18, 19, 20, or more, e.g., 25, 30, 40, 50, 60, 70, 80, 90, 100, 200, 300, 400, 500, or more, or any integer in between.

As used herein, the term "about" means within 20%, more preferably within 10% and most preferably within 5%. The term "substantially" means more than 50%, preferably more than 80%, and most preferably more than 90% or 95%.

Other terms used in the fields of recombinant nucleic acid technology, synthetic biology, and molecular biology as used herein will be generally understood by one of ordinary skill in the applicable arts.

### Synthetic Oligonucleotides

Typically, oligonucleotide synthesis involves a number of chemical steps that are performed in a cycle repetitive manner throughout the synthesis with each cycle adding one nucleotide to the growing oligonucleotide chain. The chemical steps involved in a cycle are a deprotection step that liberates a functional group for further chain elongation, a coupling step that incorporates a nucleotide into the oligonucleotide to be synthesized, and other steps as required by the particular chemistry used in the oligonucleotide synthesis, such as e.g. an oxidation step required with the phosphoramidite chemistry. Optionally, a capping step that blocks those functional groups which were not elongated in the coupling step can be inserted in the cycle. The nucleotide can be added to the 5'-hydroxyl group of the terminal nucleotide, in the case in which the oligonucleotide synthesis is conducted in a 3'→5" direction' or at the 3'-hydroxyl group of the terminal nucleotide' in the case in which the oligonucleotide synthesis is conducted in a 5'→3' direction.

For clarity, the two complementary strands of a double stranded nucleic acid are referred to herein as the positive (P) and negative (N) strands. This designation is not intended to imply that the strands are sense and anti-sense strands of a coding sequence. They refer only to the two complementary strands of a nucleic acid (e.g., a target nucleic acid, an intermediate nucleic acid fragment, etc.) regardless of the sequence or function of the nucleic acid. Accordingly, in some embodiments of the disclosure the P strand may be a sense strand of a coding sequence, whereas in other embodiments of the disclosure the P strand may be an anti-sense strand of a coding sequence. It should be appreciated that the reference to complementary nucleic acids or complementary nucleic acid regions herein refers to nucleic acids or regions thereof that have sequences which are reverse complements of each other so that they can hybridize in an antiparallel fashion typical of natural DNA.

In some aspects of the disclosure, the oligonucleotides synthesized or otherwise prepared according to the methods described herein can be used as building blocks for the assembly of a target polynucleotide of interest.

Oligonucleotides may be synthesized on solid support. As used herein, the term "solid support", "support" and "substrate" are used interchangeably and refers to a porous or non-porous solvent insoluble material on which polymers such as nucleic acids are synthesized or immobilized. As used herein "porous" means that the material contains pores having substantially uniform diameters (for example in the nm range). Porous materials can include' but are not limited to, paper, synthetic filters and the like. In such porous materials, the reaction may take place within the pores. The support can have any one of a number of shapes, such as pin, strip, plate, disk, rod, bends, cylindrical structure, particle, including bead, nanoparticle and the like. The support can have variable widths.

The support can be hydrophilic or capable of being rendered hydrophilic. The support can include inorganic powders such as silica, magnesium sulfate, and alumina; natural polymeric materials, particularly cellulosic materials and materials derived from cellulose, such as fiber containing papers, e.g., filter paper, chromatographic paper, etc.; synthetic or modified naturally occurring polymers, such as nitrocellulose, cellulose acetate, poly (vinyl chloride), polyacrylamide, cross linked dextran, agarose, polyacrylate, polyethylene, polypropylene, poly (4-methylbutene), polystyrene, polymethacrylate, poly(ethylene terephthalate), nylon, poly(vinyl butyrate), polyvinylidene difluoride (PVDF) membrane, glass, controlled pore glass, magnetic controlled pore glass, ceramics, metals, and the like; either used by themselves or in conjunction with other materials.

In some embodiments of the disclosure, pluralities of different single-stranded oligonucleotides are immobilized at different features of a solid support. In some embodiments of the disclosure, the support-bound oligonucleotides may be attached through their 5' end or their 3' end. In some embodiments of the disclosure, the support-bound oligonucleotides may be immobilized on the support via a nucleotide sequence (e.g. degenerate binding sequence), or a linker (e.g. photocleavable linker or chemical linker). It should be appreciated that by 3' end, it is meant the sequence downstream to the 5' end and by 5' end it is meant the sequence upstream to the 3' end. For example, an oligonucleotide may be immobilized on the support via a nucleotide sequence or linker that is not involved in subsequent reactions.

Certain embodiments of the disclosure may make use of a solid support comprised of an inert substrate and a porous reaction layer. The porous reaction layer can provide a chemical functionality for the immobilization of pre-synthesized oligonucleotides or for the synthesis of oligonucleotides. In some embodiments of the disclosure, the surface of the array can be treated or coated with a material comprising suitable reactive groups for the immobilization or covalent attachment of nucleic acids. Any material, known in the art, having suitable reactive groups for the immobilization or in situ synthesis of oligonucleotides can be used.

In some embodiments of the disclosure, the porous reaction layer can be treated so as to comprise hydroxyl reactive groups. For example, the porous reaction layer can comprise sucrose.

According to some aspects of the disclosure, oligonucleotides terminated with 3' phosphoryl group oligonucleotides can be synthesized in a 3'→5' direction on a solid support having a chemical phosphorylation reagent attached to the solid support. In some embodiments of the disclosure, the phosphorylation reagent can be coupled to the porous layer before synthesis of the oligonucleotides. In an exemplary embodiment of the disclosure, the phosphorylation reagent can be coupled to the sucrose. For example, the phosphorylation reagent can be 2-[2-(4,4'-Dimethoxytrityloxy)ethylsulfonyl]ethyl-(2-cyanoethyl)-(N,N-diisopropyl)-phosphoramidite. In some embodiments of the disclosure, the 3' phosphorylated oligonucleotide can be released from the solid support and undergo subsequent modifications according to the methods described herein. In some embodiments of the disclosure, the 3' phosphorylated oligonucleotide can be released from the solid support using ammonium hydroxide.

In some embodiments of the disclosure, synthetic oligonucleotides for the assembly may be designed (e.g. sequence, size, and number). Synthetic oligonucleotides can be generated using standard DNA synthesis chemistry (e.g. phosphoramidite method). Synthetic oligonucleotides may be synthesized on a solid support, such as for example a microarray, using any appropriate technique as described in more detail herein. Oligonucleotides can be eluted from the microarray prior to be subjected to amplification or can be amplified on the microarray. It should be appreciated that different oligonucleotides may be designed to have different lengths.

In some embodiments of the disclosure, oligonucleotides are synthesized (e.g., on an array format) as described in U.S. Patent No. 7,563,600, U.S. Patent Application Ser. No. 13/592,827, and PCT/US2013/047370 published as WO 2014/004393. For example, single-stranded oligonucleotides are synthesized in situ on a common support wherein each oligonucleotide is synthesized on a separate or discrete feature (or spot) on the substrate. In some embodiments of the disclosure, single-stranded oligonucleotides are bound to the surface of the support or feature. As used herein, the term "array" refers to an arrangement of discrete features for storing, routing, amplifying and releasing oligonucleotides or complementary oligonucleotides for further reactions. In an embodiment of the disclosure, the support or array is addressable: the support includes two or more discrete addressable features at a particular predetermined location (i.e., an "address") on the support. Therefore, each oligonucleotide molecule of the array is localized to a known and defined location on the support. The sequence of each oligonucleotide can be determined from its position on the support. Moreover, addressable supports or arrays enable the direct control of individual isolated volumes such as droplets. The size of the defined feature can be chosen to allow formation of a microvolume droplet on the feature, each droplet being kept separate from each other. As described herein, features are typically, but need not be, separated by interfeature spaces to ensure that droplets between two adjacent features do not merge. Interfeatures will typically not carry any oligonucleotide on their surface and will correspond to inert space. In some embodiments of the disclosure, features and interfeatures may differ in their hydrophilicity or hydrophobicity properties.

In various embodiments of the disclosure, the synthetic single-stranded or double-stranded oligonucleotides can be non-naturally occurring, e.g., being unmethylated or modified in a way (e.g., chemically or biochemically modified in vitro) such that they become hemi-methylated (only one strand is methylated) or semi-methylated (only a portion of the normal methylation sites are methylated on one or both strands) or hypomethylated (more than the normal methylation sites are methylated on one or both strands), or have non-naturally occurring methylation patterns (some of the normal methylation sites are methylated on one or both strands and/or normally unmethylated sites are methylated). In contrast, naturally-occurring DNA typically contains epigenetic modifications such as methylation at, e.g., the C-5 position of the cytosine ring of DNA by DNA methyltransferases (DNMTs) in vivo. DNA methylation is reviewed by Jin et al., Genes & Cancer 2011 Jun; 2(6): 607-617.

### Site-directed Nicking and Cleaving

In some embodiments of the disclosure, compositions and methods for site-directed nicking and/or cleaving are disclosed. One exemplary composition includes a fusion protein comprising a catalytically inactive Cas9 fused directly or indirectly to the catalytic domain of a nuclease. The nuclease catalytic domain may be, for example, the cleaving domain of an endonuclease. In some aspects of the disclosure, the endonuclease may be a restriction endonuclease, including, for example a type IIS restriction endonuclease. Embodiments of the disclosure include endonucleases that are catalytically active in a dimeric or multimeric form, including, without limitation, FokI, AlwI, and BfilI. The nuclease catalytic domain may include a mutation that modifies the cleavage activity. For example, a catalytic domain may include a modification that renders the nuclease catalytic domain a nickase, e.g., one that cleaves only one strand of a double stranded oligonucleotide. In embodiments of the disclosure where the catalytic domain functions in a dimeric or multimeric form, the catalytic domain may include a mutation on fewer than all of the monomers that make up the dimer or multimers, and/or two or more monomers may include different mutations.

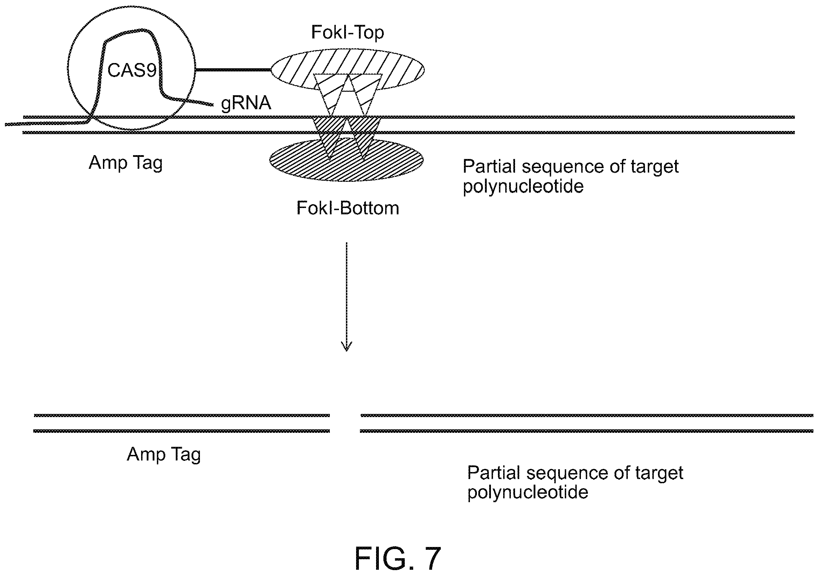

As shown in FIGS. 7-9, aspects of the disclosure relate to compositions and systems for site-directed nicking and cleaving of synthetic oligonucleotides, comprising (a) a fusion protein comprising a Cas9 linked, directly or indirectly, to one or more monomers of a dimeric or multimeric catalytic domain of a nuclease; and (b) a second or more such monomers that are not linked to the same Cas9 as the Cas9-linked monomers. Such second monomer may be linked to another protein (including, for example, a second Cas9) or stand-alone. Components (a) and (b) can bind or complex with each other, forming a dimer (homodimer or heterodimer) that has nuclease activity. According to an embodiment of the disclosure, such compositions and systems may further comprise one or more gRNAs bound to the Cas9; and may further comprise one or more oligonucleotides having a region that is complementary to the gRNA sequence or a part thereof. The gRNA sequence may be naturally occurring or non-naturally occurring and designed to be complementary to a portion of a target oligonucleotide to be nicked or cleaved. The dimer:gRNA complex, by way of annealing of the gRNA to the target oligonucleotide, can bind thereto and exercise its nuclease activity.

In some embodiments of the disclosure, a plurality of oligonucleotides each having a region that is complementary to or is the same as the gRNA can be included, wherein the plurality of oligonucleotides together comprise a target polynucleotide to be assembled from the plurality of oligonucleotides. According to one embodiment of the disclosure, each of the plurality of oligonucleotides can have a flanking region on the 3' terminus, 5' terminus, or both termini. The flanking region can include a primer site for PCR amplification and/or a recognition region complementary to the gRNA sequence. The primer site may be or include, in whole or in part, the recognition sequence. The plurality of oligonucleotides may together comprise the target polynucleotide with or without the flanking regions.

It should be noted that one or more primers used herein can be methylated such that the amplified product can be digested with a methylation-sensitive nuclease such as MspJI, SgeI and FspEI. Such nuclease shares both type IIM and type IIS properties; thus, it only recognizes the methylation-specific 4-bp sites, ^{m}CNNR (N = A or T or C or G; R =A or G), and cuts DNA outside of this recognition sequences. Methylated primers and use thereof are disclosed in Chen et al., Nucleic Acids Research, 2013, Vol. 41, No. 8, e93.

According to one embodiment of the disclosure, a composition comprising a first Cas9-nuclease fusion protein bound to a first gRNA and a second Cas9-nuclease fusion protein bound to a second gRNA is provided. The first and second gRNAs can be different. In some embodiments of the disclosure, the first and second gRNA sequences are designed to guide the first and second Cas9-nuclease fusion proteins, respectively, to specific positions on a double-stranded DNA sequence, to perform site-directed DNA nicking or cleaving. For example, the first and second fusion proteins can be used to target and nick the P and N strand of the same oligonucleotide, respectively, at predetermined positions, thereby producing a predesigned sticky end. Alternatively, the first and second fusion proteins can be used to target and cleave double strands of different oligonucleotides, thereby producing two or more predesigned sticky ends. Additional different gRNAs (a third, fourth, fifth, etc. gRNA) may be employed to produce nicks at different sites or cuts on more oligonucleotides. In one example, the first and second gRNA sequences comprise sequences that are completely or partially complementary to each other and which may be employed in separate or the same nicking or cleaving step. The first and second gRNA sequences, in some embodiments of the disclosure, are not complementary.

The disclosure also discloses methods of using the compositions and systems described herein in applications of, for example, synthetic biology. For example, methods for nucleic acid synthesis and assembly using the compositions and systems disclosed herein are disclosed. According to one aspect of the disclosure, a plurality of oligonucleotides that together comprise a target polynucleotide are provided. Each of the plurality of oligonucleotides is designed to add a flanking region on one or both termini. The flanking regions can have a primer site completely or partially within, or outside, which is a recognition region for gRNA binding. The oligonucleotides may be amplified by a template-driven enzymatic reaction such as PCR using a primer against the primer site. Following amplification, the plurality of oligonucleotides (each comprising a P strand and a complementary N strand) can be contacted with a Cas9-nuclease fusion protein such as a catalytically inactive Cas9 fused to a first monomer of a type IIS endonuclease (e.g., FokI) catalytic domain. Bound to the Cas9 is a pre-designed, synthetic gRNA complementary to the recognition region of the P and/or the N strand of each of the plurality of oligonucleotides. The plurality of oligonucleotides are brought into contact with the gRNA and the Cas9-nuclease (e.g., Cas9-FokI) fusion protein in the presence of a second monomer of the nuclease (e.g., FokI) catalytic domain under conditions suitable for binding of the gRNA to the recognition region, as well as dimerization between the first nuclease monomer of the Cas9-nuclease fusion protein and the second nuclease monomer. Upon dimerization, the nicking or cleavage activity present in the Cas9-nuclease and/or the second nuclease monomer can act to nick or cleave the target oligonucleotide.

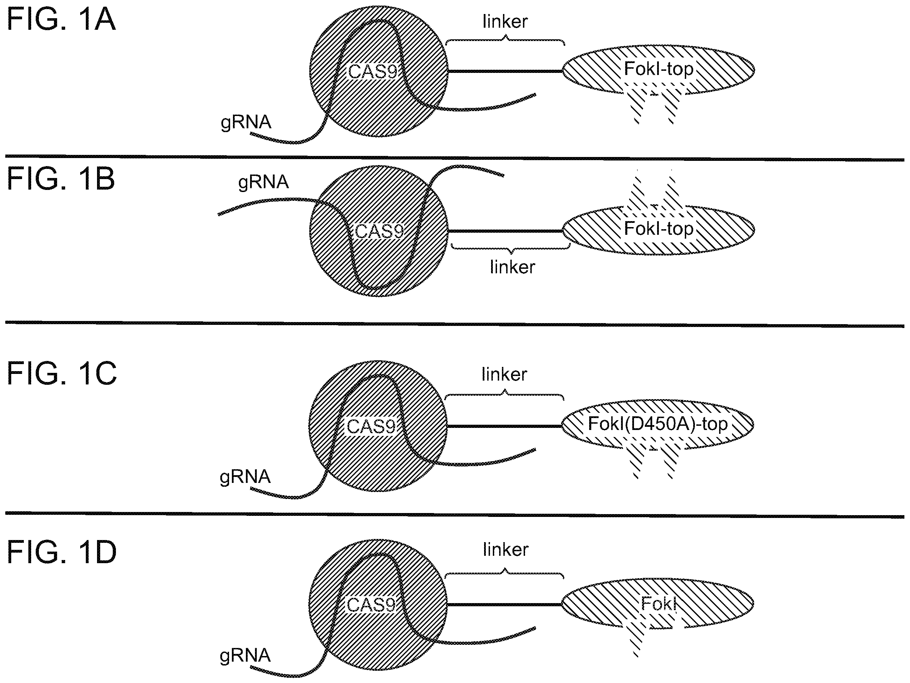

FIGS. 1A-1D depict a few exemplary fusion proteins, fCas9 or Cas9f, for use to nick and/or cleave a double stranded DNA sequence. For example, FIG. 1A illustrates a fusion protein fCas9' including Cas9 (e.g., dCas9)' linked at one terminus (N or C) to a monomer of a catalytic domain of FokI (e.g., wild type) by a linker sequence. The Cas9 can bind to a gRNA which anneals with a complementary sequence DNA at a specific position upstream of FokI. FIG. 1B depicts a fusion protein Cas9f in which Cas9 is linked at the opposite terminus to FokI such that when Cas9 binds a nucleic acid (via complementary gRNA) at a specific position, FokI is placed upstream to Cas9. FIG. 1C illustrates a fusion protein bound to a gRNA, in which Cas9 (e.g., dCas9) is linked by a linker sequence to a monomer of a catalytic domain of FokI that is a catalytically inactive mutant (e.g., D450A). The fusion proteins in FIGS. 1A - 1C contain a FokI portion that can dimerize with another FokI monomer (wild type or mutant) to form, for example, a heterodimer. FIG. 1D depicts a fusion protein bound to a gRNA, in which Cas9 (e.g., dCas9) is linked to FokI by a linker sequence. The fusion protein of FIG. 1D is capable of homo-dimerization.

In various embodiments of the disclosure, the gRNA used herein can be designed to contain a sequence that is complementary to the sequence of the desired binding site. As a result, the gRNA can specifically bind to the desired binding site under suitable conditions, directing fCas9 or Cas9f thereto. The FokI portion of fCas9 or Cas9f can then bind, e.g., nonspecifically, to the DNA molecule at a distance from the gRNA binding site. The geometry of fCas9 or Cas9f, e.g., the space between Cas9:gRNA and FokI may determine where FokI binds. The length and/or geometry of the linker can also affect FokI binding. In some embodiments of the disclosure, each specific Cas9-nuclease fusion protein may have a corresponding, specific position where FokI binds. For example, fusion protein 1 may have a FokI binding position that is X1 nucleotides from the gRNA binding site, fusion protein 2 may have a FokI binding position that is X2 nucleotides from the gRNA binding site, and so on. In certain embodiments of the disclosure, the FokI binding position is not fixed; rather, some flexibility (e.g., 1 or 2 or more nucleotides) can be present. For example, for the same fusion protein, FokI may bind at X nucleotides from the gRNA binding site in one reaction, and may bind at X+N (+ indicates downstream) or X-N (- indicates upstream) nucleotides from the gRNA binding site in another reaction, where N is 1 or 2 or 3 or more. After binding, the FokI region of fCas9 or Cas9f can dimerize with a second FokI (e.g., full-length or a fragment, catalytically active or inactive) and can nick or cleave the double stranded nucleic acid. In certain embodiments of the disclosure, FokI nicks or cleaves DNA without binding.

In some embodiments of the disclosure, the fusion protein can further include a nuclear localization sequence ("NLS") that locates the protein to the nucleus. In certain embodiments of the disclosure, a linker can be used to generate the fusion protein disclosed herein. The linker may be positioned between NLS and FokI, and/or between FokI and dCas9. Table 1 below identifies some non-limiting examples of such linkers. In an embodiment of the disclosure, FokI-L8 is used to generate a fusion protein of the present disclosure (e.g., fCas9).

**TABLE 1: Exemplary Linker Sequences**

| Name | NKS-linker-FokI | FokI-linker-dCas9 |
|---|---|---|
| FokI-(GGS)x3 | GGS | GGSGGSGGS (SEQ ID NO.:13) |
| FokI-(GGS)x6 | GGS | GGSGGSGGSGGSGGSGGS (SEQ ID NO.:14) |
| FokI-L0 | GGS | - |
| FokI-L1 | GGS | MKIIEQLPSA (SEQ ID NO.:15) |
| FokI-L2 | GGS | VRHKLKRVGS (SEQ ID NO.:16) |
| FokI-L3 | GGS | VPFLLEPDNINGKTC (SEQ ID NO.:17) |
| FokI-L4 | GGS | GHGTGSTGSGSS (SEQ ID NO.:18) |
| FokI-L5 | GGS | MSRPDPA (SEQ ID NO.:19) |
| FokI-L6 | GGS | GSAGSAAGSGEF (SEQ ID NO.:20) |
| FokI-L7 | GGS | SGSETPGTSESA (SEQ ID NO.:21) |
| FokI-L8 | GGS | SGSETPGTSESATPES (SEQ ID NO.:22) |
| FokI-L9 | GGS | SGSETPGTSESATPEGGSGGS (SEQ ID NO.:23) |
| NLS-(GGS) | GGS | GGSM |
| NLS-(GGS)x3 | GGSGGSGGS | GGSM |
| NLS-L1 | VPFLLEPDNINGKTC (SEQ ID NO.:17) | GGSM |
| NLS-L2 | GSAGSAAGSGEF (SEQ ID NO.:20) | GGSM |
| NLS-L3 | SIVAQLSRPDPA (SEQ ID NO.:24) | GGSM |
| Wild-type Cas9 | N/A | N/A |
| Cas9 nickase | N/A | N/A |

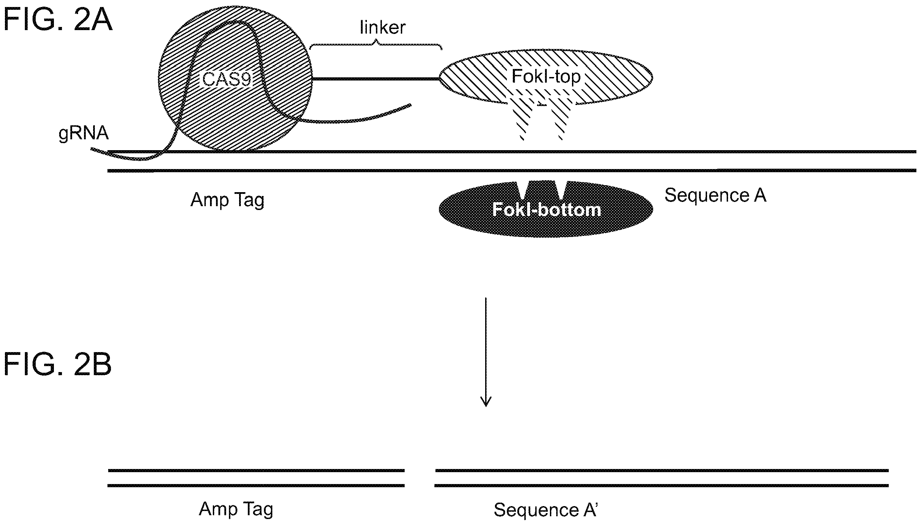

FIGS. 2A-2B depict a method of removing a double stranded "Amp tag" (primer sequence for amplification) from a double stranded sequence. FIG. 2A illustrates a fusion protein including dCas9, FokI, and a linker sequence therebetween (sometimes designated as "fCas9"), bound to at least one gRNA molecule that facilitates site-directed localization of the fusion protein to a specific location on a double stranded molecule comprising, e.g., Amp tag and Sequence A. In an embodiment of the disclosure, the fusion protein is Fokl-XTEN-dCas9, as provided in SEQ ID NO.: 12 (excluding NLS-GGS). After binding, the FokI region of fCas9 can dimerize with a second, catalytically active FokI and the resulting dimer can cleave the double stranded sequence, producing two double stranded segments as shown in FIG. 2B: the Amp tag and the desired DNA sequence (e.g., Sequence A') which can be further subject to additional assembly.

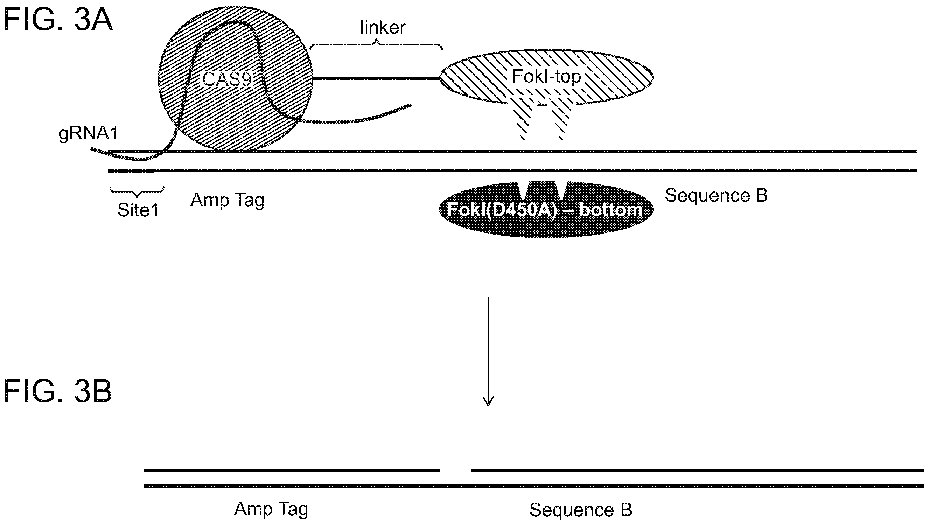

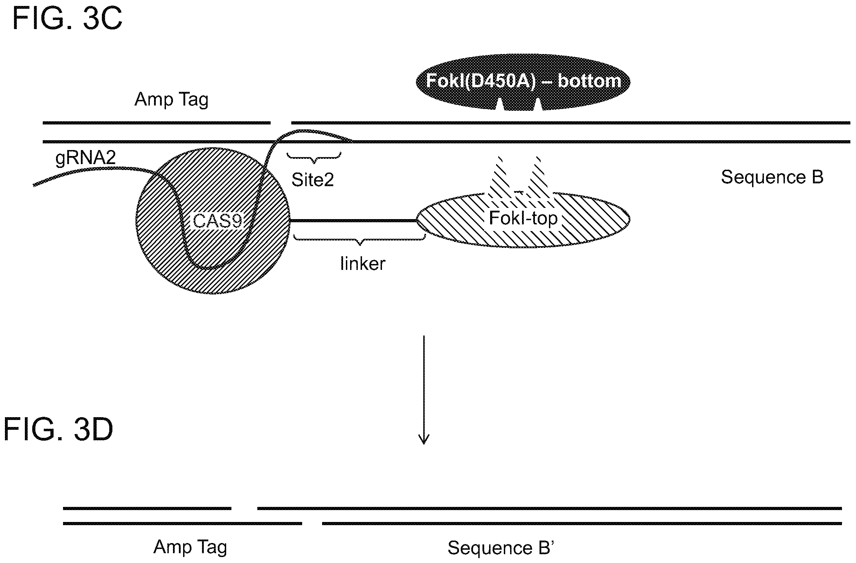

FIGS. 3A-3D illustrate a two-step method of removing an Amp tag from a double stranded sequence. The first step is shown in FIGS. 3A-3B, and the second step is shown in FIGS. 3C-3D. In FIG. 3A, a first fusion protein including dCas9, FokI, and a linker sequence therebetween ("fCas9"), bound to at least one gRNA molecule (e.g., gRNA1), is selectively localized to a specific location, site1, on a double stranded molecule comprising, e.g., Amp tag and Sequence B. gRNA1 contains sequence that is complementary to and anneals with the top strand in the Amp tag at site1. fCas9 is configured such that when bound to the top strand at site1, the FokI portion ("FokI-top") is positioned downstream to dCas9. In an embodiment of the disclosure, the fusion protein is FokI-XTEN-dCas9 as provided in SEQ ID NO.:12 (excluding NLS-GGS). The FokI-top of fCas9 dimerizes with a second FokI* (e.g., full length or fragment) which contains a mutation, rendering it nuclease-dead (e.g., "FokI (D450A)-bottom" and/or "FokI (D69A)-bottom"). The resulting fCas9:FokI* heterodimer nicks the top strand of the double stranded nucleic acid, producing a nicked molecule having a first nick on the top strand as shown in FIG. 3B. In the second step, a second fusion protein Cas9f:gRNA2, shown in FIG. 3C, is directed to the bottom strand of the nicked molecule at site2, by gRNA2 having a sequence complementary to site2. Cas9f is configured such that when bound to the bottom strand at site2, the FokI-top is positioned upstream to dCas9, while dimerizing with a catalytically inactive FokI* monomer (e.g., "FokI(D450A)-bottom" and/or "FokI (D69A)-bottom"). This Cas9f:FokI* heterodimer produces a second nick on the bottom strand. The net result is a double stranded break, with the Amp tag separated from Sequence B, producing Sequence B' (FIG. 3D) having a sticky end for further assembly or other manipulation. In some embodiments of the disclosure, site1 and site2 are designed in a way such that the sticky end in Sequence B' has a desired overhang with a predetermined length and sequence.

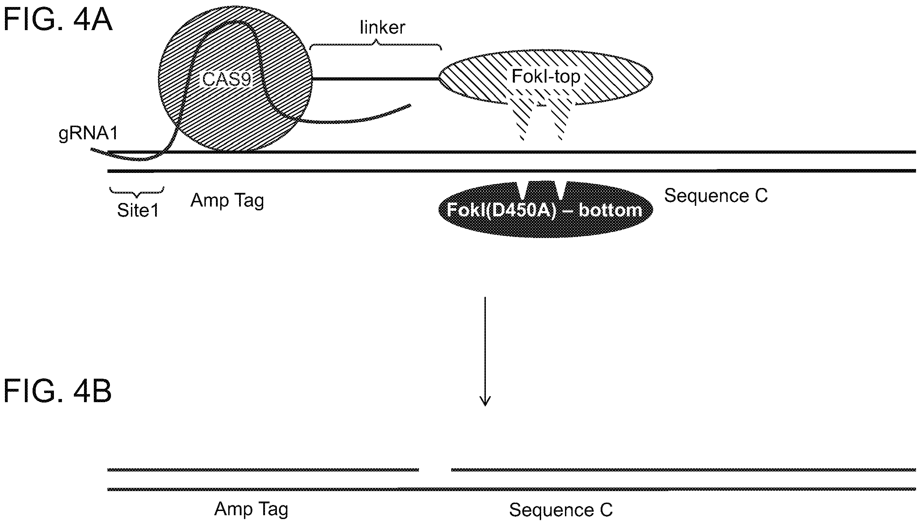

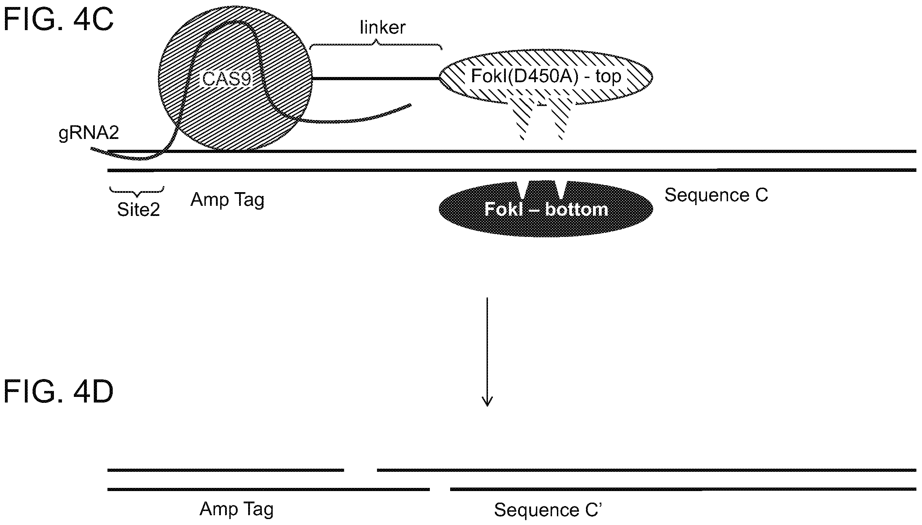

FIGS. 4A-4D illustrate another 2-step method of removing an Amp tag from a double stranded sequence. The first step is shown in FIGS. 4A-4B, and the second step is shown in FIGS. 4C-4D. In FIG. 4A, a first fusion protein including dCas9, FokI, and a linker sequence therebetween ("fCas9"), bound to at least one gRNA molecule (e.g., gRNA1), is selectively localized to a specific location, site1, on a double stranded molecule comprising, e.g., Amp tag and Sequence C. gRNA1 contains sequence that is complementary to and binds with the top strand in the Amp tag at site1. fCas9 is configured such that when bound to the top strand at site1, the FokI portion ("FokI-top") is downstream to dCas9. In an embodiment of the disclosure, the fusion protein is FokI-XTEN-dCas9 as provided in SEQ ID NO.:12 (excluding NLS-GGS). The FokI-top of fCas9 dimerizes with a second FokI* (e.g., full length or fragment) which contains a mutation, rendering it nuclease-dead (e.g., "FokI (D450A)-bottom" and/or "FokI (D69A)-bottom"). The resulting fCas9:FokI* heterodimer nicks the top strand of the double stranded nucleic acid, producing a nicked molecule having a first nick on the top strand as shown in FIG. 4B. In the second step, a second fusion protein fCas9*:gRNA2, shown in FIG. 4C, is directed to the top strand of the nicked molecule at site2, by gRNA2 having a sequence complementary to site2. fCas9* contains a mutant FokI* portion (e.g., "FokI(D450A)-top" and/or "FokI (D69A)-top") and is configured such that when bound to the top strand at site2, the FokI*-top is positioned downstream to dCas9, while dimerizing with a catalytically active FokI monomer (FokI-bottom). This fCas9*:FokI heterodimer produces a second nick on the bottom strand. The net result is a double stranded break, with the Amp tag separated from Sequence C, producing Sequence C' (FIG. 4D) having a sticky end for further assembly or other manipulation. In some embodiments of the disclosure, site1 and site2 are designed in a way such that the sticky end in Sequence C' has a desired overhang with a predetermined length and sequence.

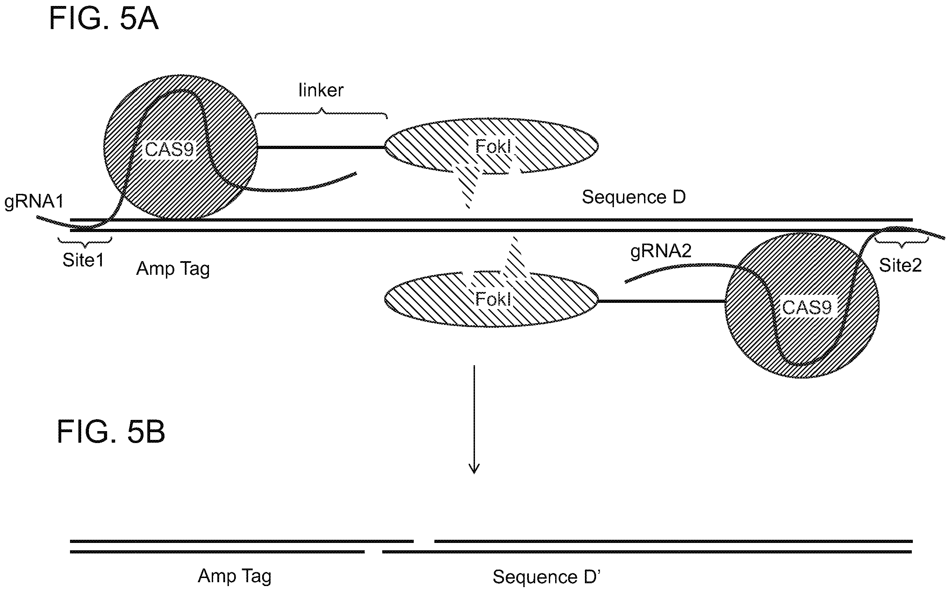

FIGS. 5A and 5B depict a further method of removing an Amp tag from a double stranded sequence. FIG. 5A illustrates a first and second fusion protein, each including Cas9, FokI, and a linker sequence ("fCas9"), bound to gRNA1 and gRNA2, respectively, which direct selective localization of the first and second fusion proteins to specific locations, site1 and site2, respectively, on a double stranded molecule comprising, e.g., Amp tag and Sequence D. In an embodiment of the disclosure, each of the first and second fusion protein is FokI-XTEN-dCas9, as provided in SEQ ID NO.:12 (excluding NLS-GGS). Site1 and site2 are designed such that when the first and second fusion proteins are localized, the two FokI regions of fCas9 can contact and dimerize with each other to act as a catalytically active endonuclease. As such, the fCas9:fCas9 homodimer can cleave the double stranded molecule, separating the Amp tag and producing Sequence D' (FIG. 4D) having a sticky end for further assembly or other manipulation.

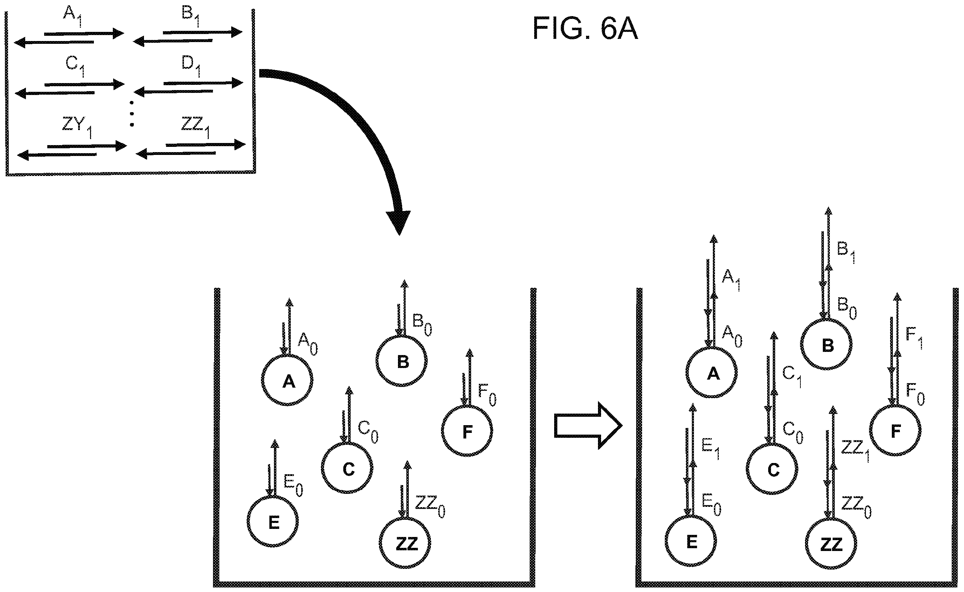

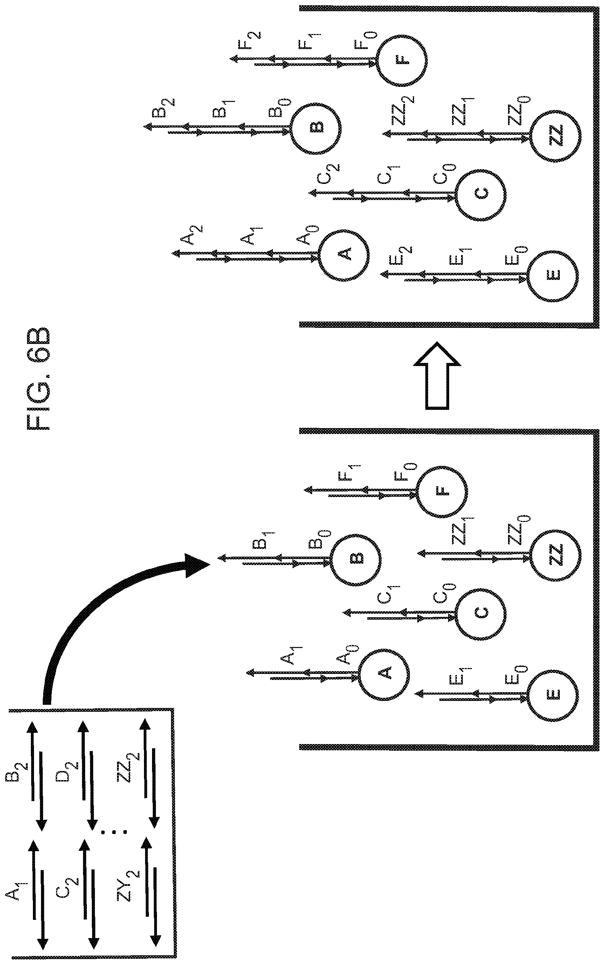

FIGS. 2A-5B show exemplary methods for generating at least one overhang that can be used for polynucleotide assembly as discussed below (FIGS. 6A and 6B). It should be noted that the overhangs can each be designed to have a predetermined length and/or sequence such that it can specifically and at least partially anneal with another overhang to facilitate ligation with another oligonucleotide. In some embodiments of the disclosure, the overhang can be 1, 2, 3, 4, 5, 6, 7, 8, 9, 10, 11, 12, 13, 14, 15, 16, 17, 18, 19 or 20 nucleotides in length, or longer.

FIGS. 6A-6B illustrate oligonucleotide sequences comprising DNA sequences produced from any of the methods described herein and illustrated in, for example, FIGS. 2A-2B, 3A-3D, 4A-4D and 5A-5B (e.g., Sequences A', B', C', and/or D') being subject to polynucleotide synthesis and assembly on bead solid support. For illustration purpose only, beads are shown in FIGS. 6A-6B but other solid supports such as a microarray device can also be used. A first plurality of oligonucleotides, A₀, B₀, C₀, ... and ZZo, can each be operably linked to a bead (e.g., each oligonucleotide can have a cleavable linker moiety synthesized or built therein), such that after synthesis, polynucleotides can be cleaved therefrom into a solution. The first plurality of oligonucleotides can be assembled with a second plurality of oligonucleotides, A₁, B₁, C₁, ... and ZZ₁, each having a sticky end that is completely or partially complementary to that of A₀, B₀, C₀, ... and ZZ₀, respectively, as shown in FIG. 6A. Assembly can be achieved using any one or more of ligation, primer extension, and PCR. A second assembly step is shown in FIG. 6B, adding a third plurality of oligonucleotides, A₂, B₂, C₂, ... and ZZ₂. This process can be repeated multiple times until a desired plurality of polynucleotide products are built. In some embodiments of the disclosure, the added oligonucleotides do not have a cleavable linker.

Additional examples of nicking and cleaving are shown in FIGS. 7-9B. In some embodiments of the disclosure, to form a dimer with an endonuclease activity, the first and second FokI monomers can both be wild type. As shown in the embodiment of the disclosure of FIG. 7, both the FokI monomer of the fusion protein and the second FokI monomer are catalytically active such that both the P and N strands are cut and the flanking region is cleaved from the remainder of the oligonucleotide. The oligonucleotides may then be ligated in a predefined order to assemble a target polynucleotide, or subject to further processing such as the production of cohesive single-stranded overhanging ends, or polymerase assembly.

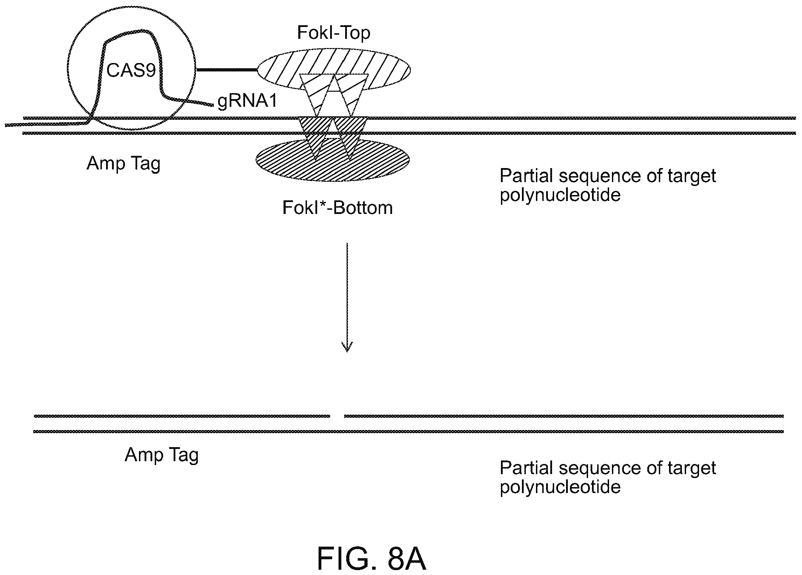

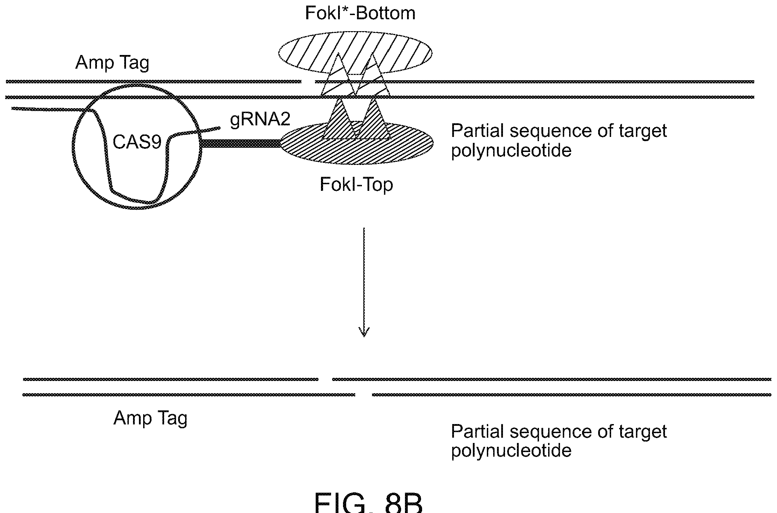

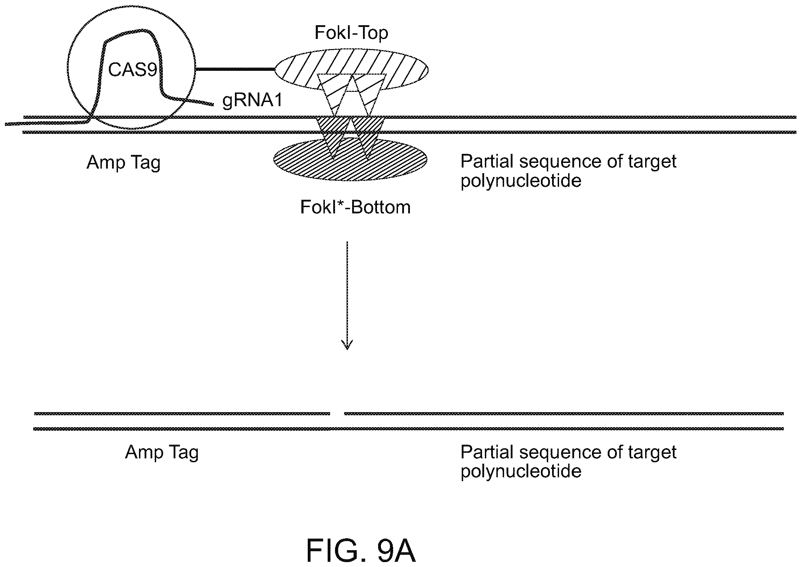

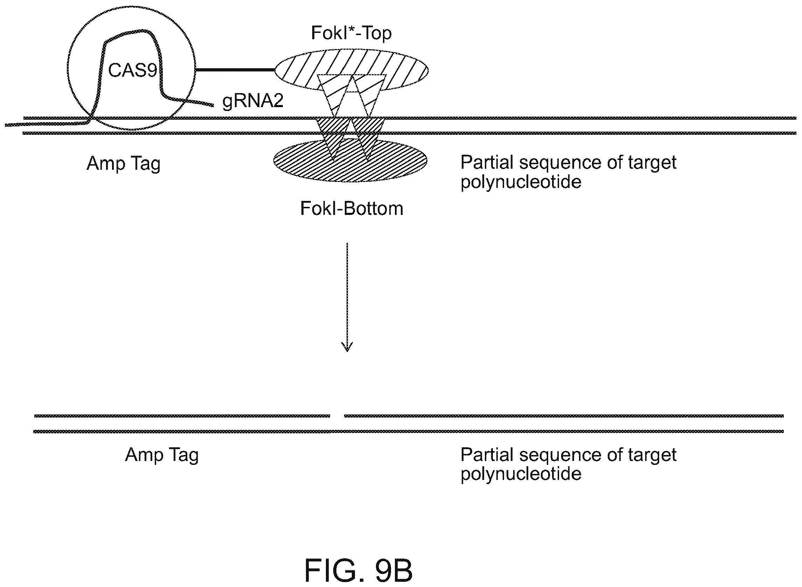

As shown in the embodiments of the disclosure of FIGS. 8A-8B and 9A-9B, one of the two FokI monomers is mutated such that only the P or N strand is cut, making the catalytic activity of the FokI dimer that of a nickase. For example, in one embodiment of the disclosure, the FokI monomer of the fusion protein is modified such that it does not cut the P or top strand, but the second FokI monomer cuts the N or bottom strand (FIG. 9B). In another embodiment of the disclosure, the second FokI monomer is mutated such that it does not cut the N strand, but the FokI monomer of the fusion protein cuts the P strand (FIGS. 8A, 8B and 9A).

FIGS. 8A-8B and 9A-9B show two different designs where the flanking regions of the plurality of oligonucleotides are cleaved from the remainder of the oligonucleotides in two nicking steps. In the first nicking step shown in FIGS. 8A and 9A, the top strand is cut by the top FokI monomer (FokI - top) of the fusion protein which is directed thereto by annealing of the gRNA1, while the bottom strand remains intact due to the inactive, second FokI monomer (FokI*-bottom). The second nicking steps in FIGS. 8B and 9B are different. In FIG. 8B, the bottom strand is cut by the bottom FokI monomer (FokI - bottom) of the fusion protein which is directed thereto by annealing of the gRNA2, without further nicking the top strand due to the inactive, second FokI monomer (FokI*- top). gRNA1 and gRNA2 can be designed to be completely or partially complementary to each other, such that the first nick and the second nick are offset by a pre-selected number (X) of nucleotides. For example, gRNA1 and gRNA2 can be designed to be completely complementary to each other, while the two fusion proteins in FIGS. 8A and 8B are engineered to have different linkers such that they nick at different distances from the gRNA binding position. Alternatively, the two fusion proteins may be identical and cut at the same distance from the gRNA binding site, but gRNA1 and gRNA2 are designed to offset by X nucleotides. In further embodiments of the disclosure, both linker length and gRNA1 and/or gRNA2 sequence can be varied, with the combination of the two strategies resulting in the predesigned overhang of X nucleotides.

Referring now to FIG. 9B, in the second nicking step, the FokI monomer of the fusion protein is inactive and does not cut the top strand, but the second FokI monomer cuts the bottom strand. Here, gRNA1 and gRNA2 can be designed to be completely or partially identical to each other, such that the first nick and the second nick are offset by a pre-selected number (X) of nucleotides. For example, gRNA1 and gRNA2 can be designed to be completely identical, while the two fusion proteins in FIGS. 9A and 9B are engineered to have different linkers such that they nick at different distance from the gRNA binding position. Alternatively, the two fusion proteins may have the same or similar linker and cut at the same distance from the gRNA binding site, but gRNA1 and gRNA2 are designed to offset by X nucleotides. In further embodiments of the disclosure, both linker length and gRNA1 and/or gRNA2 sequence can be varied, with the combination of the two strategies resulting in the predesigned overhang of X nucleotides.

In any of the embodiments of the disclosure of FIGS. 2A-5B and 7-9B, different linkers of different length/geometry in the Cas9-nuclease fusion proteins, and/or different gRNAs designed to position the catalytic domains of the fusion protein at different locations on the P and N strands may be used so as to produce single-stranded overhanging ends that are designed to permit cohesive end ligation and/or polymerase assembly of the construction oligonucleotides to form a target polynucleotide.

The target polynucleotide can be produced in a one-pot reaction where all construction oligonucleotides are mixed and ligated together. Ligation can also be performed sequentially (ligating oligonucleotides one by one) or hierarchically (ligating subpools of the oligonucleotides into one or more subconstructs which are then ligated into the final target construct). It should be noted that one or more of the construction oligonucleotides, one or more of the guide sequences, one or more of the subconstructs, and/or the final target construct can be non-naturally occurring, e.g., being unmethylated or modified in a way (e.g., chemically or biochemically modified in vitro) such that they become hemi-methylated or semi-methylated or hypomethylated, or have non-naturally occurring methylation patterns. Such non-naturally occurring methylation and methylation patterns can be used to regulate, for example, gene expression.

It should be noted that while FIGS. 1-9 illustrate cleavage and assembly of linear oligonucleotides, circular materials such as plasmids can also be subject to similar cleavage and assembly steps. For example, genes or fragments thereof can be first cloned into a plasmid, which can be amplified in vitro via culturing of the host, isolated and purified, cleaved using methods and compositions of the present disclosure, and then subjected to further manipulation such as assembly. Furthermore, circular products (e.g., a plasmid) can also be produced by the methods and composition disclosed herein. For example, one or more of the construction oligonucleotides may be derived from a vector, such that when assembled, a full vector can be produced. The vector can then be transformed into a host cell (e.g., *E. coli*) for propagation.

Methods and compositions of the present disclosure can be used in the assembly of long-length polynucleotides (e.g., 10kb or longer). In certain embodiments of the disclosure, small oligonucleotides (e.g., 100-800bp or 500-800bp) synthesized off of a chip can be first assembled into an intermediate polynucleotide, with or without using methods and compositions of the present disclosure. The intermediate polynucleotide can then be cloned into a plasmid, which can be introduced into a host, amplified via culturing, isolated and purified, cleaved using methods and compositions of the present disclosure, and then subjected to further assembly. This process can be repeated multiple times till the final long-length product is assembled.

In addition or as an alternative to direct ligation or polymerase assisted assembly, other methods can also be used to assemble cleavage products of the present disclosure. In some embodiments of the disclosure, the cleavage products can be subject to homologous recombination via SLiCE (Seamless Ligation Cloning Extract), as described in, for example, Zhang et al., Nucleic acids research 40.8 (2012): e55-e55 and U.S. Pub. No. 20130045508,. Briefly, SLiCE is a restriction site independent cloning/assembly method that is based on in vitro recombination between short regions of homologies (15-52 bp) in bacterial cell extracts derived from a RecA deficient bacterial strain engineered to contain an optimized λ prophage Red recombination system. Other recombination methods can also be used, such as recombination in yeast or phage. The cleavage products can be subject to Gibson assembly as described in, for example, Gibson et al., Nature Methods 6 (5): 343-345, and U.S. Pub. Nos. 20090275086 and 20100035768. In Gibson assembly, DNA fragments containing ∼20-40 base pair overlap with adjacent DNA fragments are mixed with three enzymes, an exonuclease, a DNA polymerase, and a DNA ligase. In a one-tube reaction, the exonuclease creates overhangs so that adjacent DNA fragments can anneal, the DNA polymerase incorporates nucleotides to fill in any gaps, and the ligase covalently joins the DNA fragments.

As will be appreciated, the compositions and systems of the disclosure are useful in various areas of biotechnology, and particularly synthetic biotechnology, where site-directed nicking or cleaving of oligonucleotides is desired. For example, methods of the disclosure may be employed to cleave markers or selectable tags from nucleic acids. In one embodiment of the disclosure, the gRNA directs a fusion protein (e.g., fCas9) to a double stranded DNA sequence coding for an amino acid sequence selected from selectable marker(s) and/or tag(s) such as: ampicillin, kanamycin (KAN), tetracyclin (TET), glutathione-s-transferase (GST), maltose-binding protein (MBP), horse radish peroxidase (HRP), alkaline phosphatase (AP), red fluorescent protein (RFP), yellow fluorescent protein (YFP), green fluorescent protein (GFP), cyan fluorescent protein (CFP), FLAG, c-myc, human influenza hemaglutinin (HA), 6x histidine (6xHis), and/or any combination thereof. In an embodiment of the disclosure, gRNA directs a fusion protein to a segment of a double stranded DNA that does not code for a selectable marker and/or tag.

Use of ordinal terms such as "first," "second," "third," etc., in the claims to modify a claim element does not by itself connote any priority, precedence, or order of one claim element over another or the temporal order in which acts of a method are performed, but are used merely as labels to distinguish one claim element having a certain name from another element having a same name (but for the use of the ordinal term) to distinguish the claim elements.

Also, the phraseology and terminology used herein is for the purpose of description and should not be regarded as limiting. The use of "including," "comprising," or "having," "containing," "involving," and variations thereof herein, is meant to encompass the items listed thereafter and equivalents thereof as well as additional items. "Consisting essentially of' means inclusion of the items listed thereafter and which is open to unlisted items that do not materially affect the basic and novel properties of the invention.

### EXAMPLE

A 2,000-mer is nicked at a first location using the fCas9 fusion protein bound to gRNA1 and at a second location using the fCas9 fusion protein bound to gRNA2. The resulting products are (1) a released Amp tag of 851 nucleotides (e.g., Amp sequence: ADA79624.1) and (2) a sequence of 1,149-mer, which contains an overhang. The 1,149-mer is bound to a bead at the non-overhang end and is then combined with a second sequence, which contains a complementary sticky end. A ligase is added to join the two sequences together on the bead. This additive process is continued until the desired polynucleotide length/sequence is synthesized. Then, the final product is cleaved from the bead and eluted. This process is sequential assembly.

Alternatively, multiple construction sequences can be nicked and/or cleaved in parallel to produce sticky ends that are pre-designed to be complementary to one another in a predetermined order, such that when combined in a ligation reaction, the construction sequences assemble in the predetermined arrangement.

Hierarchical assembly can also be used to produce the target product. For example, the construction sequences can be divided into two or more pools, each pool comprising a subsequence of the target product. After assembly of each pool of construction sequences into two or more subproducts, the subproducts can then be assembled into the final product.

The sequences below are non-limiting examples of the present disclosure:
SEQ IDNO.:1: Cas9
SEQ ID NO.:2: Cas9 nickase (D10A)
SEQ ID NO.:3: dCas9 (D10A and H840A); inactive Cas
SEQ ID NO.:4: *FokI* [partial amino acid sequence]
SEQ ID NO.:5: *FokI* (D69A) [partial amino acid sequence]
SEQ ID NO.:6: DNA coding sequence of wild-type Cas9 nuclease
SEQ ID NO.:7: DNA coding sequence of Cas9 nickase
SEQ ID NO.:8: DNA coding sequence of dCas9-NLS-GGS3linker-*FokI*
SEQ ID NO.:9: DNA coding sequence of NLS-dCas9-GGS3linker-*FokI*
SEQ ID NO.:10: DNA coding sequence of *FokI*-GGS3linker-dCas9-NLS
SEQ ID NO.:11: DNA coding sequence of NLS-*FokI*-GGS3linker-dCas9
SEQ ID NO.:12: DNA coding sequence of NLS-GGS-FokI-XTEN-dCas9 ("fCas9")
SEQ ID NO.:13: (GGS)x3
SEQ ID NO.:14: FokI-(GGS)x6
SEQ ID NO.:15: FokI-L1
SEQ ID NO.:16: FokI-L2
SEQ ID NO.:17: FokI-L3
SEQ ID NO.:18: FokI-L4
SEQ ID NO.:19: FokI-L5
SEQ ID NO.:20: FokI-L6
SEQ ID NO.:21: FokI-L7
SEQ ID NO.:22: FokI-L8
SEQ ID NO.:23: FokI-L9
SEQ ID NO.:24: NLS-L3

### REFERENCES

In addition to all other publications, patents, and sequence database entries referenced, reference is made to the following publications:
- Miller, et al., "An improved zinc-finger nuclease architecture for highly specific genome editing," Nature Biotechnology, 25 (7), pp. 778-85 (2007)
- Ramirez, et al., "Engineered zinc finger nickases induce homology-directed repair with reduced mutagenic effects," Nucleic Acids Research, 40 (12), pp 5560-68 (2012)
- Guilinger, et al., "Fusion of catalytically inactive Cas9 to FokI nuclease improves the specificity of genome modification," Nature Biotechnology, 32 (6) pp. 577-83 (2014)
- Tsia, et al, "Dimeric CRISPR RNA-guided FokI nucleases for highly specific genome editing," Nature Biotechnology, 32 (6) pp. 569-77 (2014)
- Mali, et al., "Cas9 as a versatile tool for engineering biology," Nat Methods, 10 (10), pp. 957-63 (2013)
- Bassett, et al., "Highly Efficient Targeted Mutagenesis of Drosophila with CRISPR/Cas9 System," Cell Reports 4, pp. 220-28 (2013)
- Christian, et al., "Targeting DNA Double-Strand Breaks with TAL Effector Nucleases," Genetics 186, pp. 757-61 (2010)
- Lippow, et al., "Creation of a type IIS restriction endonuclease with a long recognition sequence," Nucleic Acids Research, 37 (9), pp. 3061-73 (2009)
- Looney, et al., "Nucleotide sequence of the FokI restriction-modification system: separate strand-specificity domains in the methyltransferase," Gene, 80 (2), pp. 193-208
- Jacobson, et al., "Methods and Devices for Nucleic Acid Synthesis," U.S. Patent Application Publication No. 2013/0296294
- Kung, et al., "Methods for Preparative In Vitro Cloning," International Patent Application Publication No. WO2012/174337
- Jacobson, et al., "Compositions and Methods for High Fidelity Assembly of Nucleic Acids," International Patent Application Publication No. WO2013/032850
- Jacobson, et al., "Methods for Nucleic Acid Assembly and High Throughput Sequencing," International Patent Application Publication No. WO2014/004393
- Kung, et al., "Methods for Sorting Nucleic Acids and Muliplexed Preparative In Vitro Cloning," International Patent Application Publication No. WO2013/163263
- Joung, et al., "TALENs: a widely applicable technology for targeted genome editing," Nat. Rev. Mol. Cell Biol. 14, 49-55 (2012).
- Urnov, et al., "Genome editing with engineered zinc finger nucleases," Nat. Rev. Genet. 11, 636-646 (2010).
- Silva et al., "Meganucleases and Other Tools for Targeted Genome Engineering: Perspectives and Challenges for Gene Therapy," Curr Gene Ther. Feb 2011; 11(1):

### SEQUENCE LISTING

<110> Gen9, Inc.
<120> COMPOSITIONS AND METHODS FOR SITE-DIRECTED DNA NICKING AND CLEAVING
<130> 127662-014902/PCT
<150> US 62/022,617
   <151> 2014-07-09
<150> US 62/065,238
   <151> 2014-10-17
<160> 24
<170> PatentIn version 3.5
<210> 1
   <211> 1368
   <212> PRT
   <213> Streptococcus pyogenes
<400> 1
   Met Asp Lys Lys Tyr Ser Ile Gly Leu Ala Ile Gly Thr Asn Ser Val
   1               5                   10                  15
   Gly Trp Ala Val Ile Thr Asp Glu Tyr Lys Val Pro Ser Lys Lys Phe
               20                  25                  30
   Lys Val Leu Gly Asn Thr Asp Arg His Ser Ile Lys Lys Asn Leu Ile
           35                  40                  45
   Gly Ala Leu Leu Phe Asp Ser Gly Glu Thr Ala Glu Ala Thr Arg Leu
       50                  55                  60
   Lys Arg Thr Ala Arg Arg Arg Tyr Thr Arg Arg Lys Asn Arg Ile Cys
   65                  70                  75                  80
   Tyr Leu Gln Glu Ile Phe Ser Asn Glu Met Ala Lys Val Asp Asp Ser
                   85                  90                  95
   Phe Phe His Arg Leu Glu Glu Ser Phe Leu Val Glu Glu Asp Lys Lys
               100                 105                 110
   His Glu Arg His Pro Ile Phe Gly Asn Ile Val Asp Glu Val Ala Tyr
           115                 120                 125
   His Glu Lys Tyr Pro Thr Ile Tyr His Leu Arg Lys Lys Leu Val Asp
       130                 135                 140
   Ser Thr Asp Lys Ala Asp Leu Arg Leu Ile Tyr Leu Ala Leu Ala His
   145 150 155 160
   Met Ile Lys Phe Arg Gly His Phe Leu Ile Glu Gly Asp Leu Asn Pro
   165 170 175
   Asp Asn Ser Asp Val Asp Lys Leu Phe Ile Gln Leu Val Gln Thr Tyr
   180 185 190
   Asn Gln Leu Phe Glu Glu Asn Pro Ile Asn Ala Ser Gly Val Asp Ala
   195 200 205
   Lys Ala Ile Leu Ser Ala Arg Leu Ser Lys Ser Arg Arg Leu Glu Asn
   210 215 220
   Leu Ile Ala Gln Leu Pro Gly Glu Lys Lys Asn Gly Leu Phe Gly Asn
   225 230 235 240
   Leu Ile Ala Leu Ser Leu Gly Leu Thr Pro Asn Phe Lys Ser Asn Phe
   245 250 255
   Asp Leu Ala Glu Asp Ala Lys Leu Gln Leu Ser Lys Asp Thr Tyr Asp
   260 265 270
   Asp Asp Leu Asp Asn Leu Leu Ala Gln Ile Gly Asp Gln Tyr Ala Asp
   275 280 285
   Leu Phe Leu Ala Ala Lys Asn Leu Ser Asp Ala Ile Leu Leu Ser Asp
   290 295 300
   Ile Leu Arg Val Asn Thr Glu Ile Thr Lys Ala Pro Leu Ser Ala Ser
   305 310 315 320
   Met Ile Lys Arg Tyr Asp Glu His His Gln Asp Leu Thr Leu Leu Lys
   325 330 335
   Ala Leu Val Arg Gln Gln Leu Pro Glu Lys Tyr Lys Glu Ile Phe Phe
   340 345 350
   Asp Gln Ser Lys Asn Gly Tyr Ala Gly Tyr Ile Asp Gly Gly Ala Ser
   355 360 365
   Gln Glu Glu Phe Tyr Lys Phe Ile Lys Pro Ile Leu Glu Lys Met Asp
   370 375 380
   Gly Thr Glu Glu Leu Leu Val Lys Leu Asn Arg Glu Asp Leu Leu Arg
   385 390 395 400
   Lys Gln Arg Thr Phe Asp Asn Gly Ser Ile Pro His Gln Ile His Leu
   405 410 415
   Gly Glu Leu His Ala Ile Leu Arg Arg Gln Glu Asp Phe Tyr Pro Phe
   420 425 430
   Leu Lys Asp Asn Arg Glu Lys Ile Glu Lys Ile Leu Thr Phe Arg Ile
   435 440 445
   Pro Tyr Tyr Val Gly Pro Leu Ala Arg Gly Asn Ser Arg Phe Ala Trp
   450 455 460
   Met Thr Arg Lys Ser Glu Glu Thr Ile Thr Pro Trp Asn Phe Glu Glu
   465 470 475 480
   Val Val Asp Lys Gly Ala Ser Ala Gln Ser Phe Ile Glu Arg Met Thr
   485 490 495
   Asn Phe Asp Lys Asn Leu Pro Asn Glu Lys Val Leu Pro Lys His Ser
   500 505 510
   Leu Leu Tyr Glu Tyr Phe Thr Val Tyr Asn Glu Leu Thr Lys Val Lys
   515 520 525
   Tyr Val Thr Glu Gly Met Arg Lys Pro Ala Phe Leu Ser Gly Glu Gln
   530 535 540
   Lys Lys Ala Ile Val Asp Leu Leu Phe Lys Thr Asn Arg Lys Val Thr
   545 550 555 560
   Val Lys Gln Leu Lys Glu Asp Tyr Phe Lys Lys Ile Glu Cys Phe Asp
   565 570 575
   Ser Val Glu Ile Ser Gly Val Glu Asp Arg Phe Asn Ala Ser Leu Gly
   580 585 590
   Thr Tyr His Asp Leu Leu Lys Ile Ile Lys Asp Lys Asp Phe Leu Asp
   595 600 605
   Asn Glu Glu Asn Glu Asp Ile Leu Glu Asp Ile Val Leu Thr Leu Thr
   610 615 620
   Leu Phe Glu Asp Arg Glu Met Ile Glu Glu Arg Leu Lys Thr Tyr Ala
   625 630 635 640
   His Leu Phe Asp Asp Lys Val Met Lys Gln Leu Lys Arg Arg Arg Tyr
   645 650 655
   Thr Gly Trp Gly Arg Leu Ser Arg Lys Leu Ile Asn Gly Ile Arg Asp
               660                 665                 670
   Lys Gln Ser Gly Lys Thr Ile Leu Asp Phe Leu Lys Ser Asp Gly Phe
           675                 680                 685
   Ala Asn Arg Asn Phe Met Gln Leu Ile His Asp Asp Ser Leu Thr Phe
       690                 695                 700
   Lys Glu Asp Ile Gln Lys Ala Gln Val Ser Gly Gln Gly Asp Ser Leu
   705                 710                 715                 720
   His Glu His Ile Ala Asn Leu Ala Gly Ser Pro Ala Ile Lys Lys Gly
                   725                 730                 735
   Ile Leu Gln Thr Val Lys Val Val Asp Glu Leu Val Lys Val Met Gly
               740                 745                 750
   Arg His Lys Pro Glu Asn Ile Val Ile Glu Met Ala Arg Glu Asn Gln
           755                 760                 765
   Thr Thr Gln Lys Gly Gln Lys Asn Ser Arg Glu Arg Met Lys Arg Ile
       770                 775                 780
   Glu Glu Gly Ile Lys Glu Leu Gly Ser Gln Ile Leu Lys Glu His Pro
   785                 790                 795                 800
   Val Glu Asn Thr Gln Leu Gln Asn Glu Lys Leu Tyr Leu Tyr Tyr Leu
                   805                 810                 815
   Gln Asn Gly Arg Asp Met Tyr Val Asp Gln Glu Leu Asp Ile Asn Arg
               820                 825                 830
   Leu Ser Asp Tyr Asp Val Asp His Ile Val Pro Gln Ser Phe Leu Lys
           835                 840                 845
   Asp Asp Ser Ile Asp Asn Lys Val Leu Thr Arg Ser Asp Lys Asn Arg
       850                 855                 860
   Gly Lys Ser Asp Asn Val Pro Ser Glu Glu Val Val Lys Lys Met Lys
   865                 870                 875                 880
   Asn Tyr Trp Arg Gln Leu Leu Asn Ala Lys Leu Ile Thr Gln Arg Lys
                   885                 890                 895
   Phe Asp Asn Leu Thr Lys Ala Glu Arg Gly Gly Leu Ser Glu Leu Asp
               900                 905                 910
   Lys Ala Gly Phe Ile Lys Arg Gln Leu Val Glu Thr Arg Gln Ile Thr
           915                 920                 925
   Lys His Val Ala Gln Ile Leu Asp Ser Arg Met Asn Thr Lys Tyr Asp
       930                 935                 940
   Glu Asn Asp Lys Leu Ile Arg Glu Val Lys Val Ile Thr Leu Lys Ser
   945                 950                 955                 960
   Lys Leu Val Ser Asp Phe Arg Lys Asp Phe Gln Phe Tyr Lys Val Arg
                   965                 970                 975
   Glu Ile Asn Asn Tyr His His Ala His Asp Ala Tyr Leu Asn Ala Val
               980                 985                 990
   Val Gly Thr Ala Leu Ile Lys Lys  Tyr Pro Lys Leu Glu  Ser Glu Phe
           995                 1000                1005
   Val Tyr  Gly Asp Tyr Lys Val  Tyr Asp Val Arg Lys  Met Ile Ala
       1010                 1015                 1020
   Lys Ser  Glu Gln Glu Ile Gly  Lys Ala Thr Ala Lys  Tyr Phe Phe
       1025                 1030                 1035
   Tyr Ser  Asn Ile Met Asn Phe  Phe Lys Thr Glu Ile  Thr Leu Ala
       1040                 1045                 1050
   Asn Gly  Glu Ile Arg Lys Arg  Pro Leu Ile Glu Thr  Asn Gly Glu
       1055                 1060                 1065
   Thr Gly  Glu Ile Val Trp Asp  Lys Gly Arg Asp Phe  Ala Thr Val
       1070                 1075                 1080
   Arg Lys  Val Leu Ser Met Pro  Gln Val Asn Ile Val  Lys Lys Thr
       1085                 1090                 1095
   Glu Val  Gln Thr Gly Gly Phe  Ser Lys Glu Ser Ile  Leu Pro Lys
       1100                 1105                 1110
   Arg Asn  Ser Asp Lys Leu Ile  Ala Arg Lys Lys Asp  Trp Asp Pro
       1115                 1120                 1125
   Lys Lys  Tyr Gly Gly Phe Asp  Ser Pro Thr Val Ala  Tyr Ser Val
       1130                 1135                 1140
   Leu Val  Val Ala Lys Val Glu  Lys Gly Lys Ser Lys  Lys Leu Lys
   1145 1150 1155
   Ser Val Lys Glu Leu Leu Gly Ile Thr Ile Met Glu Arg Ser Ser
   1160 1165 1170
   Phe Glu Lys Asn Pro Ile Asp Phe Leu Glu Ala Lys Gly Tyr Lys
   1175 1180 1185
   Glu Val Lys Lys Asp Leu Ile Ile Lys Leu Pro Lys Tyr Ser Leu
   1190 1195 1200
   Phe Glu Leu Glu Asn Gly Arg Lys Arg Met Leu Ala Ser Ala Gly
   1205 1210 1215
   Glu Leu Gln Lys Gly Asn Glu Leu Ala Leu Pro Ser Lys Tyr Val
   1220 1225 1230
   Asn Phe Leu Tyr Leu Ala Ser His Tyr Glu Lys Leu Lys Gly Ser
   1235 1240 1245
   Pro Glu Asp Asn Glu Gln Lys Gln Leu Phe Val Glu Gln His Lys
   1250 1255 1260
   His Tyr Leu Asp Glu Ile Ile Glu Gln Ile Ser Glu Phe Ser Lys
   1265 1270 1275
   Arg Val Ile Leu Ala Asp Ala Asn Leu Asp Lys Val Leu Ser Ala
   1280 1285 1290
   Tyr Asn Lys His Arg Asp Lys Pro Ile Arg Glu Gln Ala Glu Asn
   1295 1300 1305
   Ile Ile His Leu Phe Thr Leu Thr Asn Leu Gly Ala Pro Ala Ala
   1310 1315 1320
   Phe Lys Tyr Phe Asp Thr Thr Ile Asp Arg Lys Arg Tyr Thr Ser
   1325 1330 1335
   Thr Lys Glu Val Leu Asp Ala Thr Leu Ile His Gln Ser Ile Thr
   1340 1345 1350
   Gly Leu Tyr Glu Thr Arg Ile Asp Leu Ser Gln Leu Gly Gly Asp
   1355 1360 1365
<210> 2
   <211> 1368
   <212> PRT
   <213> Unknown
<220>
   <223> Mutant
<400> 2
   Met Asp Lys Lys Tyr Ser Ile Gly Leu Ala Ile Gly Thr Asn Ser Val
   1               5                   10                  15
   Gly Trp Ala Val Ile Thr Asp Glu Tyr Lys Val Pro Ser Lys Lys Phe
               20                  25                  30
   Lys Val Leu Gly Asn Thr Asp Arg His Ser Ile Lys Lys Asn Leu Ile
           35                  40                  45
   Gly Ala Leu Leu Phe Asp Ser Gly Glu Thr Ala Glu Ala Thr Arg Leu
       50                  55                  60
   Lys Arg Thr Ala Arg Arg Arg Tyr Thr Arg Arg Lys Asn Arg Ile Cys
   65                  70                  75                  80
   Tyr Leu Gln Glu Ile Phe Ser Asn Glu Met Ala Lys Val Asp Asp Ser
                   85                  90                  95
   Phe Phe His Arg Leu Glu Glu Ser Phe Leu Val Glu Glu Asp Lys Lys
               100                 105                 110
   His Glu Arg His Pro Ile Phe Gly Asn Ile Val Asp Glu Val Ala Tyr
           115                 120                 125
   His Glu Lys Tyr Pro Thr Ile Tyr His Leu Arg Lys Lys Leu Val Asp
       130                 135                 140
   Ser Thr Asp Lys Ala Asp Leu Arg Leu Ile Tyr Leu Ala Leu Ala His
   145                 150                 155                 160
   Met Ile Lys Phe Arg Gly His Phe Leu Ile Glu Gly Asp Leu Asn Pro
                   165                 170                 175
   Asp Asn Ser Asp Val Asp Lys Leu Phe Ile Gln Leu Val Gln Thr Tyr
               180                 185                 190
   Asn Gln Leu Phe Glu Glu Asn Pro Ile Asn Ala Ser Gly Val Asp Ala
           195                 200                 205
   Lys Ala Ile Leu Ser Ala Arg Leu Ser Lys Ser Arg Arg Leu Glu Asn
       210                 215                 220
   Leu Ile Ala Gln Leu Pro Gly Glu Lys Lys Asn Gly Leu Phe Gly Asn
   225                 230                 235                 240
   Leu Ile Ala Leu Ser Leu Gly Leu Thr Pro Asn Phe Lys Ser Asn Phe
                   245                 250                 255
   Asp Leu Ala Glu Asp Ala Lys Leu Gln Leu Ser Lys Asp Thr Tyr Asp
               260                 265                 270
   Asp Asp Leu Asp Asn Leu Leu Ala Gln Ile Gly Asp Gln Tyr Ala Asp
           275                 280                 285
   Leu Phe Leu Ala Ala Lys Asn Leu Ser Asp Ala Ile Leu Leu Ser Asp
       290                 295                 300
   Ile Leu Arg Val Asn Thr Glu Ile Thr Lys Ala Pro Leu Ser Ala Ser
   305                 310                 315                 320
   Met Ile Lys Arg Tyr Asp Glu His His Gln Asp Leu Thr Leu Leu Lys
                   325                 330                 335
   Ala Leu Val Arg Gln Gln Leu Pro Glu Lys Tyr Lys Glu Ile Phe Phe
               340                 345                 350
   Asp Gln Ser Lys Asn Gly Tyr Ala Gly Tyr Ile Asp Gly Gly Ala Ser
           355                 360                 365
   Gln Glu Glu Phe Tyr Lys Phe Ile Lys Pro Ile Leu Glu Lys Met Asp
       370                 375                 380
   Gly Thr Glu Glu Leu Leu Val Lys Leu Asn Arg Glu Asp Leu Leu Arg
   385                 390                 395                 400
   Lys Gln Arg Thr Phe Asp Asn Gly Ser Ile Pro His Gln Ile His Leu
                   405                 410                 415
   Gly Glu Leu His Ala Ile Leu Arg Arg Gln Glu Asp Phe Tyr Pro Phe
               420                 425                 430
   Leu Lys Asp Asn Arg Glu Lys Ile Glu Lys Ile Leu Thr Phe Arg Ile
           435                 440                 445
   Pro Tyr Tyr Val Gly Pro Leu Ala Arg Gly Asn Ser Arg Phe Ala Trp
       450                 455                 460
   Met Thr Arg Lys Ser Glu Glu Thr Ile Thr Pro Trp Asn Phe Glu Glu
   465                 470                 475                 480
   Val Val Asp Lys Gly Ala Ser Ala Gln Ser Phe Ile Glu Arg Met Thr
                   485                 490                 495
   Asn Phe Asp Lys Asn Leu Pro Asn Glu Lys Val Leu Pro Lys His Ser
               500                 505                 510
   Leu Leu Tyr Glu Tyr Phe Thr Val Tyr Asn Glu Leu Thr Lys Val Lys
           515                 520                 525
   Tyr Val Thr Glu Gly Met Arg Lys Pro Ala Phe Leu Ser Gly Glu Gln
       530                 535                 540
   Lys Lys Ala Ile Val Asp Leu Leu Phe Lys Thr Asn Arg Lys Val Thr
   545                 550                 555                 560
   Val Lys Gln Leu Lys Glu Asp Tyr Phe Lys Lys Ile Glu Cys Phe Asp
                   565                 570                 575
   Ser Val Glu Ile Ser Gly Val Glu Asp Arg Phe Asn Ala Ser Leu Gly
               580                 585                 590
   Thr Tyr His Asp Leu Leu Lys Ile Ile Lys Asp Lys Asp Phe Leu Asp
           595                 600                 605
   Asn Glu Glu Asn Glu Asp Ile Leu Glu Asp Ile Val Leu Thr Leu Thr
       610                 615                 620
   Leu Phe Glu Asp Arg Glu Met Ile Glu Glu Arg Leu Lys Thr Tyr Ala
   625                 630                 635                 640
   His Leu Phe Asp Asp Lys Val Met Lys Gln Leu Lys Arg Arg Arg Tyr
                   645                 650                 655
   Thr Gly Trp Gly Arg Leu Ser Arg Lys Leu Ile Asn Gly Ile Arg Asp
               660                 665                 670
   Lys Gln Ser Gly Lys Thr Ile Leu Asp Phe Leu Lys Ser Asp Gly Phe
           675                 680                 685
   Ala Asn Arg Asn Phe Met Gln Leu Ile His Asp Asp Ser Leu Thr Phe
       690                 695                 700
   Lys Glu Asp Ile Gln Lys Ala Gln Val Ser Gly Gln Gly Asp Ser Leu
   705                 710                 715                 720
   His Glu His Ile Ala Asn Leu Ala Gly Ser Pro Ala Ile Lys Lys Gly
                   725                 730                 735
   Ile Leu Gln Thr Val Lys Val Val Asp Glu Leu Val Lys Val Met Gly
               740                 745                 750
   Arg His Lys Pro Glu Asn Ile Val Ile Glu Met Ala Arg Glu Asn Gln
           755                 760                 765
   Thr Thr Gln Lys Gly Gln Lys Asn Ser Arg Glu Arg Met Lys Arg Ile
       770                 775                 780
   Glu Glu Gly Ile Lys Glu Leu Gly Ser Gln Ile Leu Lys Glu His Pro
   785                 790                 795                 800
   Val Glu Asn Thr Gln Leu Gln Asn Glu Lys Leu Tyr Leu Tyr Tyr Leu
                   805                 810                 815
   Gln Asn Gly Arg Asp Met Tyr Val Asp Gln Glu Leu Asp Ile Asn Arg
               820                 825                 830
   Leu Ser Asp Tyr Asp Val Asp His Ile Val Pro Gln Ser Phe Leu Lys
           835                 840                 845
   Asp Asp Ser Ile Asp Asn Lys Val Leu Thr Arg Ser Asp Lys Asn Arg
       850                 855                 860
   Gly Lys Ser Asp Asn Val Pro Ser Glu Glu Val Val Lys Lys Met Lys
   865                 870                 875                 880
   Asn Tyr Trp Arg Gln Leu Leu Asn Ala Lys Leu Ile Thr Gln Arg Lys
                   885                 890                 895
   Phe Asp Asn Leu Thr Lys Ala Glu Arg Gly Gly Leu Ser Glu Leu Asp
               900                 905                 910
   Lys Ala Gly Phe Ile Lys Arg Gln Leu Val Glu Thr Arg Gln Ile Thr
           915                 920                 925
   Lys His Val Ala Gln Ile Leu Asp Ser Arg Met Asn Thr Lys Tyr Asp
       930                 935                 940
   Glu Asn Asp Lys Leu Ile Arg Glu Val Lys Val Ile Thr Leu Lys Ser
   945                 950                 955                 960
   Lys Leu Val Ser Asp Phe Arg Lys Asp Phe Gln Phe Tyr Lys Val Arg
                   965                 970                 975
   Glu Ile Asn Asn Tyr His His Ala His Asp Ala Tyr Leu Asn Ala Val
               980                 985                 990
   Val Gly Thr Ala Leu Ile Lys Lys Tyr Pro Lys Leu Glu Ser Glu Phe
   995 1000 1005
   Val Tyr Gly Asp Tyr Lys Val Tyr Asp Val Arg Lys Met Ile Ala
   1010 1015 1020
   Lys Ser Glu Gln Glu Ile Gly Lys Ala Thr Ala Lys Tyr Phe Phe
   1025 1030 1035
   Tyr Ser Asn Ile Met Asn Phe Phe Lys Thr Glu Ile Thr Leu Ala
   1040 1045 1050
   Asn Gly Glu Ile Arg Lys Arg Pro Leu Ile Glu Thr Asn Gly Glu
   1055 1060 1065
   Thr Gly Glu Ile Val Trp Asp Lys Gly Arg Asp Phe Ala Thr Val
   1070 1075 1080
   Arg Lys Val Leu Ser Met Pro Gln Val Asn Ile Val Lys Lys Thr
   1085 1090 1095
   Glu Val Gln Thr Gly Gly Phe Ser Lys Glu Ser Ile Leu Pro Lys
   1100 1105 1110
   Arg Asn Ser Asp Lys Leu Ile Ala Arg Lys Lys Asp Trp Asp Pro
   1115 1120 1125
   Lys Lys Tyr Gly Gly Phe Asp Ser Pro Thr Val Ala Tyr Ser Val
   1130 1135 1140
   Leu Val Val Ala Lys Val Glu Lys Gly Lys Ser Lys Lys Leu Lys
   1145 1150 1155
   Ser Val Lys Glu Leu Leu Gly Ile Thr Ile Met Glu Arg Ser Ser
   1160 1165 1170
   Phe Glu Lys Asn Pro Ile Asp Phe Leu Glu Ala Lys Gly Tyr Lys
   1175 1180 1185
   Glu Val Lys Lys Asp Leu Ile Ile Lys Leu Pro Lys Tyr Ser Leu
   1190 1195 1200
   Phe Glu Leu Glu Asn Gly Arg Lys Arg Met Leu Ala Ser Ala Gly
   1205 1210 1215
   Glu Leu Gln Lys Gly Asn Glu Leu Ala Leu Pro Ser Lys Tyr Val
   1220 1225 1230
   Asn Phe Leu Tyr Leu Ala Ser His Tyr Glu Lys Leu Lys Gly Ser
   1235 1240 1245
   Pro Glu Asp Asn Glu Gln Lys Gln Leu Phe Val Glu Gln His Lys
   1250 1255 1260
   His Tyr Leu Asp Glu Ile Ile Glu Gln Ile Ser Glu Phe Ser Lys
   1265 1270 1275
   Arg Val Ile Leu Ala Asp Ala Asn Leu Asp Lys Val Leu Ser Ala
   1280 1285 1290
   Tyr Asn Lys His Arg Asp Lys Pro Ile Arg Glu Gln Ala Glu Asn
   1295 1300 1305
   Ile Ile His Leu Phe Thr Leu Thr Asn Leu Gly Ala Pro Ala Ala
   1310 1315 1320
   Phe Lys Tyr Phe Asp Thr Thr Ile Asp Arg Lys Arg Tyr Thr Ser
   1325 1330 1335
   Thr Lys Glu Val Leu Asp Ala Thr Leu Ile His Gln Ser Ile Thr
   1340 1345 1350
   Gly Leu Tyr Glu Thr Arg Ile Asp Leu Ser Gln Leu Gly Gly Asp
   1355 1360 1365
<210> 3
   <211> 1368
   <212> PRT
   <213> Unknown
<220>
   <223> Mutant
<400> 3
   Met Asp Lys Lys Tyr Ser Ile Gly Leu Ala Ile Gly Thr Asn Ser Val
   1               5                   10                  15
   Gly Trp Ala Val Ile Thr Asp Glu Tyr Lys Val Pro Ser Lys Lys Phe
               20                  25                  30
   Lys Val Leu Gly Asn Thr Asp Arg His Ser Ile Lys Lys Asn Leu Ile
           35                  40                  45
   Gly Ala Leu Leu Phe Asp Ser Gly Glu Thr Ala Glu Ala Thr Arg Leu
       50                  55                  60
   Lys Arg Thr Ala Arg Arg Arg Tyr Thr Arg Arg Lys Asn Arg Ile Cys
   65                  70                  75                  80
   Tyr Leu Gln Glu Ile Phe Ser Asn Glu Met Ala Lys Val Asp Asp Ser
                   85                  90                  95
   Phe Phe His Arg Leu Glu Glu Ser Phe Leu Val Glu Glu Asp Lys Lys
               100                 105                 110
   His Glu Arg His Pro Ile Phe Gly Asn Ile Val Asp Glu Val Ala Tyr
           115                 120                 125
   His Glu Lys Tyr Pro Thr Ile Tyr His Leu Arg Lys Lys Leu Val Asp
       130                 135                 140
   Ser Thr Asp Lys Ala Asp Leu Arg Leu Ile Tyr Leu Ala Leu Ala His
   145                 150                 155                 160
   Met Ile Lys Phe Arg Gly His Phe Leu Ile Glu Gly Asp Leu Asn Pro
                   165                 170                 175
   Asp Asn Ser Asp Val Asp Lys Leu Phe Ile Gln Leu Val Gln Thr Tyr
               180                 185                 190
   Asn Gln Leu Phe Glu Glu Asn Pro Ile Asn Ala Ser Gly Val Asp Ala
           195                 200                 205
   Lys Ala Ile Leu Ser Ala Arg Leu Ser Lys Ser Arg Arg Leu Glu Asn
       210                 215                 220
   Leu Ile Ala Gln Leu Pro Gly Glu Lys Lys Asn Gly Leu Phe Gly Asn
   225                 230                 235                 240
   Leu Ile Ala Leu Ser Leu Gly Leu Thr Pro Asn Phe Lys Ser Asn Phe
                   245                 250                 255
   Asp Leu Ala Glu Asp Ala Lys Leu Gln Leu Ser Lys Asp Thr Tyr Asp
               260                 265                 270
   Asp Asp Leu Asp Asn Leu Leu Ala Gln Ile Gly Asp Gln Tyr Ala Asp
           275                 280                 285
   Leu Phe Leu Ala Ala Lys Asn Leu Ser Asp Ala Ile Leu Leu Ser Asp
       290                 295                 300
   Ile Leu Arg Val Asn Thr Glu Ile Thr Lys Ala Pro Leu Ser Ala Ser
   305 310 315 320
   Met Ile Lys Arg Tyr Asp Glu His His Gln Asp Leu Thr Leu Leu Lys
   325 330 335
   Ala Leu Val Arg Gln Gln Leu Pro Glu Lys Tyr Lys Glu Ile Phe Phe
   340 345 350
   Asp Gln Ser Lys Asn Gly Tyr Ala Gly Tyr Ile Asp Gly Gly Ala Ser
   355 360 365
   Gln Glu Glu Phe Tyr Lys Phe Ile Lys Pro Ile Leu Glu Lys Met Asp
   370 375 380
   Gly Thr Glu Glu Leu Leu Val Lys Leu Asn Arg Glu Asp Leu Leu Arg
   385 390 395 400
   Lys Gln Arg Thr Phe Asp Asn Gly Ser Ile Pro His Gln Ile His Leu
   405 410 415
   Gly Glu Leu His Ala Ile Leu Arg Arg Gln Glu Asp Phe Tyr Pro Phe
   420 425 430
   Leu Lys Asp Asn Arg Glu Lys Ile Glu Lys Ile Leu Thr Phe Arg Ile
   435 440 445
   Pro Tyr Tyr Val Gly Pro Leu Ala Arg Gly Asn Ser Arg Phe Ala Trp
   450 455 460
   Met Thr Arg Lys Ser Glu Glu Thr Ile Thr Pro Trp Asn Phe Glu Glu
   465 470 475 480
   Val Val Asp Lys Gly Ala Ser Ala Gln Ser Phe Ile Glu Arg Met Thr
   485 490 495
   Asn Phe Asp Lys Asn Leu Pro Asn Glu Lys Val Leu Pro Lys His Ser
   500 505 510
   Leu Leu Tyr Glu Tyr Phe Thr Val Tyr Asn Glu Leu Thr Lys Val Lys
   515 520 525
   Tyr Val Thr Glu Gly Met Arg Lys Pro Ala Phe Leu Ser Gly Glu Gln
   530 535 540
   Lys Lys Ala Ile Val Asp Leu Leu Phe Lys Thr Asn Arg Lys Val Thr
   545 550 555 560
   Val Lys Gln Leu Lys Glu Asp Tyr Phe Lys Lys Ile Glu Cys Phe Asp
   565 570 575
   Ser Val Glu Ile Ser Gly Val Glu Asp Arg Phe Asn Ala Ser Leu Gly
   580 585 590
   Thr Tyr His Asp Leu Leu Lys Ile Ile Lys Asp Lys Asp Phe Leu Asp
   595 600 605
   Asn Glu Glu Asn Glu Asp Ile Leu Glu Asp Ile Val Leu Thr Leu Thr
   610 615 620
   Leu Phe Glu Asp Arg Glu Met Ile Glu Glu Arg Leu Lys Thr Tyr Ala
   625 630 635 640
   His Leu Phe Asp Asp Lys Val Met Lys Gln Leu Lys Arg Arg Arg Tyr
   645 650 655
   Thr Gly Trp Gly Arg Leu Ser Arg Lys Leu Ile Asn Gly Ile Arg Asp
   660 665 670
   Lys Gln Ser Gly Lys Thr Ile Leu Asp Phe Leu Lys Ser Asp Gly Phe
   675 680 685
   Ala Asn Arg Asn Phe Met Gln Leu Ile His Asp Asp Ser Leu Thr Phe
   690 695 700
   Lys Glu Asp Ile Gln Lys Ala Gln Val Ser Gly Gln Gly Asp Ser Leu
   705 710 715 720
   His Glu His Ile Ala Asn Leu Ala Gly Ser Pro Ala Ile Lys Lys Gly
   725 730 735
   Ile Leu Gln Thr Val Lys Val Val Asp Glu Leu Val Lys Val Met Gly
   740 745 750
   Arg His Lys Pro Glu Asn Ile Val Ile Glu Met Ala Arg Glu Asn Gln
   755 760 765
   Thr Thr Gln Lys Gly Gln Lys Asn Ser Arg Glu Arg Met Lys Arg Ile
   770 775 780
   Glu Glu Gly Ile Lys Glu Leu Gly Ser Gln Ile Leu Lys Glu His Pro
   785 790 795 800
   Val Glu Asn Thr Gln Leu Gln Asn Glu Lys Leu Tyr Leu Tyr Tyr Leu
   805 810 815
   Gln Asn Gly Arg Asp Met Tyr Val Asp Gln Glu Leu Asp Ile Asn Arg
   820 825 830
   Leu Ser Asp Tyr Asp Val Asp Ala Ile Val Pro Gln Ser Phe Leu Lys
   835 840 845
   Asp Asp Ser Ile Asp Asn Lys Val Leu Thr Arg Ser Asp Lys Asn Arg
   850 855 860
   Gly Lys Ser Asp Asn Val Pro Ser Glu Glu Val Val Lys Lys Met Lys
   865 870 875 880
   Asn Tyr Trp Arg Gln Leu Leu Asn Ala Lys Leu Ile Thr Gln Arg Lys
   885 890 895
   Phe Asp Asn Leu Thr Lys Ala Glu Arg Gly Gly Leu Ser Glu Leu Asp
   900 905 910
   Lys Ala Gly Phe Ile Lys Arg Gln Leu Val Glu Thr Arg Gln Ile Thr
   915 920 925
   Lys His Val Ala Gln Ile Leu Asp Ser Arg Met Asn Thr Lys Tyr Asp
   930 935 940
   Glu Asn Asp Lys Leu Ile Arg Glu Val Lys Val Ile Thr Leu Lys Ser
   945 950 955 960
   Lys Leu Val Ser Asp Phe Arg Lys Asp Phe Gln Phe Tyr Lys Val Arg
   965 970 975
   Glu Ile Asn Asn Tyr His His Ala His Asp Ala Tyr Leu Asn Ala Val
   980 985 990
   Val Gly Thr Ala Leu Ile Lys Lys Tyr Pro Lys Leu Glu Ser Glu Phe
   995 1000 1005
   Val Tyr Gly Asp Tyr Lys Val Tyr Asp Val Arg Lys Met Ile Ala
   1010 1015 1020
   Lys Ser Glu Gln Glu Ile Gly Lys Ala Thr Ala Lys Tyr Phe Phe
   1025 1030 1035
   Tyr Ser Asn Ile Met Asn Phe Phe Lys Thr Glu Ile Thr Leu Ala
   1040 1045 1050
   Asn Gly Glu Ile Arg Lys Arg Pro Leu Ile Glu Thr Asn Gly Glu
   1055 1060 1065
   Thr Gly  Glu Ile Val Trp Asp  Lys Gly Arg Asp Phe  Ala Thr Val
       1070                 1075                 1080
   Arg Lys  Val Leu Ser Met Pro  Gln Val Asn Ile Val  Lys Lys Thr
       1085                 1090                 1095
   Glu Val  Gln Thr Gly Gly Phe  Ser Lys Glu Ser Ile  Leu Pro Lys
       1100                 1105                 1110
   Arg Asn  Ser Asp Lys Leu Ile  Ala Arg Lys Lys Asp  Trp Asp Pro
       1115                 1120                 1125
   Lys Lys  Tyr Gly Gly Phe Asp  Ser Pro Thr Val Ala  Tyr Ser Val
       1130                 1135                 1140
   Leu Val  Val Ala Lys Val Glu  Lys Gly Lys Ser Lys  Lys Leu Lys
       1145                 1150                 1155
   Ser Val  Lys Glu Leu Leu Gly  Ile Thr Ile Met Glu  Arg Ser Ser
       1160                 1165                 1170
   Phe Glu  Lys Asn Pro Ile Asp  Phe Leu Glu Ala Lys  Gly Tyr Lys
       1175                 1180                 1185
   Glu Val  Lys Lys Asp Leu Ile  Ile Lys Leu Pro Lys  Tyr Ser Leu
       1190                 1195                 1200
   Phe Glu  Leu Glu Asn Gly Arg  Lys Arg Met Leu Ala  Ser Ala Gly
       1205                 1210                 1215
   Glu Leu  Gln Lys Gly Asn Glu  Leu Ala Leu Pro Ser  Lys Tyr Val
       1220                 1225                 1230
   Asn Phe  Leu Tyr Leu Ala Ser  His Tyr Glu Lys Leu  Lys Gly Ser
       1235                 1240                 1245
   Pro Glu  Asp Asn Glu Gln Lys  Gln Leu Phe Val Glu  Gln His Lys
       1250                 1255                 1260
   His Tyr  Leu Asp Glu Ile Ile  Glu Gln Ile Ser Glu  Phe Ser Lys
       1265                 1270                 1275
   Arg Val  Ile Leu Ala Asp Ala  Asn Leu Asp Lys Val  Leu Ser Ala
       1280                 1285                 1290
   Tyr Asn  Lys His Arg Asp Lys  Pro Ile Arg Glu Gln  Ala Glu Asn
           1295                1300                1305
   Ile Ile  His Leu Phe Thr Leu  Thr Asn Leu Gly Ala  Pro Ala Ala
       1310                1315                1320
   Phe Lys  Tyr Phe Asp Thr Thr  Ile Asp Arg Lys Arg  Tyr Thr Ser
       1325                1330                1335
   Thr Lys  Glu Val Leu Asp Ala  Thr Leu Ile His Gln  Ser Ile Thr
       1340                1345                1350
   Gly Leu  Tyr Glu Thr Arg Ile  Asp Leu Ser Gln Leu  Gly Gly Asp
       1355                1360                1365
<210> 4
   <211> 198
   <212> PRT
   <213> Flavobacterium okeanokoites
<400> 4
   Gly Ser Gln Leu Val Lys Ser Glu Leu Glu Glu Lys Lys Ser Glu Leu
   1               5                   10                  15
   Arg His Lys Leu Lys Tyr Val Pro His Glu Tyr Ile Glu Leu Ile Glu
               20                  25                  30
   Ile Ala Arg Asn Ser Thr Gln Asp Arg Ile Leu Glu Met Lys Val Met
           35                  40                  45
   Glu Phe Phe Met Lys Val Tyr Gly Tyr Arg Gly Lys His Leu Gly Gly
       50                  55                  60
   Ser Arg Lys Pro Asp Gly Ala Ile Tyr Thr Val Gly Ser Pro Ile Asp
   65                  70                  75                  80
   Tyr Gly Val Ile Val Asp Thr Lys Ala Tyr Ser Gly Gly Tyr Asn Leu
                   85                  90                  95
   Pro Ile Gly Gln Ala Asp Glu Met Gln Arg Tyr Val Glu Glu Asn Gln
               100                 105                 110
   Thr Arg Asn Lys His Ile Asn Pro Asn Glu Trp Trp Lys Val Tyr Pro
           115                 120                 125
   Ser Ser Val Thr Glu Phe Lys Phe Leu Phe Val Ser Gly His Phe Lys
       130                 135                 140
   Gly Asn Tyr Lys Ala Gln Leu Thr Arg Leu Asn His Ile Thr Asn Cys
   145                 150                 155                 160
   Asn Gly Ala Val Leu Ser Val Glu Glu Leu Leu Ile Gly Gly Glu Met
                   165                 170                 175
   Ile Lys Ala Gly Thr Leu Thr Leu Glu Glu Val Arg Arg Lys Phe Asn
               180                 185                 190
   Asn Gly Glu Ile Asn Phe
           195
<210> 5
   <211> 198
   <212> PRT
   <213> Unknown
<220>
   <223> Mutant
<400> 5
   Gly Ser Gln Leu Val Lys Ser Glu Leu Glu Glu Lys Lys Ser Glu Leu
   1               5                   10                  15
   Arg His Lys Leu Lys Tyr Val Pro His Glu Tyr Ile Glu Leu Ile Glu
               20                  25                  30
   Ile Ala Arg Asn Ser Thr Gln Asp Arg Ile Leu Glu Met Lys Val Met
           35                  40                  45
   Glu Phe Phe Met Lys Val Tyr Gly Tyr Arg Gly Lys His Leu Gly Gly
       50                  55                  60
   Ser Arg Lys Pro Ala Gly Ala Ile Tyr Thr Val Gly Ser Pro Ile Asp
   65                  70                  75                  80
   Tyr Gly Val Ile Val Asp Thr Lys Ala Tyr Ser Gly Gly Tyr Asn Leu
                   85                  90                  95
   Pro Ile Gly Gln Ala Asp Glu Met Gln Arg Tyr Val Glu Glu Asn Gln
               100                 105                 110
   Thr Arg Asn Lys His Ile Asn Pro Asn Glu Trp Trp Lys Val Tyr Pro
           115                 120                 125
   Ser Ser Val Thr Glu Phe Lys Phe Leu Phe Val Ser Gly His Phe Lys
       130                 135                 140
   Gly Asn Tyr Lys Ala Gln Leu Thr Arg Leu Asn His Ile Thr Asn Cys
   145                 150                 155                 160
   Asn Gly Ala Val Leu Ser Val Glu Glu Leu Leu Ile Gly Gly Glu Met
                   165                 170                 175
   Ile Lys Ala Gly Thr Leu Thr Leu Glu Glu Val Arg Arg Lys Phe Asn
               180                 185                 190
   Asn Gly Glu Ile Asn Phe
           195
<210> 6
   <211> 4212
   <212> DNA
   <213> Streptococcus pyogenes
<400> 6
   atggataaaa agtattctat tggtttagac atcggcacta attccgttgg atgggctgtc      60
   ataaccgatg aatacaaagt accttcaaag aaatttaagg tgttggggaa cacagaccgt     120
   cattcgatta aaaagaatct tatcggtgcc ctcctattcg atagtggcga aacggcagag     180
   gcgactcgcc tgaaacgaac cgctcggaga aggtatacac gtcgcaagaa ccgaatatgt     240
   tacttacaag aaatttttag caatgagatg gccaaagttg acgattcttt ctttcaccgt     300
   ttggaagagt ccttccttgt cgaagaggac aagaaacatg aacggcaccc catctttgga     360
   aacatagtag atgaggtggc atatcatgaa aagtacccaa cgatttatca cctcagaaaa     420
   aagctagttg actcaactga taaagcggac ctgaggttaa tctacttggc tcttgcccat     480
   atgataaagt tccgtgggca ctttctcatt gagggtgatc taaatccgga caactcggat     540
   gtcgacaaac tgttcatcca gttagtacaa acctataatc agttgtttga agagaaccct     600
   ataaatgcaa gtggcgtgga tgcgaaggct attcttagcg cccgcctctc taaatcccga     660
   cggctagaaa acctgatcgc acaattaccc ggagagaaga aaaatgggtt gttcggtaac     720
   cttatagcgc tctcactagg cctgacacca aattttaagt cgaacttcga cttagctgaa     780
   gatgccaaat tgcagcttag taaggacacg tacgatgacg atctcgacaa tctactggca     840
   caaattggag atcagtatgc ggacttattt ttggctgcca aaaaccttag cgatgcaatc     900
   ctcctatctg acatactgag agttaatact gagattacca aggcgccgtt atccgcttca     960
   atgatcaaaa ggtacgatga acatcaccaa gacttgacac ttctcaaggc cctagtccgt    1020
   cagcaactgc ctgagaaata taaggaaata ttctttgatc agtcgaaaaa cgggtacgca    1080
   ggttatattg acggcggagc gagtcaagag gaattctaca agtttatcaa acccatatta    1140
   gagaagatgg atgggacgga agagttgctt gtaaaactca atcgcgaaga tctactgcga    1200
   aagcagcgga ctttcgacaa cggtagcatt ccacatcaaa tccacttagg cgaattgcat    1260
   gctatactta gaaggcagga ggatttttat ccgttcctca aagacaatcg tgaaaagatt    1320
   gagaaaatcc taacctttcg cataccttac tatgtgggac ccctggcccg agggaactct 1380
   cggttcgcat ggatgacaag aaagtccgaa gaaacgatta ctccatggaa ttttgaggaa 1440
   gttgtcgata aaggtgcgtc agctcaatcg ttcatcgaga ggatgaccaa ctttgacaag 1500
   aatttaccga acgaaaaagt attgcctaag cacagtttac tttacgagta tttcacagtg 1560
   tacaatgaac tcacgaaagt taagtatgtc actgagggca tgcgtaaacc cgcctttcta 1620
   agcggagaac agaagaaagc aatagtagat ctgttattca agaccaaccg caaagtgaca 1680
   gttaagcaat tgaaagagga ctactttaag aaaattgaat gcttcgattc tgtcgagatc 1740
   tccggggtag aagatcgatt taatgcgtca cttggtacgt atcatgacct cctaaagata 1800
   attaaagata aggacttcct ggataacgaa gagaatgaag atatcttaga agatatagtg 1860
   ttgactctta ccctctttga agatcgggaa atgattgagg aaagactaaa aacatacgct 1920
   cacctgttcg acgataaggt tatgaaacag ttaaagaggc gtcgctatac gggctgggga 1980
   cgattgtcgc ggaaacttat caacgggata agagacaagc aaagtggtaa aactattctc 2040
   gattttctaa agagcgacgg cttcgccaat aggaacttta tgcagctgat ccatgatgac 2100
   tctttaacct tcaaagagga tatacaaaag gcacaggttt ccggacaagg ggactcattg 2160
   cacgaacata ttgcgaatct tgctggttcg ccagccatca aaaagggcat actccagaca 2220
   gtcaaagtag tggatgagct agttaaggtc atgggacgtc acaaaccgga aaacattgta 2280
   atcgagatgg cacgcgaaaa tcaaacgact cagaaggggc aaaaaaacag tcgagagcgg 2340
   atgaagagaa tagaagaggg tattaaagaa ctgggcagcc agatcttaaa ggagcatcct 2400
   gtggaaaata cccaattgca gaacgagaaa ctttacctct attacctaca aaatggaagg 2460
   gacatgtatg ttgatcagga actggacata aaccgtttat ctgattacga cgtcgatcac 2520
   attgtacccc aatccttttt gaaggacgat tcaatcgaca ataaagtgct tacacgctcg 2580
   gataagaacc gagggaaaag tgacaatgtt ccaagcgagg aagtcgtaaa gaaaatgaag 2640
   aactattggc ggcagctcct aaatgcgaaa ctgataacgc aaagaaagtt cgataactta 2700
   actaaagctg agaggggtgg cttgtctgaa cttgacaagg ccggatttat taaacgtcag 2760
   ctcgtggaaa cccgccaaat cacaaagcat gttgcacaga tactagattc ccgaatgaat 2820
   acgaaatacg acgagaacga taagctgatt cgggaagtca aagtaatcac tttaaagtca 2880
   aaattggtgt cggacttcag aaaggatttt caattctata aagttaggga gataaataac 2940
   taccaccatg cgcacgacgc ttatcttaat gccgtcgtag ggaccgcact cattaagaaa 3000
   tacccgaagc tagaaagtga gtttgtgtat ggtgattaca aagtttatga cgtccgtaag 3060
   atgatcgcga aaagcgaaca ggagataggc aaggctacag ccaaatactt cttttattct 3120
   aacattatga atttctttaa gacggaaatc actctggcaa acggagagat acgcaaacga 3180
   cctttaattg aaaccaatgg ggagacaggt gaaatcgtat gggataaggg ccgggacttc 3240
   gcgacggtga gaaaagtttt gtccatgccc caagtcaaca tagtaaagaa aactgaggtg 3300
   cagaccggag ggttttcaaa ggaatcgatt cttccaaaaa ggaatagtga taagctcatc 3360
   gctcgtaaaa aggactggga cccgaaaaag tacggtggct tcgatagccc tacagttgcc 3420
   tattctgtcc tagtagtggc aaaagttgag aagggaaaat ccaagaaact gaagtcagtc 3480
   aaagaattat tggggataac gattatggag cgctcgtctt ttgaaaagaa ccccatcgac 3540
   ttccttgagg cgaaaggtta caaggaagta aaaaaggatc tcataattaa actaccaaag 3600
   tatagtctgt ttgagttaga aaatggccga aaacggatgt tggctagcgc cggagagctt 3660
   caaaagggga acgaactcgc actaccgtct aaatacgtga atttcctgta tttagcgtcc 3720
   cattacgaga agttgaaagg ttcacctgaa gataacgaac agaagcaact ttttgttgag 3780
   cagcacaaac attatctcga cgaaatcata gagcaaattt cggaattcag taagagagtc 3840
   atcctagctg atgccaatct ggacaaagta ttaagcgcat acaacaagca cagggataaa 3900
   cccatacgtg agcaggcgga aaatattatc catttgttta ctcttaccaa cctcggcgct 3960
   ccagccgcat tcaagtattt tgacacaacg atagatcgca aacgatacac ttctaccaag 4020
   gaggtgctag acgcgacact gattcaccaa tccatcacgg gattatatga aactcggata 4080
   gatttgtcac agcttggggg tgacggatcc cccaagaaga agaggaaagt ctcgagcgac 4140
   tacaaagacc atgacggtga ttataaagat catgacatcg attacaagga tgacgatgac 4200
   aaggctgcag ga 4212
<210> 7
   <211> 4221
   <212> DNA
   <213> Unknown
<220>
   <223> Mutant
<400> 7
   atggactata aggaccacga cggagactac aaggatcatg atattgatta caaagacgat 60
   gacgataaga tggccccaaa gaagaagcgg aaggtcggta tccacggagt cccagcagcc 120
   gataaaaagt attctattgg tttagctatc ggcactaatt ccgttggatg ggctgtcata 180
   accgatgaat acaaagtacc ttcaaagaaa tttaaggtgt tggggaacac agaccgtcat 240
   tcgattaaaa agaatcttat cggtgccctc ctattcgata gtggcgaaac ggcagaggcg 300
   actcgcctga aacgaaccgc tcggagaagg tatacacgtc gcaagaaccg aatatgttac 360
   ttacaagaaa tttttagcaa tgagatggcc aaagttgacg attctttctt tcaccgtttg 420
   gaagagtcct tccttgtcga agaggacaag aaacatgaac ggcaccccat ctttggaaac 480
   atagtagatg aggtggcata tcatgaaaag tacccaacga tttatcacct cagaaaaaag 540
   ctagttgact caactgataa agcggacctg aggttaatct acttggctct tgcccatatg 600
   ataaagttcc gtgggcactt tctcattgag ggtgatctaa atccggacaa ctcggatgtc 660
   gacaaactgt tcatccagtt agtacaaacc tataatcagt tgtttgaaga gaaccctata 720
   aatgcaagtg gcgtggatgc gaaggctatt cttagcgccc gcctctctaa atcccgacgg 780
   ctagaaaacc tgatcgcaca attacccgga gagaagaaaa atgggttgtt cggtaacctt 840
   atagcgctct cactaggcct gacaccaaat tttaagtcga acttcgactt agctgaagat 900
   gccaaattgc agcttagtaa ggacacgtac gatgacgatc tcgacaatct actggcacaa 960
   attggagatc agtatgcgga cttatttttg gctgccaaaa accttagcga tgcaatcctc 1020
   ctatctgaca tactgagagt taatactgag attaccaagg cgccgttatc cgcttcaatg 1080
   atcaaaaggt acgatgaaca tcaccaagac ttgacacttc tcaaggccct agtccgtcag 1140
   caactgcctg agaaatataa ggaaatattc tttgatcagt cgaaaaacgg gtacgcaggt 1200
   tatattgacg gcggagcgag tcaagaggaa ttctacaagt ttatcaaacc catattagag 1260
   aagatggatg ggacggaaga gttgcttgta aaactcaatc gcgaagatct actgcgaaag 1320
   cagcggactt tcgacaacgg tagcattcca catcaaatcc acttaggcga attgcatgct 1380
   atacttagaa ggcaggagga tttttatccg ttcctcaaag acaatcgtga aaagattgag 1440
   aaaatcctaa cctttcgcat accttactat gtgggacccc tggcccgagg gaactctcgg 1500
   ttcgcatgga tgacaagaaa gtccgaagaa acgattactc catggaattt tgaggaagtt 1560
   gtcgataaag gtgcgtcagc tcaatcgttc atcgagagga tgaccaactt tgacaagaat 1620
   ttaccgaacg aaaaagtatt gcctaagcac agtttacttt acgagtattt cacagtgtac 1680
   aatgaactca cgaaagttaa gtatgtcact gagggcatgc gtaaacccgc ctttctaagc 1740
   ggagaacaga agaaagcaat agtagatctg ttattcaaga ccaaccgcaa agtgacagtt 1800
   aagcaattga aagaggacta ctttaagaaa attgaatgct tcgattctgt cgagatctcc 1860
   ggggtagaag atcgatttaa tgcgtcactt ggtacgtatc atgacctcct aaagataatt 1920
   aaagataagg acttcctgga taacgaagag aatgaagata tcttagaaga tatagtgttg 1980
   actcttaccc tctttgaaga tcgggaaatg attgaggaaa gactaaaaac atacgctcac 2040
   ctgttcgacg ataaggttat gaaacagtta aagaggcgtc gctatacggg ctggggacga 2100
   ttgtcgcgga aacttatcaa cgggataaga gacaagcaaa gtggtaaaac tattctcgat 2160
   tttctaaaga gcgacggctt cgccaatagg aactttatgc agctgatcca tgatgactct 2220
   ttaaccttca aagaggatat acaaaaggca caggtttccg gacaagggga ctcattgcac 2280
   gaacatattg cgaatcttgc tggttcgcca gccatcaaaa agggcatact ccagacagtc 2340
   aaagtagtgg atgagctagt taaggtcatg ggacgtcaca aaccggaaaa cattgtaatc 2400
   gagatggcac gcgaaaatca aacgactcag aaggggcaaa aaaacagtcg agagcggatg 2460
   aagagaatag aagagggtat taaagaactg ggcagccaga tcttaaagga gcatcctgtg 2520
   gaaaataccc aattgcagaa cgagaaactt tacctctatt acctacaaaa tggaagggac 2580
   atgtatgttg atcaggaact ggacataaac cgtttatctg attacgacgt cgatcacatt 2640
   gtaccccaat cctttttgaa ggacgattca atcgacaata aagtgcttac acgctcggat 2700
   aagaaccgag ggaaaagtga caatgttcca agcgaggaag tcgtaaagaa aatgaagaac 2760
   tattggcggc agctcctaaa tgcgaaactg ataacgcaaa gaaagttcga taacttaact 2820
   aaagctgaga ggggtggctt gtctgaactt gacaaggccg gatttattaa acgtcagctc 2880
   gtggaaaccc gccaaatcac aaagcatgtt gcacagatac tagattcccg aatgaatacg 2940
   aaatacgacg agaacgataa gctgattcgg gaagtcaaag taatcacttt aaagtcaaaa 3000
   ttggtgtcgg acttcagaaa ggattttcaa ttctataaag ttagggagat aaataactac 3060
   caccatgcgc acgacgctta tcttaatgcc gtcgtaggga ccgcactcat taagaaatac 3120
   ccgaagctag aaagtgagtt tgtgtatggt gattacaaag tttatgacgt ccgtaagatg 3180
   atcgcgaaaa gcgaacagga gataggcaag gctacagcca aatacttctt ttattctaac 3240
   attatgaatt tctttaagac ggaaatcact ctggcaaacg gagagatacg caaacgacct 3300
   ttaattgaaa ccaatgggga gacaggtgaa atcgtatggg ataagggccg ggacttcgcg 3360
   acggtgagaa aagttttgtc catgccccaa gtcaacatag taaagaaaac tgaggtgcag 3420
   accggagggt tttcaaagga atcgattctt ccaaaaagga atagtgataa gctcatcgct 3480
   cgtaaaaagg actgggaccc gaaaaagtac ggtggcttcg atagccctac agttgcctat 3540
   tctgtcctag tagtggcaaa agttgagaag ggaaaatcca agaaactgaa gtcagtcaaa 3600
   gaattattgg ggataacgat tatggagcgc tcgtcttttg aaaagaaccc catcgacttc 3660
   cttgaggcga aaggttacaa ggaagtaaaa aaggatctca taattaaact accaaagtat 3720
   agtctgtttg agttagaaaa tggccgaaaa cggatgttgg ctagcgccgg agagcttcaa 3780
   aaggggaacg aactcgcact accgtctaaa tacgtgaatt tcctgtattt agcgtcccat 3840
   tacgagaagt tgaaaggttc acctgaagat aacgaacaga agcaactttt tgttgagcag 3900
   cacaaacatt atctcgacga aatcatagag caaatttcgg aattcagtaa gagagtcatc 3960
   ctagctgatg ccaatctgga caaagtatta agcgcataca acaagcacag ggataaaccc 4020
   atacgtgagc aggcggaaaa tattatccat ttgtttactc ttaccaacct cggcgctcca 4080
   gccgcattca agtattttga cacaacgata gatcgcaaac gatacacttc taccaaggag 4140
   gtgctagacg cgacactgat tcaccaatcc atcacgggat tatatgaaac tcggatagat 4200
   ttgtcacagc ttgggggtga c 4221
<210> 8
   <211> 4834
   <212> DNA
   <213> Unknown
<220>
   <223> Synthetic
<400> 8
   atggataaaa agtattctat tggtttagct atcggcacta attccgttgg atgggctgtc 60
   ataaccgatg aatacaaagt accttcaaag aaatttaagg tgttggggaa cacagaccgt 120
   cattcgatta aaaagaatct tatcggtgcc ctcctattcg atagtggcga aacggcagag 180
   gcgactcgcc tgaaacgaac cgctcggaga aggtatacac gtcgcaagaa ccgaatatgt 240
   tacttacaag aaatttttag caatgagatg gccaaagttg acgattcttt ctttcaccgt 300
   ttggaagagt ccttccttgt cgaagaggac aagaaacatg aacggcaccc catctttgga 360
   aacatagtag atgaggtggc atatcatgaa aagtacccaa cgatttatca cctcagaaaa 420
   aagctagttg actcaactga taaagcggac ctgaggttaa tctacttggc tcttgcccat 480
   atgataaagt tccgtgggca ctttctcatt gagggtgatc taaatccgga caactcggat 540
   gtcgacaaac tgttcatcca gttagtacaa acctataatc agttgtttga agagaaccct 600
   ataaatgcaa gtggcgtgga tgcgaaggct attcttagcg cccgcctctc taaatcccga 660
   cggctagaaa acctgatcgc acaattaccc ggagagaaga aaaatgggtt gttcggtaac 720
   cttatagcgc tctcactagg cctgacacca aattttaagt cgaacttcga cttagctgaa 780
   gatgccaaat tgcagcttag taaggacacg tacgatgacg atctcgacaa tctactggca 840
   caaattggag atcagtatgc ggacttattt ttggctgcca aaaaccttag cgatgcaatc 900
   ctcctatctg acatactgag agttaatact gagattacca aggcgccgtt atccgcttca 960
   atgatcaaaa ggtacgatga acatcaccaa gacttgacac ttctcaaggc cctagtccgt 1020
   cagcaactgc ctgagaaata taaggaaata ttctttgatc agtcgaaaaa cgggtacgca 1080
   ggttatattg acggcggagc gagtcaagag gaattctaca agtttatcaa acccatatta 1140
   gagaagatgg atgggacgga agagttgctt gtaaaactca atcgcgaaga tctactgcga 1200
   aagcagcgga ctttcgacaa cggtagcatt ccacatcaaa tccacttagg cgaattgcat 1260
   gctatactta gaaggcagga ggatttttat ccgttcctca aagacaatcg tgaaaagatt 1320
   gagaaaatcc taacctttcg cataccttac tatgtgggac ccctggcccg agggaactct 1380
   cggttcgcat ggatgacaag aaagtccgaa gaaacgatta ctccatggaa ttttgaggaa 1440
   gttgtcgata aaggtgcgtc agctcaatcg ttcatcgaga ggatgaccaa ctttgacaag 1500
   aatttaccga acgaaaaagt attgcctaag cacagtttac tttacgagta tttcacagtg 1560
   tacaatgaac tcacgaaagt taagtatgtc actgagggca tgcgtaaacc cgcctttcta 1620
   agcggagaac agaagaaagc aatagtagat ctgttattca agaccaaccg caaagtgaca 1680
   gttaagcaat tgaaagagga ctactttaag aaaattgaat gcttcgattc tgtcgagatc 1740
   tccggggtag aagatcgatt taatgcgtca cttggtacgt atcatgacct cctaaagata 1800
   attaaagata aggacttcct ggataacgaa gagaatgaag atatcttaga agatatagtg 1860
   ttgactctta ccctctttga agatcgggaa atgattgagg aaagactaaa aacatacgct 1920
   cacctgttcg acgataaggt tatgaaacag ttaaagaggc gtcgctatac gggctggggc 1980
   gattgtcgcg gaaacttatc aacgggataa gagacaagca aagtggtaaa actattctcg 2040
   attttctaaa gagcgacggc ttcgccaata ggaactttat gcagctgatc catgatgact 2100
   ctttaacctt caaagaggat atacaaaagg cacaggtttc cggacaaggg gactcattgc 2160
   acgaacatat tgcgaatctt gctggttcgc cagccatcaa aaagggcata ctccagacag 2220
   tcaaagtagt ggatgagcta gttaaggtca tgggacgtca caaaccggaa aacattgtaa 2280
   tcgagatggc acgcgaaaat caaacgactc agaaggggca aaaaaacagt cgagagcgga 2340
   tgaagagaat agaagagggt attaaagaac tgggcagcca gatcttaaag gagcatcctg 2400
   tggaaaatac ccaattgcag aacgagaaac tttacctcta ttacctacaa aatggaaggg 2460
   acatgtatgt tgatcaggaa ctggacataa accgtttatc tgattacgac gtcgatgcca 2520
   ttgtacccca atcctttttg aaggacgatt caatcgacaa taaagtgctt acacgctcgg 2580
   ataagaaccg agggaaaagt gacaatgttc caagcgagga agtcgtaaag aaaatgaaga 2640
   actattggcg gcagctccta aatgcgaaac tgataacgca aagaaagttc gataacttaa 2700
   ctaaagctga gaggggtggc ttgtctgaac ttgacaaggc cggatttatt aaacgtcagc 2760
   tcgtggaaac ccgccaaatc acaaagcatg ttgcacagat actagattcc cgaatgaata 2820
   cgaaatacga cgagaacgat aagctgattc gggaagtcaa agtaatcact ttaaagtcaa 2880
   aattggtgtc ggacttcaga aaggattttc aattctataa agttagggag ataaataact 2940
   accaccatgc gcacgacgct tatcttaatg ccgtcgtagg gaccgcactc attaaaaata 3000
   cccgaagcta gaaagtgagt ttgtgtatgg tgattacaaa gtttatgacg tccgtaagat 3060
   gatcgcgaaa agcgaacagg agataggcaa ggctacagcc aaatacttct tttattctaa 3120
   cattatgaat ttctttaaga cggaaatcac tctggcaaac ggagagatac gcaaacgacc 3180
   tttaattgaa accaatgggg agacaggtga aatcgtatgg gataagggcc gggacttcgc 3240
   gacggtgaga aaagttttgt ccatgcccca agtcaacata gtaaagaaaa ctgaggtgca 3300
   gaccggaggg ttttcaaagg aatcgattct tccaaaaagg aatagtgata agctcatcgc 3360
   tcgtaaaaag gactgggacc cgaaaaagta cggtggcttc gatagcccta cagttgccta 3420
   ttctgtccta gtagtggcaa aagttgagaa gggaaaatcc aagaaactga agtcagtcaa 3480
   agaattattg gggataacga ttatggagcg ctcgtctttt gaaaagaacc ccatcgactt 3540
   ccttgaggcg aaaggttaca aggaagtaaa aaaggatctc ataattaaac taccaaagta 3600
   tagtctgttt gagttagaaa atggccgaaa acggatgttg gctagcgccg gagagcttca 3660
   aaaggggaac gaactcgcac taccgtctaa atacgtgaat ttcctgtatt tagcgtccca 3720
   ttacgagaag ttgaaaggtt cacctgaaga taacgaacag aagcaacttt ttgttgagca 3780
   gcacaaacat tatctcgacg aaatcataga gcaaatttcg gaattcagta agagagtcat 3840
   cctagctgat gccaatctgg acaaagtatt aagcgcatac aacaagcaca gggataaacc 3900
   catacgtgag caggcggaaa atattatcca tttgtttact cttaccaacc tcggcgctcc 3960
   agccgcattc aagtattttg acacaacgat agatcgcaaa cgatacactt ctaccaagga 4020
   ggtgctagac gcgacactga ttcaccaatc catcacggga ttatatgaaa ctcggataga 4080
   tttgtcacag cttgggggtg acggatcccc caagaagaag aggaaagtct cgagcgacta 4140
   caaagaccat gacggtgatt ataaagatca tgacatcgat tacaaggatg acgatgacaa 4200
   ggctgcagga tcaggtggaa gtggcggcag cggaggttct ggatcccaac tagtcaaaag 4260
   tgaactggag gagaagaaat ctgaacttcg tcataaattg aaatatgtgc ctcatgaata 4320
   tattgaatta attgaaattg ccagaaattc cactcaggat agaattcttg aaatgaaggt 4380
   aatggaattt tttatgaaag tttatggata tagaggtaaa catttgggtg gatcaaggaa 4440
   accggacgga gcaatttata ctgtcggatc tcctattgat tacggtgtga tcgtggatac 4500
   taaagcttat agcggaggtt ataatctgcc aattggccaa gcagatgaaa tgcaacgata 4560
   tgtcgaagaa aatcaaacac gaaacaaaca tatcaaccct aatgaatggt ggaaagtcta 4620
   tccatcttct gtaacggaat ttaagttttt atttgtgagt ggtcacttta aaggaaacta 4680
   caaagctcag cttacacgat taaatcatat cactaattgt aatggagctg ttcttagtgt 4740
   agaagagctt ttaattggtg gagaaatgat taaagccggc acattaacct tagaggaagt 4800
   cagacggaaa tttaataacg gcgagataaa cttt 4834
<210> 9
   <211> 4845
   <212> DNA
   <213> Unknown
<220>
   <223> Synthetic
<400> 9
   atggactaca aagaccatga cggtgattat aaagatcatg acatcgatta caaggatgac      60
   gatgacaaga tggcccccaa gaagaagagg aaggtgggca ttcaccgcgg ggtacctatg     120
   gataaaaagt attctattgg tttagctatc ggcactaatt ccgttggatg ggctgtcata     180
   accgatgaat acaaagtacc ttcaaagaaa tttaaggtgt tggggaacac agaccgtcat     240
   tcgattaaaa agaatcttat cggtgccctc ctattcgata gtggcgaaac ggcagaggcg      300
   actcgcctga aacgaaccgc tcggagaagg tatacacgtc gcaagaaccg aatatgttac      360
   ttacaagaaa tttttagcaa tgagatggcc aaagttgacg attctttctt tcaccgtttg      420
   gaagagtcct tccttgtcga agaggacaag aaacatgaac ggcaccccat ctttggaaac      480
   atagtagatg aggtggcata tcatgaaaag tacccaacga tttatcacct cagaaaaaag      540
   ctagttgact caactgataa agcggacctg aggttaatct acttggctct tgcccatatg      600
   ataaagttcc gtgggcactt tctcattgag ggtgatctaa atccggacaa ctcggatgtc      660
   gacaaactgt tcatccagtt agtacaaacc tataatcagt tgtttgaaga gaaccctata      720
   aatgcaagtg gcgtggatgc gaaggctatt cttagcgccc gcctctctaa atcccgacgg      780
   ctagaaaacc tgatcgcaca attacccgga gagaagaaaa atgggttgtt cggtaacctt      840
   atagcgctct cactaggcct gacaccaaat tttaagtcga acttcgactt agctgaagat      900
   gccaaattgc agcttagtaa ggacacgtac gatgacgatc tcgacaatct actggcacaa      960
   attggagatc agtatgcgga cttatttttg gctgccaaaa accttagcga tgcaatcctc     1020
   ctatctgaca tactgagagt taatactgag attaccaagg cgccgttatc cgcttcaatg     1080
   atcaaaaggt acgatgaaca tcaccaagac ttgacacttc tcaaggccct agtccgtcag     1140
   caactgcctg agaaatataa ggaaatattc tttgatcagt cgaaaaacgg gtacgcaggt     1200
   tatattgacg gcggagcgag tcaagaggaa ttctacaagt ttatcaaacc catattagag     1260
   aagatggatg ggacggaaga gttgcttgta aaactcaatc gcgaagatct actgcgaaag     1320
   cagcggactt tcgacaacgg tagcattcca catcaaatcc acttaggcga attgcatgct     1380
   atacttagaa ggcaggagga tttttatccg ttcctcaaag acaatcgtga aaagattgag     1440
   aaaatcctaa cctttcgcat accttactat gtgggacccc tggcccgagg gaactctcgg     1500
   ttcgcatgga tgacaagaaa gtccgaagaa acgattactc catggaattt tgaggaagtt     1560
   gtcgataaag gtgcgtcagc tcaatcgttc atcgagagga tgaccaactt tgacaagaat     1620
   ttaccgaacg aaaaagtatt gcctaagcac agtttacttt acgagtattt cacagtgtac     1680
   aatgaactca cgaaagttaa gtatgtcact gagggcatgc gtaaacccgc ctttctaagc     1740
   ggagaacaga agaaagcaat agtagatctg ttattcaaga ccaaccgcaa agtgacagtt     1800
   aagcaattga aagaggacta ctttaagaaa attgaatgct tcgattctgt cgagatctcc     1860
   ggggtagaag atcgatttaa tgcgtcactt ggtacgtatc atgacctcct aaagataatt     1920
   aaagataagg acttcctgga taacgaagag aatgaagata tcttagaaga tatagtgttg     1980
   actcttaccc tctttgaaga tcgggaaatg attgaggaaa gactaaaaac atacgctcac     2040
   ctgttcgacg ataaggttat gaaacagtta aagaggcgtc gctatacggg ctggggacga     2100
   ttgtcgcgga aacttatcaa cgggataaga gacaagcaaa gtggtaaaac tattctcgat     2160
   tttctaaaga gcgacggctt cgccaatagg aactttatgc agctgatcca tgatgactct 2220
   ttaaccttca aagaggatat acaaaaggca caggtttccg gacaagggga ctcattgcac 2280
   gaacatattg cgaatcttgc tggttcgcca gccatcaaaa agggcatact ccagacagtc 2340
   aaagtagtgg atgagctagt taaggtcatg ggacgtcaca aaccggaaaa cattgtaatc 2400
   gagatggcac gcgaaaatca aacgactcag aaggggcaaa aaaacagtcg agagcggatg 2460
   aagagaatag aagagggtat taaagaactg ggcagccaga tcttaaagga gcatcctgtg 2520
   gaaaataccc aattgcagaa cgagaaactt tacctctatt acctacaaaa tggaagggac 2580
   atgtatgttg atcaggaact ggacataaac cgtttatctg attacgacgt cgatgccatt 2640
   gtaccccaat cctttttgaa ggacgattca atcgacaata aagtgcttac acgctcggat 2700
   aagaaccgag ggaaaagtga caatgttcca agcgaggaag tcgtaaagaa aatgaagaac 2760
   tattggcggc agctcctaaa tgcgaaactg ataacgcaaa gaaagttcga taacttaact 2820
   aaagctgaga ggggtggctt gtctgaactt gacaaggccg gatttattaa acgtcagctc 2880
   gtggaaaccc gccaaatcac aaagcatgtt gcacagatac tagattcccg aatgaatacg 2940
   aaatacgacg agaacgataa gctgattcgg gaagtcaaag taatcacttt aaagtcaaaa 3000
   ttggtgtcgg acttcagaaa ggattttcaa ttctataaag ttagggagat aaataactac 3060
   caccatgcgc acgacgctta tcttaatgcc gtcgtaggga ccgcactcat taagaaatac 3120
   ccgaagctag aaagtgagtt tgtgtatggt gattacaaag tttatgacgt ccgtaagatg 3180
   atcgcgaaaa gcgaacagga gataggcaag gctacagcca aatacttctt ttattctaac 3240
   attatgaatt tctttaagac ggaaatcact ctggcaaacg gagagatacg caaacgacct 3300
   ttaattgaaa ccaatgggga gacaggtgaa atcgtatggg ataagggccg ggacttcgcg 3360
   acggtgagaa aagttttgtc catgccccaa gtcaacatag taaagaaaac tgaggtgcag 3420
   accggagggt tttcaaagga atcgattctt ccaaaaagga atagtgataa gctcatcgct 3480
   cgtaaaaagg actgggaccc gaaaaagtac ggtggcttcg atagccctac agttgcctat 3540
   tctgtcctag tagtggcaaa agttgagaag ggaaaatcca agaaactgaa gtcagtcaaa 3600
   gaattattgg ggataacgat tatggagcgc tcgtcttttg aaaagaaccc catcgacttc 3660
   cttgaggcga aaggttacaa ggaagtaaaa aaggatctca taattaaact accaaagtat 3720
   agtctgtttg agttagaaaa tggccgaaaa cggatgttgg ctagcgccgg agagcttcaa 3780
   aaggggaacg aactcgcact accgtctaaa tacgtgaatt tcctgtattt agcgtcccat 3840
   tacgagaagt tgaaaggttc acctgaagat aacgaacaga agcaactttt tgttgagcag 3900
   cacaaacatt atctcgacga aatcatagag caaatttcgg aattcagtaa gagagtcatc 3960
   ctagctgatg ccaatctgga caaagtatta agcgcataca acaagcacag ggataaaccc 4020
   atacgtgagc aggcggaaaa tattatccat ttgtttactc ttaccaacct cggcgctcca 4080
   gccgcattca agtattttga cacaacgata gatcgcaaac gatacacttc taccaaggag 4140
   gtgctagacg cgacactgat tcaccaatcc atcacgggat tatatgaaac tcggatagat 4200
   ttgtcacagc ttgggggtga ctcaggtgga agtggcggca gcggaggttc tggatcccaa 4260
   ctagtcaaaa gtgaactgga ggagaagaaa tctgaacttc gtcataaatt gaaatatgtg 4320
   cctcatgaat atattgaatt aattgaaatt gccagaaatt ccactcagga tagaattctt 4380
   gaaatgaagg taatggaatt ttttatgaaa gtttatggat atagaggtaa acatttgggt 4440
   ggatcaagga aaccggacgg agcaatttat actgtcggat ctcctattga ttacggtgtg 4500
   atcgtggata ctaaagctta tagcggaggt tataatctgc caattggcca agcagatgaa 4560
   atgcaacgat atgtcgaaga aaatcaaaca cgaaacaaac atatcaaccc taatgaatgg 4620
   tggaaagtct atccatcttc tgtaacggaa tttaagtttt tatttgtgag tggtcacttt 4680
   aaaggaaact acaaagctca gcttacacga ttaaatcata tcactaattg taatggagct 4740
   gttcttagtg tagaagagct tttaattggt ggagaaatga ttaaagccgg cacattaacc 4800
   ttagaggaag tcagacggaa atttaataac ggcgagataa acttt 4845
<210> 10
   <211> 4836
   <212> DNA
   <213> Unknown
<220>
   <223> Synthetic
<400> 10
   atgggatccc aactagtcaa aagtgaactg gaggagaaga aatctgaact tcgtcataaa 60
   ttgaaatatg tgcctcatga atatattgaa ttaattgaaa ttgccagaaa ttccactcag 120
   gatagaattc ttgaaatgaa ggtaatggaa ttttttatga aagtttatgg atatagaggt 180
   aaacatttgg gtggatcaag gaaaccggac ggagcaattt atactgtcgg atctcctatt 240
   gattacggtg tgatcgtgga tactaaagct tatagcggag gttataatct gccaattggc 300
   caagcagatg aaatgcaacg atatgtcgaa gaaaatcaaa cacgaaacaa acatatcaac 360
   cctaatgaat ggtggaaagt ctatccatct tctgtaacgg aatttaagtt tttatttgtg 420
   agtggtcact ttaaaggaaa ctacaaagct cagcttacac gattaaatca tatcactaat 480
   tgtaatggag ctgttcttag tgtagaagag cttttaattg gtggagaaat gattaaagcc 540
   ggcacattaa ccttagagga agtcagacgg aaatttaata acggcgagat aaactttggc 600
   ggtagtgggg gatctggggg aagtatggat aaaaagtatt ctattggttt agctatcggc 660
   actaattccg ttggatgggc tgtcataacc gatgaataca aagtaccttc aaagaaattt 720
   aaggtgttgg ggaacacaga ccgtcattcg attaaaaaga atcttatcgg tgccctccta 780
   ttcgatagtg gcgaaacggc agaggcgact cgcctgaaac gaaccgctcg gagaaggtat      840
   acacgtcgca agaaccgaat atgttactta caagaaattt ttagcaatga gatggccaaa      900
   gttgacgatt ctttctttca ccgtttggaa gagtccttcc ttgtcgaaga ggacaagaaa      960
   catgaacggc accccatctt tggaaacata gtagatgagg tggcatatca tgaaaagtac     1020
   ccaacgattt atcacctcag aaaaaagcta gttgactcaa ctgataaagc ggacctgagg     1080
   ttaatctact tggctcttgc ccatatgata aagttccgtg ggcactttct cattgagggt     1140
   gatctaaatc cggacaactc ggatgtcgac aaactgttca tccagttagt acaaacctat     1200
   aatcagttgt ttgaagagaa ccctataaat gcaagtggcg tggatgcgaa ggctattctt     1260
   agcgcccgcc tctctaaatc ccgacggcta gaaaacctga tcgcacaatt acccggagag     1320
   aagaaaaatg ggttgttcgg taaccttata gcgctctcac taggcctgac accaaatttt     1380
   aagtcgaact tcgacttagc tgaagatgcc aaattgcagc ttagtaagga cacgtacgat     1440
   gacgatctcg acaatctact ggcacaaatt ggagatcagt atgcggactt atttttggct     1500
   gccaaaaacc ttagcgatgc aatcctccta tctgacatac tgagagttaa tactgagatt     1560
   accaaggcgc cgttatccgc ttcaatgatc aaaaggtacg atgaacatca ccaagacttg     1620
   acacttctca aggccctagt ccgtcagcaa ctgcctgaga aatataagga aatattcttt     1680
   gatcagtcga aaaacgggta cgcaggttat attgacggcg gagcgagtca agaggaattc     1740
   tacaagttta tcaaacccat attagagaag atggatggga cggaagagtt gcttgtaaaa     1800
   ctcaatcgcg aagatctact gcgaaagcag cggactttcg acaacggtag cattccacat     1860
   caaatccact taggcgaatt gcatgctata cttagaaggc aggaggattt ttatccgttc     1920
   ctcaaagaca atcgtgaaaa gattgagaaa atcctaacct ttcgcatacc ttactatgtg     1980
   ggacccctgg cccgagggaa ctctcggttc gcatggatga caagaaagtc cgaagaaacg     2040
   attactccat ggaattttga ggaagttgtc gataaaggtg cgtcagctca atcgttcatc     2100
   gagaggatga ccaactttga caagaattta ccgaacgaaa aagtattgcc taagcacagt     2160
   ttactttacg agtatttcac agtgtacaat gaactcacga aagttaagta tgtcactgag     2220
   ggcatgcgta aacccgcctt tctaagcgga gaacagaaga aagcaatagt agatctgtta     2280
   ttcaagacca accgcaaagt gacagttaag caattgaaag aggactactt taagaaaatt     2340
   gaatgcttcg attctgtcga gatctccggg gtagaagatc gatttaatgc gtcacttggt     2400
   acgtatcatg acctcctaaa gataattaaa gataaggact tcctggataa cgaagagaat     2460
   gaagatatct tagaagatat agtgttgact cttaccctct ttgaagatcg ggaaatgatt     2520
   gaggaaagac taaaaacata cgctcacctg ttcgacgata aggttatgaa acagttaaag     2580
   aggcgtcgct atacgggctg gggacgattg tcgcggaaac ttatcaacgg gataagagac      2640
   aagcaaagtg gtaaaactat tctcgatttt ctaaagagcg acggcttcgc caataggaac 2700
   tttatgcagc tgatccatga tgactcttta accttcaaag aggatataca aaaggcacag 2760
   gtttccggac aaggggactc attgcacgaa catattgcga atcttgctgg ttcgccagcc 2820
   atcaaaaagg gcatactcca gacagtcaaa gtagtggatg agctagttaa ggtcatggga 2880
   cgtcacaaac cggaaaacat tgtaatcgag atggcacgcg aaaatcaaac gactcagaag 2940
   gggcaaaaaa acagtcgaga gcggatgaag agaatagaag agggtattaa agaactgggc 3000
   agccagatct taaaggagca tcctgtggaa aatacccaat tgcagaacga gaaactttac 3060
   ctctattacc tacaaaatgg aagggacatg tatgttgatc aggaactgga cataaaccgt 3120
   ttatctgatt acgacgtcga tgccattgta ccccaatcct ttttgaagga cgattcaatc 3180
   gacaataaag tgcttacacg ctcggataag aaccgaggga aaagtgacaa tgttccaagc 3240
   gaggaagtcg taaagaaaat gaagaactat tggcggcagc tcctaaatgc gaaactgata 3300
   acgcaaagaa agttcgataa cttaactaaa gctgagaggg gtggcttgtc tgaacttgac 3360
   aaggccggat ttattaaacg tcagctcgtg gaaacccgcc aaatcacaaa gcatgttgca 3420
   cagatactag attcccgaat gaatacgaaa tacgacgaga acgataagct gattcgggaa 3480
   gtcaaagtaa tcactttaaa gtcaaaattg gtgtcggact tcagaaagga ttttcaattc 3540
   tataaagtta gggagataaa taactaccac catgcgcacg acgcttatct taatgccgtc 3600
   gtagggaccg cactcattaa gaaatacccg aagctagaaa gtgagtttgt gtatggtgat 3660
   tacaaagttt atgacgtccg taagatgatc gcgaaaagcg aacaggagat aggcaaggct 3720
   acagccaaat acttctttta ttctaacatt atgaatttct ttaagacgga aatcactctg 3780
   gcaaacggag agatacgcaa acgaccttta attgaaacca atggggagac aggtgaaatc 3840
   gtatgggata agggccggga cttcgcgacg gtgagaaaag ttttgtccat gccccaagtc 3900
   aacatagtaa agaaaactga ggtgcagacc ggagggtttt caaaggaatc gattcttcca 3960
   aaaaggaata gtgataagct catcgctcgt aaaaaggact gggacccgaa aaagtacggt 4020
   ggcttcgata gccctacagt tgcctattct gtcctagtag tggcaaaagt tgagaaggga 4080
   aaatccaaga aactgaagtc agtcaaagaa ttattgggga taacgattat ggagcgctcg 4140
   tcttttgaaa agaaccccat cgacttcctt gaggcgaaag gttacaagga agtaaaaaag 4200
   gatctcataa ttaaactacc aaagtatagt ctgtttgagt tagaaaatgg ccgaaaacgg 4260
   atgttggcta gcgccggaga gcttcaaaag gggaacgaac tcgcactacc gtctaaatac 4320
   gtgaatttcc tgtatttagc gtcccattac gagaagttga aaggttcacc tgaagataac 4380
   gaacagaagc aactttttgt tgagcagcac aaacattatc tcgacgaaat catagagcaa 4440
   atttcggaat tcagtaagag agtcatccta gctgatgcca atctggacaa agtattaagc 4500
   gcatacaaca agcacaggga taaacccata cgtgagcagg cggaaaatat tatccatttg 4560
   tttactctta ccaacctcgg cgctccagcc gcattcaagt attttgacac aacgatagat 4620
   cgcaaacgat acacttctac caaggaggtg ctagacgcga cactgattca ccaatccatc 4680
   acgggattat atgaaactcg gatagatttg tcacagcttg ggggtgacgg atcccccaag 4740
   aagaagagga aagtctcgag cgactacaaa gaccatgacg gtgattataa agatcatgac 4800
   atcgattaca aggatgacga tgacaaggct gcagga 4836
<210> 11
   <211> 4854
   <212> DNA
   <213> Unknown
<220>
   <223> Synthetic
<400> 11
<210> 12
   <211> 4875
   <212> DNA
   <213> Unknown
<220>
   <223> Synthetic
<400> 12
   atggactaca aagaccatga cggtgattat aaagatcatg acatcgatta caaggatgac 60
   gatgacaaga tggcccccaa gaagaagagg aaggtgggca ttcaccgcgg ggtacctgga 120
   ggttctatgg gatcccaact agtcaaaagt gaactggagg agaagaaatc tgaacttcgt 180
   cataaattga aatatgtgcc tcatgaatat attgaattaa ttgaaattgc cagaaattcc 240
   actcaggata gaattcttga aatgaaggta atggaatttt ttatgaaagt ttatggatat 300
   agaggtaaac atttgggtgg atcaaggaaa ccggacggag caatttatac tgtcggatct 360
   cctattgatt acggtgtgat cgtggatact aaagcttata gcggaggtta taatctgcca 420
   attggccaag cagatgaaat gcaacgatat gtcgaagaaa atcaaacacg aaacaaacat 480
   atcaacccta atgaatggtg gaaagtctat ccatcttctg taacggaatt taagttttta 540
   tttgtgagtg gtcactttaa aggaaactac aaagctcagc ttacacgatt aaatcatatc 600
   actaattgta atggagctgt tcttagtgta gaagagcttt taattggtgg agaaatgatt 660
   aaagccggca cattaacctt agaggaagtc agacggaaat ttaataacgg cgagataaac 720
   tttagcggca gcgagactcc cgggacctca gagtccgcca cacccgaaag tatggataaa 780
   aagtattcta ttggtttagc tatcggcact aattccgttg gatgggctgt cataaccgat 840
   gaatacaaag taccttcaaa gaaatttaag gtgttgggga acacagaccg tcattcgatt 900
   aaaaagaatc ttatcggtgc cctcctattc gatagtggcg aaacggcaga ggcgactcgc 960
   ctgaaacgaa ccgctcggag aaggtataca cgtcgcaaga accgaatatg ttacttacaa 1020
   gaaattttta gcaatgagat ggccaaagtt gacgattctt tctttcaccg tttggaagag 1080
   tccttccttg tcgaagagga caagaaacat gaacggcacc ccatctttgg aaacatagta 1140
   gatgaggtgg catatcatga aaagtaccca acgatttatc acctcagaaa aaagctagtt 1200
   gactcaactg ataaagcgga cctgaggtta atctacttgg ctcttgccca tatgataaag 1260
   ttccgtgggc actttctcat tgagggtgat ctaaatccgg acaactcgga tgtcgacaaa 1320
   ctgttcatcc agttagtaca aacctataat cagttgtttg aagagaaccc tataaatgca 1380
   agtggcgtgg atgcgaaggc tattcttagc gcccgcctct ctaaatcccg acggctagaa 1440
   aacctgatcg cacaattacc cggagagaag aaaaatgggt tgttcggtaa ccttatagcg 1500
   ctctcactag gcctgacacc aaattttaag tcgaacttcg acttagctga agatgccaaa 1560
   ttgcagctta gtaaggacac gtacgatgac gatctcgaca atctactggc acaaattgga 1620
   gatcagtatg cggacttatt tttggctgcc aaaaacctta gcgatgcaat cctcctatct 1680
   gacatactga gagttaatac tgagattacc aaggcgccgt tatccgcttc aatgatcaaa 1740
   aggtacgatg aacatcacca agacttgaca cttctcaagg ccctagtccg tcagcaactg 1800
   cctgagaaat ataaggaaat attctttgat cagtcgaaaa acgggtacgc aggttatatt 1860
   gacggcggag cgagtcaaga ggaattctac aagtttatca aacccatatt agagaagatg 1920
   gatgggacgg aagagttgct tgtaaaactc aatcgcgaag atctactgcg aaagcagcgg 1980
   actttcgaca acggtagcat tccacatcaa atccacttag gcgaattgca tgctatactt 2040
   agaaggcagg aggattttta tccgttcctc aaagacaatc gtgaaaagat tgagaaaatc 2100
   ctaacctttc gcatacctta ctatgtggga cccctggccc gagggaactc tcggttcgca 2160
   tggatgacaa gaaagtccga agaaacgatt actccatgga attttgagga agttgtcgat 2220
   aaaggtgcgt cagctcaatc gttcatcgag aggatgacca actttgacaa gaatttaccg 2280
   aacgaaaaag tattgcctaa gcacagttta ctttacgagt atttcacagt gtacaatgaa 2340
   ctcacgaaag ttaagtatgt cactgagggc atgcgtaaac ccgcctttct aagcggagaa 2400
   cagaagaaag caatagtaga tctgttattc aagaccaacc gcaaagtgac agttaagcaa 2460
   ttgaaagagg actactttaa gaaaattgaa tgcttcgatt ctgtcgagat ctccggggta 2520
   gaagatcgat ttaatgcgtc acttggtacg tatcatgacc tcctaaagat aattaaagat 2580
   aaggacttcc tggataacga agagaatgaa gatatcttag aagatatagt gttgactctt 2640
   accctctttg aagatcggga aatgattgag gaaagactaa aaacatacgc tcacctgttc 2700
   gacgataagg ttatgaaaca gttaaagagg cgtcgctata cgggctgggg acgattgtcg 2760
   cggaaactta tcaacgggat aagagacaag caaagtggta aaactattct cgattttcta 2820
   aagagcgacg gcttcgccaa taggaacttt atgcagctga tccatgatga ctctttaacc 2880
   ttcaaagagg atatacaaaa ggcacaggtt tccggacaag gggactcatt gcacgaacat 2940
   attgcgaatc ttgctggttc gccagccatc aaaaagggca tactccagac agtcaaagta 3000
   gtggatgagc tagttaaggt catgggacgt cacaaaccgg aaaacattgt aatcgagatg 3060
   gcacgcgaaa atcaaacgac tcagaagggg caaaaaaaca gtcgagagcg gatgaagaga 3120
   atagaagagg gtattaaaga actgggcagc cagatcttaa aggagcatcc tgtggaaaat 3180
   acccaattgc agaacgagaa actttacctc tattacctac aaaatggaag ggacatgtat 3240
   gttgatcagg aactggacat aaaccgttta tctgattacg acgtcgatgc cattgtaccc 3300
   caatcctttt tgaaggacga ttcaatcgac aataaagtgc ttacacgctc ggataagaac 3360
   cgagggaaaa gtgacaatgt tccaagcgag gaagtcgtaa agaaaatgaa gaactattgg 3420
   cggcagctcc taaatgcgaa actgataacg caaagaaagt tcgataactt aactaaagct 3480
   gagaggggtg gcttgtctga acttgacaag gccggattta ttaaacgtca gctcgtggaa 3540
   acccgccaaa tcacaaagca tgttgcacag atactagatt cccgaatgaa tacgaaatac 3600
   gacgagaacg ataagctgat tcgggaagtc aaagtaatca ctttaaagtc aaaattggtg 3660
   tcggacttca gaaaggattt tcaattctat aaagttaggg agataaataa ctaccaccat 3720
   gcgcacgacg cttatcttaa tgccgtcgta gggaccgcac tcattaagaa atacccgaag 3780
   ctagaaagtg agtttgtgta tggtgattac aaagtttatg acgtccgtaa gatgatcgcg 3840
   aaaagcgaac aggagatagg caaggctaca gccaaatact tcttttattc taacattatg 3900
   aatttcttta agacggaaat cactctggca aacggagaga tacgcaaacg acctttaatt 3960
   gaaaccaatg gggagacagg tgaaatcgta tgggataagg gccgggactt cgcgacggtg 4020
   agaaaagttt tgtccatgcc ccaagtcaac atagtaaaga aaactgaggt gcagaccgga 4080
   gggttttcaa aggaatcgat tcttccaaaa aggaatagtg ataagctcat cgctcgtaaa 4140
   aaggactggg acccgaaaaa gtacggtggc ttcgatagcc ctacagttgc ctattctgtc 4200
   ctagtagtgg caaaagttga gaagggaaaa tccaagaaac tgaagtcagt caaagaatta 4260
   ttggggataa cgattatgga gcgctcgtct tttgaaaaga accccatcga cttccttgag 4320
   gcgaaaggtt acaaggaagt aaaaaaggat ctcataatta aactaccaaa gtatagtctg 4380
   tttgagttag aaaatggccg aaaacggatg ttggctagcg ccggagagct tcaaaagggg 4440
   aacgaactcg cactaccgtc taaatacgtg aatttcctgt atttagcgtc ccattacgag 4500
   aagttgaaag gttcacctga agataacgaa cagaagcaac tttttgttga gcagcacaaa 4560
   cattatctcg acgaaatcat agagcaaatt tcggaattca gtaagagagt catcctagct 4620
   gatgccaatc tggacaaagt attaagcgca tacaacaagc acagggataa acccatacgt 4680
   gagcaggcgg aaaatattat ccatttgttt actcttacca acctcggcgc tccagccgca 4740
   ttcaagtatt ttgacacaac gatagatcgc aaacgataca cttctaccaa ggaggtgcta 4800
   gacgcgacac tgattcacca atccatcacg ggattatatg aaactcggat agatttgtca 4860
   cagcttgggg gtgac 4875
<210> 13
   <211> 9
   <212> PRT
   <213> Unknown
<220>
   <223> Synthetic
<400> 13
   Gly Gly Ser Gly Gly Ser Gly Gly Ser
   1               5
<210> 14
   <211> 18
   <212> PRT
   <213> Unknown
<220>
   <223> Synthetic
<400> 14
   Gly Gly Ser Gly Gly Ser Gly Gly Ser Gly Gly Ser Gly Gly Ser Gly
   1               5                   10                  15
   Gly Ser
<210> 15
   <211> 10
   <212> PRT
   <213> Unknown
<220>
   <223> Synthetic
<400> 15
   Met Lys Ile Ile Glu Gln Leu Pro Ser Ala
   1               5                   10
<210> 16
   <211> 10
   <212> PRT
   <213> Unknown
<220>
   <223> Synthetic
<400> 16
   Val Arg His Lys Leu Lys Arg Val Gly Ser
   1               5                   10
<210> 17
   <211> 15
   <212> PRT
   <213> Unknown
<220>
   <223> Synthetic
<400> 17
   Val Pro Phe Leu Leu Glu Pro Asp Asn Ile Asn Gly Lys Thr Cys
   1               5                   10                  15
<210> 18
   <211> 12
   <212> PRT
   <213> Unknown
<220>
   <223> Synthetic
<400> 18
   Gly His Gly Thr Gly Ser Thr Gly Ser Gly Ser Ser
   1               5                   10
<210> 19
   <211> 7
   <212> PRT
   <213> Unknown
<220>
   <223> Synthetic
<400> 19
   Met Ser Arg Pro Asp Pro Ala
   1               5
<210> 20
   <211> 12
   <212> PRT
   <213> Unknown
<220>
   <223> Synthetic
<400> 20
   Gly Ser Ala Gly Ser Ala Ala Gly Ser Gly Glu Phe
   1               5                   10
<210> 21
   <211> 12
   <212> PRT
   <213> Unknown
<220>
   <223> Synthetic
<400> 21
   Ser Gly Ser Glu Thr Pro Gly Thr Ser Glu Ser Ala
   1               5                   10
<210> 22
   <211> 16
   <212> PRT
   <213> Unknown
<220>
   <223> synthetic
<400> 22
   Ser Gly Ser Glu Thr Pro Gly Thr Ser Glu Ser Ala Thr Pro Glu Ser
   1               5                   10                  15
<210> 23
   <211> 21
   <212> PRT
   <213> Unknown
<220>
   <223> synthetic
<400> 23
   Ser Gly Ser Glu Thr Pro Gly Thr Ser Glu Ser Ala Thr Pro Glu Gly
   1               5                   10                  15
   Gly Ser Gly Gly Ser
               20
<210> 24
   <211> 12
   <212> PRT
   <213> Unknown
<220>
   <223> synthetic
<400> 24
   Ser Ile Val Ala Gln Leu Ser Arg Pro Asp Pro Ala
   1               5                   10

## Claims

1. A method for assembling a target nucleic acid, the method comprising:
(a) providing a plurality of double-stranded polynucleotides, each polynucleotide comprising a flanking region on the 3' end, or 5' end, or 3' end and 5' end, wherein the flanking region comprises a first recognition site and a second recognition site, wherein the plurality of double-stranded polynucleotides together comprise the target nucleic acid;
(b) nicking, in vitro, a first strand of each polynucleotide with a first nickase to produce a first nick, wherein the first nickase is configured to recognize and bind to the first recognition site; and
(c) nicking, in vitro, a second strand of each polynucleotide with a second nickase to produce a second nick, wherein the second nickase is configured to recognize and bind to the second recognition site, thereby producing cleaved polynucleotide fragments, each having an overhang defined by the first nick and the second nick, wherein the overhang is predesigned by selecting the first and second recognition sites, and
(d) assembling the cleaved polynucleotide fragments at the overhangs to form the target nucleic acid, wherein a first overhang of a first cleaved polynucleotide fragment is complementary to a second overhang of a second cleaved polynucleotide fragment, and wherein said assembling comprises ligating the assembled cleaved polynucleotide fragments to produce a ligated product,
wherein at least one of the plurality of double-stranded polynucleotides was provided on a solid support.

2. The method of claim 1, wherein the first nickase or the second nickase comprises one or more of: Cas9 fused to a nuclease via a linker at the N terminus ("fCas9"), Cas9 fused to a nuclease via a linker at the C terminus ("Cas9f'), RISC complexed with or fused to a nuclease, transcription activator-like effector (TALE) complexed with or fused to a nuclease, zinc-finger complexed with or fused to a nuclease, meganuclease, and any combination thereof.

3. The method of claim 2, wherein the Cas9 is catalytically inactive and/or the nuclease is incapable of binding to DNA.

4. The method of claim 2 or claim 3, wherein the nuclease is FokI, optionally wherein the FokI is a catalytically inactive monomer of Fokl cleavage domain.

5. The method of claim 4, wherein the first nickase or the second nickase is a dimer wherein the FokI dimerizes with a catalytically active monomer of FokI cleavage domain.

6. The method of claim 4, wherein the FokI is a catalytically active monomer of FokI cleavage domain.

7. The method of claim 6, wherein the first nickase or the second nickase is a dimer wherein the FokI dimerizes with a catalytically active or inactive monomer of FokI cleavage domain.

8. The method of claim 5 or claim 7, wherein the first nickase or the second nickase is a heterodimer.

9. The method of claim 2, wherein in the first nickase, the Cas9 or RISC is directed by a first guide sequence such as gRNA to the first site, wherein the first guide sequence comprises a first sequence that is complementary to the first site.

10. The method of claim 9, wherein in the second nickase, the Cas9 or RISC is directed by a second guide sequence such as gRNA to the second site, wherein the second guide sequence comprises a second sequence that is complementary to the second site.

11. The method of claim 10, wherein the first guide sequence and the second guide sequence are non-naturally occurring.

12. The method of claim 10, wherein the first nickase and the second nickase nick at a predetermined position upstream or downstream to the first site and the second site, respectively, to produce the first nick and the second nick, respectively.

13. The method of claim 12, wherein the first and second sites are selected such that the first nick and the second nick are offset by a predefined number of nucleotides.

## Patentansprüche

1. Verfahren zum Assemblieren einer Zielnucleinsäure, wobei das Verfahren Folgendes umfasst:
(a) Bereitstellen einer Vielzahl von doppelsträngigen Polynucleotiden, wobei jedes Polynucleotid eine flankierende Region am 3'-Ende oder am 5'-Ende oder am 3'-Ende und am 5'-Ende umfasst, wobei die flankierende Region eine erste Erkennungsstelle und eine zweite Erkennungsstelle umfasst, wobei die Vielzahl von doppelsträngigen Polynucleotiden zusammen die Zielnucleinsäure ausmachen;
(b) Nicken eines ersten Strangs jedes Polynucleotids in vitro mit einer ersten Nickase, um einen ersten Nick zu erzeugen, wobei die erste Nickase ausgelegt ist, um die erste Erkennungsstelle zu erkennen und daran zu binden, und
(c) Nicken eines zweiten Strangs jedes Polynucleotids in vitro mit einer zweiten Nickase, um einen zweiten Nick zu erzeugen, wobei die zweite Nickase ausgelegt ist, um die zweite Erkennungsstelle zu erkennen und daran zu binden, wodurch gespaltene Polynucleotidfragmente erzeugt werden, von denen jedes einen Überhang aufweist, der durch den ersten Nick und den zweiten Nick definiert wird, wobei der Überhang durch Selektieren der ersten und der zweiten Erkennungsstelle vorkonstruiert wird, und
(d) Assemblieren der gespaltenen Polynucleotidfragmente an den Überhängen, um die Zielnucleinsäure zu bilden, wobei ein erster Überhang eines ersten gespaltenen Polynucleotidfragments komplementär zu einem zweiten Übergang eines zweiten gespaltenen Polynucleotidfragments ist und wobei das Assemblieren das Ligieren der assemblierten gespaltenen Polynucleotidfragmente umfasst, um ein ligiertes Produkt zu erzeugen,
wobei zumindest eines aus der Vielzahl von doppelsträngigen Polynucleotiden auf einem festen Träger bereitgestellt wurde.

2. Verfahren nach Anspruch 1, wobei die erste Nickase oder die zweite Nickase eines oder mehrere der folgenden umfasst: Cas9, fusioniert an eine Nuclease über einen Linker am N-Terminus ("fCas9"), Cas9, fusioniert an eine Nuclease über einen Linker am C-Terminus ("Cas9f"), RISC, komplexiert mit oder fusioniert an eine Nuclease, Transkriptionsaktivator-ähnlichen Effektor (TALE), komplexiert mit oder fusioniert an eine Nuclease, Zinkfinger, komplexiert mit oder fusioniert an eine Nuclease, Meganuclease und eine beliebige Kombination daraus.

3. Verfahren nach Anspruch 2, wobei das Cas9 katalytisch inaktiv ist und/oder die Nuclease nicht dazu in der Lage ist, an DNA zu binden.

4. Verfahren nach Anspruch 2 oder 3, wobei die Nuclease Fokl ist, wobei Fokl gegebenenfalls ein katalytisch inaktives Monomer der Fokl-Spaltungsdomäne ist.

5. Verfahren nach Anspruch 4, wobei die erste Nickase oder die zweite Nickase ein Dimer ist, wobei Fokl mit einem katalytisch aktiven Monomer der Fokl-Spaltungsdomäne dimerisiert.

6. Verfahren nach Anspruch 4, wobei Fokl ein katalytisch aktives Monomer der Fokl-Spaltungsdomäne ist.

7. Verfahren nach Anspruch 6, wobei die erste Nickase oder die zweite Nickase ein Dimer ist, wobei Fokl mit einem katalytisch aktiven oder inaktiven Monomer der Fokl-Spaltungsdomäne dimerisiert.

8. Verfahren nach Anspruch 5 oder 7, wobei die erste Nickase oder die zweite Nickase ein Heterodimer ist.

9. Verfahren nach Anspruch 2, wobei das Cas9 oder RISC in der ersten Nickase durch eine erste Führungssequenz wie gRNA auf die erste Stelle ausgerichtet ist, wobei die erste Führungssequenz eine erste Sequenz umfasst, die komplementär zur ersten Stelle ist.

10. Verfahren nach Anspruch 9, wobei das Cas9 oder RISC in der zweiten Nickase durch eine zweite Führungssequenz wie gRNA auf die zweite Stelle ausgerichtet ist, wobei die zweite Führungssequenz eine zweite Sequenz umfasst, die komplementär zur zweiten Stelle ist.

11. Verfahren nach Anspruch 10, wobei die erste Führungssequenz und die zweite Führungssequenz nicht natürlich vorkommend sind.

12. Verfahren nach Anspruch 10, wobei die erste Nickase und die zweite Nickase an einer vorbestimmten Position stromauf oder stromab der ersten Stelle bzw. der zweiten Stelle nicken, um den ersten bzw. den zweiten Nick zu erzeugen.

13. Verfahren nach Anspruch 12, wobei die erste und die zweite Stelle so ausgewählt sind, dass der erste Nick und der zweite Nick um eine vorbestimmte Anzahl an Nucleotiden versetzt sind.

## Revendications

1. Procédé d'assemblage d'un acide nucléique cible, le procédé comprenant les étapes consistant à :
(a) fournir une pluralité de polynucléotides double-brin, chaque polynucléotide comprenant une région flanquante à l'extrémité 3', ou à l'extrémité 5', ou à l'extrémité 3'et à l'extrémité 5', dans lequel la région flanquante comprend un premier site de reconnaissance et un second site de reconnaissance, dans lequel la pluralité de polynucléotides double-brin comprennent ensemble l'acide nucléique cible ;
(b) couper, in vitro, un premier brin de chaque polynucléotide à l'aide d'une première nickase pour produire une première coupure, la première nickase étant configurée pour reconnaître et se lier au premier site de reconnaissance ; et
(c) couper, in vitro, un second brin de chaque polynucléotide à l'aide d'une seconde nickase pour produire une seconde coupure, la seconde nickase étant configurée pour reconnaître et se lier au second site de reconnaissance, produisant ainsi des fragments polynucléotidiques clivés, chacun ayant un surplomb défini par la première coupure et la seconde coupure, dans lequel le surplomb est préconçu en sélectionnant les premier et second sites de reconnaissance, et
(d) assembler les fragments polynucléotidiques clivés au niveau des surplombs pour former l'acide nucléique cible, dans lequel un premier surplomb d'un premier fragment polynucléotidique clivé est complémentaire d'un second surplomb d'un second fragment polynucléotidique clivé, et dans lequel ledit assemblage comprend la ligature des fragments polynucléotidiques clivés assemblés pour produire un produit ligaturé,
dans lequel au moins l'un de la pluralité de polynucléotides double-brin a été fourni sur un support solide.

2. Procédé selon la revendication 1, dans lequel la première nickase ou la seconde nickase comprend une ou plusieurs parmi : Cas9 fusionnée à une nucléase via un lieur au niveau de l'extrémité N terminale (« fCas9 »), Cas9 fusionnée à une nucléase via un lieur au niveau de l'extrémité C terminale (« Cas9f »), RISC complexé ou fusionné à une nucléase, effecteur de type activateur de transcription (TALE) complexé ou fusionné à une nucléase, doigt de zinc complexé ou fusionné à une nucléase, méganucléase, et toute combinaison de ceux-ci.

3. Procédé selon la revendication 2, dans lequel la Cas9 est catalytiquement inactive et/ou la nucléase est incapable de se lier à un ADN.

4. Procédé selon la revendication 2 ou la revendication 3, dans lequel la nucléase est FokI, facultativement dans lequel FokI est un monomère catalytiquement inactif du domaine de clivage de FokI.

5. Procédé selon la revendication 4, dans lequel la première nickase ou la seconde nickase est un dimère dans lequel FokI se dimérise avec un monomère catalytiquement actif du domaine de clivage de FokI.

6. Procédé selon la revendication 4, dans lequel FokI est un monomère catalytiquement actif d'un domaine de clivage de FokI.

7. Procédé selon la revendication 6, dans lequel la première nickase ou la seconde nickase est un dimère dans lequel FokI se dimérise avec un monomère catalytiquement actif ou inactif du domaine de clivage de FokI.

8. Procédé selon la revendication 5 ou la revendication 7, dans lequel la première nickase ou la seconde nickase est un hétérodimère.

9. Procédé selon la revendication 2, dans lequel dans la première nickase, la Cas9 ou le RISC est dirigé par une première séquence guide telle que de l'ARNg vers le premier site, dans lequel la première séquence guide comprend une première séquence qui est complémentaire du premier site.

10. Procédé selon la revendication 9, dans lequel dans la seconde nickase, la Cas9 ou le RISC est dirigé par une seconde séquence guide telle que de l'ARNg vers le second site, dans lequel la seconde séquence guide comprend une seconde séquence qui est complémentaire du second site.

11. Procédé selon la revendication 10, dans lequel la première séquence guide et la seconde séquence guide ne sont pas d'origine naturelle.

12. Procédé selon la revendication 10, dans lequel la première nickase et la seconde nickase coupent à une position prédéterminée en amont ou en aval du premier site et du second site, respectivement, pour produire la première et la seconde coupure, respectivement.

13. Procédé selon la revendication 12, dans lequel les premier et second sites sont sélectionnés de telle sorte que la première coupure et la seconde coupure sont décalés d'un nombre prédéfini de nucléotides.
